(19) Europäisches Patentamt
European Patent Office
Office européen des brevets

(11) **EP 2 854 857 B1**

(12) **EUROPEAN PATENT SPECIFICATION**

(45) Date of publication and mention
of the grant of the patent:
**28.11.2018 Bulletin 2018/48**

(21) Application number: **12723622.2**

(22) Date of filing: **25.05.2012**

(51) Int Cl.:
*A61K 47/64* (2017.01)     *A61K 47/69* (2017.01)
*C12N 15/88* (2006.01)     *C12N 9/02* (2006.01)
*A61K 47/34* (2017.01)     *A61K 48/00* (2006.01)
*A61K 9/51* (2006.01)     *A61L 27/54* (2006.01)
*A61L 31/16* (2006.01)     *A61L 31/10* (2006.01)
*A61L 31/14* (2006.01)     *A61L 27/34* (2006.01)

(86) International application number:
**PCT/EP2012/002257**

(87) International publication number:
**WO 2013/174409 (28.11.2013 Gazette 2013/48)**

(54) **REVERSIBLE IMMOBILIZATION AND/OR CONTROLLED RELEASE OF NUCLEIC ACID CONTAINING NANOPARTICLES BY (BIODEGRADABLE) POLYMER COATINGS**

REVERSIBLE IMMOBILISIERUNG UND/ODER KONTROLLIERTE FREISETZUNG VON NUCLEINSÄUREN MIT NANOPARTIKELN DURCH (BIOLOGISCH ABBAUBARE) POLYMERBESCHICHTUNGEN

IMMOBILISATION RÉVERSIBLE ET/OU LIBÉRATION CONTRÔLÉE DE NANOPARTICULES CONTENANT DES ACIDES NUCLÉIQUES PAR LE BIAIS DE REVÊTEMENTS DE POLYMÈRE (BIODÉGRADABLE)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**08.04.2015 Bulletin 2015/15**

(60) Divisional application:
**18199190.2**

(73) Proprietor: **CureVac AG
72076 Tübingen (DE)**

(72) Inventors:
• **BAUMHOF, Patrick
72144 Dusslingen (DE)**
• **WENDEL, Hans-Peter
72336 Balingen (DE)**
• **NOLTE, Andrea
75392 Deckenpfronn (DE)**
• **WALKER, Tobias
72074 Tübingen (DE)**

(74) Representative: **Graf von Stosch, Andreas et al
Graf von Stosch
Patentanwaltsgesellschaft mbH
Prinzregentenstraße 22
80538 München (DE)**

(56) References cited:
**WO-A1-2011/026641**

• **BRITO LUIS A ET AL: "Non-viral eNOS gene delivery and transfection with stents for the treatment of restenosis", BIOMEDICAL ENGINEERING ONLINE, BIOMED CENTRAL LTD, LONDON, GB, vol. 9, no. 1, 27 September 2010 (2010-09-27), page 56, XP021077699, ISSN: 1475-925X, DOI: 10.1186/1475-925X-9-56**
• **FABIENNE DANHIER ET AL: "PLGA-based nanoparticles: An overview of biomedical applications", JOURNAL OF CONTROLLED RELEASE, vol. 161, no. 2, 4 February 2012 (2012-02-04), pages 505-522, XP055051719, ISSN: 0168-3659, DOI: 10.1016/j.jconrel.2012.01.043**

**Description**

The present invention relates to nanoparticles, as defined in the attached claims, comprising nucleic acids, the nanoparticles being coated with a biodegradable polymer, which is a poly(lactic-co-glycolic acid) (PLGA) polymer for reversible immobilization and/or controlled release of the nanoparticles and thus the nucleic acid. Furthermore, the present invention is directed to medical or diagnostic devices, particularly stents and implants, comprising said nanoparticles for reversible immobilization and/or controlled release. Furthermore, the present invention is directed to said coated nanoparticles and said medical devices and implants coated with these nanoparticles for use in the prophylactic or therapeutic treatment of diseases, particularly in the prevention or treatment of restenosis, calcification, foreign body reaction and inflammation. Additionally, the present invention is directed to a method for preparing a coated nanoparticle as defined by the claims. Further disclosed herein is a method for coating nucleic acid comprising nanoparticles with (biodegradable) polymers and to a method for coating of medical or diagnostic devices with a composition comprising said nanoparticles and a (biodegradable) polymer.

Drug delivery plays an important role in the development of pharmaceutical dosage forms for the health care industry because often the duration of drug release needs to be extended over days up to several months. This can be achieved by incorporation of drugs into polymeric materials to control drug release at a predefined and reproducible rate for a specific period of time.

In recent years the interest for biodegradable polymers as drug delivery systems, which control and prolong the action of therapeutic agents, has grown in importance. Especially delivery systems based on biodegradable polymers are advantageous because they do not require removal from the patients at the end of the treatment period due to their degradation into physiologically occurring compounds that can be degraded from the body. This provides significant benefits particularly regarding safety-concerns about non-biodegradable polymers.

The most attractive and commonly used biodegradable polymers are polyesters such as poly(lactic acid) (PLA), poly(lactic-co-glycolic acid) (PLGA) and poly(q-caprolactone (PCL). These materials are commercially available in different compositions and molecular weights which allow control of degradation of the polymer.

For medical devices and implants (e.g. stents, artificial organs, biosensors, catheters, scaffolds for tissue engineering, heart valves, etc.) such biodegradable polymers for drug-delivery are of particular interest. When such devices are implanted into the body, the body can react to these in different ways which can result in a risk for the patient. The tissue injury resulting from the implantation of the device may for example induce an inflammatory response. This might be end up in a chronic inflammation. Some possibilities are described to solve this problem, e.g. Onuki et al. describe the possibility to prevent inflammation by coating of implants with VEGF-and dexamethasone-loaded PLGA microsphere/PVA hydrogel (Onuki, Y., U. Bhardwaj, et al. (2008). "A review of the biocompatibility of implantable devices: current challenges to overcome foreign body response." J Diabetes Sci Technol 2(6): 1003-15.)

Another risk associated with the introduction of medical devices and implants is the induction of a foreign body reaction, which might result in the necessity to remove the device.

One further problem of medical devices is calcification. Calcification is the process in which mineral calcium builds up in implantable devices which can dramatically compromise the device function. To prevent this process in the body several drugs attached to the device are described, inter alia ethanehydroxydiphosphonate, $FeCl_3$, and $AlCl_3$, levamisole (inhibitor of alkaline phosphatise), and protamine sulphate and dexamethasone (reviewed in Onuki et al., see above).

Coronary artery disease is the most common cause of morbidity and mortality in the world, e.g. it is responsible for 1 out of 5 deaths in the United States. Coronary artery bypass graft surgery has been proven an effective approach to coronary heart disease. In this context, a problem of particular importance is restenosis after stent implantations. Coronary stents are used for patients undergoing coronary revascularization. The implanted stent contacts the vessel wall directly and protrudes into the endovascular tissue. This can harm the endothelial vascular tissue, resulting in an inflammatory reaction, which may play a key role in the process of neointimal proliferation, leading to restenosis.

The solution of this problem was found in drug-eluting stents which release drugs directed against the development of restenosis embedded in drug-releasing polymers.

The first polymers used for drug-eluting stents were permanent polymers which mean that they were not biodegradable. But it turned out that these polymers were associated with delayed re-endothelialization, localized vascular inflammation, thrombogenic reactions, and impaired functionality, which may have been caused by a hypersensitivity reaction to the presence of the permanent polymer (reviewed in Onuma, Y., J. Ormiston, et al. "Bioresorbable scaffold technologies." Circ J 75(3): 509-20). To solve this problem stents coated with biodegradable polymers particularly on the basis of such as poly(lactic acid) (PLA), poly(lactic-co-glycolic acid) (PLGA) and poly(q-caprolactone (PCL) were developed.

Drugs used for prevention of restenosis include immunosuppressive drugs (e.g. sirolimus, everolimus, tacrolimus, ABT-578), antiproliferative drugs (e.g. paclitaxel, antinomycin, angiopeptin), and antimigratory drugs (e.g. batimastat).

No more than a few years ago also gene therapy approaches were examined to prevent restenosis, e.g. the introduction of genes coding for proteins known to destroy dividing cells like thimidine kinase, cytosine deaminase, Fas ligand, CDK2, CDC3, cyclin B, CDK inhibitors p21 and p27, p16-p27, p53, hRAD 50, etc. or proteins which reduce intimal hyperplasia like PDGF receptor beta, TIMP-1, PAI-1 etc. Furthermore VEGF, nitric oxide synthetases (eNOS and iNOS), thrombin inhibitor hiridun, TFPI, prostacyclin synthase (PGIS), and COX-1 seem to be advantageous for preventing restenosis. In further studies the introduction of antisense or small interfering RNA (siRNA) directed against proteins involved in the development of restenosis e.g. PDGF were examined.

Most of these studies have been performed using commercially available cationic lipid transfection reagents such as Lipofectamine or Lipofectamine Plus. But the use of these cationic lipids is not a clinically viable option due to the known toxicity of the transfection reagents (Armeanu, S., J. Pelisek, et al. (2000). "Optimization of nonviral gene transfer of vascular smooth muscle cells in vitro and in vivo." Mol Ther 1(4): 366-75).

Therefore, Cohen-Sack et al. performed studies with PLGA-based nanoparticles delivering PDGF-siRNA into cells. This delivery system was made to release siRNA in a cell over a period of one month (Cohen-Sacks, H., Y. Najajreh, et al. (2002). "Novel PDGFbetaR antisense encapsulated in polymeric nanospheres for the treatment of restenosis." Gene Ther 9(23): 1607-16.). Thus, PLGA was used as transfection vehicle for delivering siRNA into the cells and not for coating nucleic-acid comprising nanoparticles for reversible immobilization and/or controlled release.

WO 2011/026641 further describes the use of polyethylenglycol/peptide conjugates for the transfection of nucleic acids.

In a recent study Brito et al. described the immobilization of lipoplexes containing plasmid DNA coding for eNOS on stents using gelatin coatings. Therefore nucleic acid containing lipoplexes were diluted in gelatine and fixed on the stent by air-drying. Subsequently, PLGA dissolved in acetone was used for coating the gelatine-layer to prevent any premature dissolution of the gelatine layer during implantation. (Brito, L. and M. Amiji (2007). "Nanoparticulate carriers for the treatment of coronary restenosis." Int J Nanomedicine 2(2): 143-61.) Similar results were reported in Brito L. et al.: "Non-viral eNos gene delivery and transfection with stents for the treatment of restenosis", Biomedical Engineering Online, vol. 9, no. 1 (2010).

PLGA based nanoparticles were also described and reviewed in Danhier et al. ("PLGA-based nanoparticles: An overview of biomedical applications"; Journal of Controlled Release; vol. 161, no. 2 (2012), 505-522).

Although a lot of progress has been made in this field there is still a need for possibilities to combine efficient transfection vehicles (particularly based on cationic peptides/proteins and polymers) with controlled release and/or immobilization of nucleic acids, particularly for the use with medical or diagnostic devices. So far, it was not possible to immobilize hydrophilic nucleic acid containing nanoparticles based on cationic peptides/proteins or polymers with biodegradable polymers, like PLGA for reversible immobilization and/or controlled drug release due to their insolubility in non-organic solvents.

Therefore the object of the underlying invention was to provide means allowing for reversibe immobilization and/or controlled release of nucleic acids while avoiding preferably in parallel the above mentioned disadvantages.

This object is solved by the subject matter of the present invention, preferably by the subject matter of the attached claims. Particularly, according to the first embodiment of the present invention the above object is solved by nanoparticles (= polymeric carrier cargo complex) based on a complex of a nucleic acid, which is DNA or RNA, and a polymeric carrier molecule according to generic fomula (I) coated with a biodegradable polymer, wherein the biodegradable polymer is PLGA for reversible immobilization and/or controlled drug release.

In this context the inventors surprisingly found that hydrophilic nucleic acids, if comprised in nanoparticles based on a polymeric carrier molecule according to generic formula (I) can be dissolved in organic solvents; or mixed with organic solvents at low water content without significant loss of physicochemical integrity of the nanoparticles and particularly of the comprised nucleic acid and their biological efficiency. This allows the mixture with those (e.g. biodegradable) polymers which are only soluble in organic solvents (or at least in solvents comprising a high percentage of an organic solvent) to generate homogenous solutions which can be utilized for reversible immobilization of such nucleic acid comprising nanoparticles in a biodegradable matrix by different coating methods. (e.g. dip coating, spray drying, flow coating, spin coating). Such immobilized nanoparticles can be utilized for controlled drug release.

The inventive nanoparticles coated with a biodegradable polymer for reversible immobilization and/or controlled drug release comprise a coating with a biodegradable polymer, wherein the biodegradable polymer is a PLGA polymer, a nucleic acid, which is a DNA or RNA, as a cargo and a polymeric carrier according to formula (I):

$$L-P^1-S-[S-P^2-S]_n-S-P^3-L$$

wherein,

P$^1$ and P$^3$     are different or identical to each other and represent a linear or branched hydrophilic polymer chain, each P$^1$ and P$^3$ exhibiting at least one -SH-moiety, capable to form a disulfide linkage upon condensation with

component P$^2$, or alternatively with (AA)$_x$, or [(AA)$_x$]$_z$ if such components are used as a linker between P$^1$ and P$^2$ or P$^3$ and P$^2$) and/or with further components (e.g. (AA)$_x$, [(AA)$_x$]$_z$ or L), the linear or branched hydrophilic polymer chain selected independent from each other from polyethylene glycol (PEG), poly-*N*-(2-hydroxypropyl)methacrylamide, poly-2-(methacryloyloxy)ethyl phosphorylcholines, poly(hydroxyalkyl L-asparagine), poly(2-(methacryloyloxy)ethyl phosphorylcholine), hydroxyethylstarch or poly(hydroxyalkyl L-glutamine), wherein the hydrophilic polymer chain exhibits a molecular weight of about 1 kDa to about 100 kDa, preferably of about 2 kDa to about 25 kDa; or more preferably of about 2 kDa to about 10 kDa, e.g. about 5 kDa to about 25 kDa or 5 kDa to about 10 kDa;

P$^2$     is a cationic or polycationic peptide or protein, and preferably having a length of about 3 to about 100 amino acids, more preferably having a length of about 3 to about 50 amino acids, even more preferably having a length of about 3 to about 25 amino acids, e.g. a length of about 3 to 10, 5 to 15, 10 to 20 or 15 to 25 amino acids, more preferably a length of about 5 to about 20 and even more preferably a length of about 10 to about 20;

is a cationic or polycationic polymer, typically having a molecular weight of about 0.5 kDa to about 30 kDa, including a molecular weight of about 1 kDa to about 20 kDa, even more preferably of about 1.5 kDa to about 10 kDa, or having a molecular weight of about 0.5 kDa to about 100 kDa, including a molecular weight of about 10 kDa to about 50 kDa, even more preferably of about 10 kDa to about 30 kDa;

each P$^2$ exhibiting at least two -SH-moieties, capable to form a disulfide linkage upon condensation with further components P$^2$ or component(s) P$^1$ and/or P$^3$ or alternatively with further components (e.g. (AA)$_x$, or [(AA)$_x$]$_z$)

-S-S-     is a (reversible) disulfide bond (the brackets are omitted for better readability), wherein S preferably represents sulphur or a -SH carrying moiety, which has formed a (reversible) disulfide bond. The (reversible) disulfide bond is preferably formed by condensation of -SH-moieties of either components P$^1$ and P$^2$, P$^2$ and P$^2$, or P$^2$ and P$^3$, or optionally of further components as defined herein (e.g. L, (AA)$_x$, [(AA)$_x$]$_z$, etc.); The -SH-moiety may be part of the structure of these components or added by a modification as defined below;

L     is an optional ligand, which may be present or not, and may be selected independent from the other from RGD, Transferrin, Folate, a signal peptide or signal sequence, a localization signal or sequence, a nuclear localization signal or sequence (NLS), an antibody, a cell penetrating peptide, (e.g. TAT or KALA), a ligand of a receptor (e.g. cytokines, hormones, growth factors etc.), small molecules (e.g. carbohydrates like mannose or galctose or synthetic ligands), small molecule agonists, inhibitors or antagonists of receptors (e.g. RGD peptidomimetic analogues) etc.;

n     is an integer, typically selected from a range of about 1 to 50, preferably from a range of about 1, 2 or 3 to 30, more preferably from a range of about 1, 2, 3, 4, or 5 to 25, or a range of about 1, 2, 3, 4, or 5 to 20, or a range of about 1, 2, 3, 4, or 5 to 15, or a range of about 1, 2, 3, 4, or 5 to 10, including e.g. a range of about 4 to 9, 4 to 10, 3 to 20, 4 to 20, 5 to 20, or 10 to 20, or a range of about 3 to 15, 4 to 15, 5 to 15, or 10 to 15, or a range of about 6 to 11 or 7 to 10. Most preferably, n is in a range of about 1, 2, 3, 4, or 5 to 10, more preferably in a range of about 1, 2, 3, or 4 to 9, in a range of about 1, 2, 3, or 4 to 8, or in a range of about 1, 2, or 3 to 7.

These nucleic acid comprising nanoparticles based on the polymeric carrier according to formula (I) are also termed herein as polymeric carrier cargo complexes.

The polymeric carrier molecule according to generic formula (I) is prepared by a synthesis strategy which allows to define the length of the polymer chain and to combine desired properties of different (short) polymers in one polymer, e.g. to efficiently compact nucleic acids for the purpose of efficient transfection of nucleic acids for the purposes of gene therapy or other therapeutic applications without loss of activity, particularly efficient transfection of a nucleic acid into different cell lines *in vitro* but also transfection *in vivo.* The polymeric carrier molecule is furthermore not toxic to cells and provides for efficient release of its nucleic acid cargo. Finally, it shows improved resistance to agglomeration due to the reversible addition of hydrophilic polymer chains (e.g. PEG-monomers) particularly to the terminal ends of the polymeric carrier molecule according to generic formula (I), which additionally confers enhanced stability of the nucleic acid cargo with respect to serum containing media and prevents recognition of the polymeric carrier cargo complex by the immune system.

Furthermore, the polymeric carrier molecule according to generic formula (I) allows to considerably vary its peptide or polymeric content and thus to modulate its biophysical/biochemical properties, particularly the cationic properties of component [S-P$^2$-S]$_n$, quite easily and fast, e.g. by incorporating as components P$^2$ the same or different cationic peptide(s), protein(s) or polymer(s) and optionally adding other components, e.g. amino acid component(s) (AA)$_x$, into the repetitive component [S-P$^2$-S] to form a modified repetitive component such as {[S-P$^2$-S]$_a$/[S-(AA)$_x$-S]$_b$} as a core motif of the polymeric carrier (wherein a + b = n, see below). Even though consisting of quite small non-toxic monomer units the polymeric carrier molecule forms a cationic binding sequence with a defined chain length providing a strong condensation of the nucleic acid cargo and complex stability. Under the reducing conditions of the cytosol (e.g. cytosolic GSH), the complex is rapidly degraded into its monomers, which are further degraded (e.g. oligopeptides) or secreted (e.g. PEG). This supports deliberation of the nucleic acid cargo in the cytosol. Due to degradation into small oligopeptides in the cytosol, no toxicity is observed as known for high-molecular oligopeptides, e.g. from high-molecular oligoarginine. The PEG-"coating" also allows to somehow "coat" the polymeric carrier with a hydrophilic coating at its terminal ends, which prevents salt-mediated agglomeration and undesired interactions with serum contents. In the cytosole, this "coating" is easily removed under the reducing conditions of the cell. Also, this effect promotes deliberation of the nucleic acid cargo in the cytosol.

As defined above, ligands (L), may be optionally used in the polymeric carrier molecule according to generic formula (I), e.g. for direction of the carrier polymer and its complexed nucleic acid into specific cells. They may be selected independent from the other from RGD, Transferrin, Folate, a signal peptide or signal sequence, a localization signal or sequence, a nuclear localization signal or sequence (NLS), an antibody, a cell penetrating peptide, (e.g. TAT), a ligand of a receptor (e.g. cytokines, hormones, growth factors etc.), small molecules (e.g. carbohydrates like mannose or galactose or synthetic ligands), small molecule agonists, inhibitors or antagonists of receptors (e.g. RGD peptidomimetic analogues) etc. Such ligands may be attached to component $P^1$ and/or $P^3$ by reversible disulfide bonds as defined below or by any other possible chemical attachment, e.g. by amide formation (e.g. carboxylic acids, sulphonic acids, amines, etc.), by Michael addition (e.g maleinimide moieties, $\alpha,\beta$ unsatured carbonyls, etc.), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow $S_n$-type substitution reactions (e.g halogenalkans, thiols, alcohols, amines, hydrazines, hydrazides, sulphonic acid esters, oxyphosphonium salts) or other chemical moieties which can be utilized in the attachment of further components.

In the context of formula (I) of the present invention components $P^1$ and $P^3$ represent a linear or branched hydrophilic polymer chain, containing at least one -SH-moiety, each $P^1$ and $P^3$ independently selected from each other, e.g. from polyethylene glycol (PEG), poly-*N*-(2-hydroxypropyl)methacrylamide, poly-2-(methacryloyloxy)ethyl phosphorylcholines, poly(hydroxyalkyl L-asparagine) or poly(hydroxyalkyl L-glutamine). $P^1$ and $P^3$ may be identical or different to each other. Preferably, each of hydrophilic polymers $P^1$ and $P^3$ exhibits a molecular weight of about 1 kDa to about 100 kDa, preferably of about 1 kDa to about 75 kDa, more preferably of about 5 kDa to about 50 kDa, even more preferably of about 5 kDa to about 25 kDa. Additionally, each of hydrophilic polymers $P^1$ and $P^3$ typically exhibits at least one -SH-moiety, wherein the at least one -SH-moiety is capable to form a disulfide linkage upon condensation with component $P^2$ or with component $(AA)_x$, if used as linker between $P^1$ and $P^2$ or $P^3$ and $P^2$ as defined below and optionally with a further component, e.g. L and/or $(AA)_x$, e.g. if two or more -SH-moieties are contained. The following subformulas "$P^1$-S-S-$P^2$" and "$P^2$-S-S-$P^3$" of generic formula (I) above (the brackets are omitted for better readability), wherein any of S, $P^1$ and $P^3$ are as defined herein, typically represent a situation, wherein one-SH-moiety of hydrophilic polymers $P^1$ and $P^3$ was condensed with one -SH-moiety of component $P^2$ of generic formula (I) above, wherein both sulphurs of these -SH-moieties form a disulfide bond -S-S- as defined herein in formula (I). These -SH-moieties are typically provided by each of the hydrophilic polymers $P^1$ and $P^3$, e.g. via an internal cysteine or any further (modified) amino acid or compound which carries a -SH moiety. Accordingly, the subformulas "$P^1$-S-S-$P^2$" and "$P^2$-S-S-$P^3$" may also be written as "$P^1$-Cys-Cys-$P^2$" and "$P^2$-Cys-Cys-$P^3$", if the -SH- moiety is provided by a cysteine, wherein the term Cys-Cys represents two cysteines coupled via a disulfide bond, not via a peptide bond. In this case, the term "-S-S-" in these formulae may also be written as "-S-Cys", as "-Cys-S" or as "-Cys-Cys-". In this context, the term "-Cys-Cys-" does not represent a peptide bond but a linkage of two cysteines via their -SH-moieties to form a disulfide bond. Accordingly, the term "-Cys-Cys-" also may be understood generally as "-(Cys-S)-(S-Cys)-", wherein in this specific case S indicates the sulfur of the -SH-moiety of cysteine. Likewise, the terms "-S-Cys" and "-Cys-S" indicate a disulfide bond between a -SH containing moiety and a cysteine, which may also be written as "-S-(S-Cys)" and "-(Cys-S)-S". Alternatively, the hydrophilic polymers $P^1$ and $P^3$ may be modified with a -SH moiety, preferably via a chemical reaction with a compound carrying a -SH moiety, such that each of the hydrophilic polymers $P^1$ and $P^3$ carries at least one such -SH moiety. Such a compound carrying a -SH moiety may be e.g. an (additional) cysteine or any further (modified) amino acid, which carries a -SH moiety. Such a compound may also be any non-amino compound or moiety, which contains or allows to introduce a -SH moiety into hydrophilic polymers $P^1$ and $P^3$ as defined herein. Such non-amino compounds may be attached to the hydrophilic polymers $P^1$ and $P^3$ of formula (I) according to the present invention via chemical reactions or binding of compounds, e.g. by binding of a 3-thio propionic acid or thioimolane, by amide formation (e.g. carboxylic acids, sulphonic acids, amines, etc.), by Michael addition (e.g maleinimide moieties, $\alpha,\beta$ unsatured carbonyls, etc.), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow $S_n$-type substitution reactions (e.g halogenalkans, thiols, alcohols, amines, hydrazines, hydrazides, sulphonic acid esters, oxyphosphonium salts) or other chemical moieties which can be utilized in the attachment of further components. A particularly preferred PEG derivate in this context is alpha-Methoxy-omega-mercapto poly(ethylene glycol). In each case, the SH-moiety, e.g. of a cysteine or of any further (modified) amino acid or compound, may be present at the terminal ends or internally at any position of hydrophilic polymers $P^1$ and $P^3$. As defined herein, each of hydrophilic polymers $P^1$ and $P^3$ typically exhibits at least one -SH-moiety preferably at one terminal end, but may also contain two or even more -SH-moieties, which may be used to additionally attach further components as defined herein, e.g. a ligand, an amino acid component $(AA)_x$, antibodies, cell penetrating peptides (e.g. TAT), etc.

According to a further preferred aspect of the first embodiment of the present invention, each of hydrophilic polymers $P^1$ and $P^3$ may also contain at least one further functional moiety, which allows attaching further components as defined herein, e.g. a ligand, an amino acid component $(AA)_x$, etc. Such functional moieties may be selected from functionalities which allow the attachment of further components, e.g. functionalities as defined herein, e.g. by amide formation (e.g. carboxylic acids, sulphonic acids, amines, etc.), by Michael addition (e.g maleinimide moieties, $\alpha,\beta$ unsatured carbonyls, etc.), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow $S_n$-type substitution reactions (e.g halogenalkans, thiols, alcohols, amines, hydrazines, hydrazides, sulphonic acid esters, oxyphosphonium salts) or other chemical moieties which can be utilized in the attachment of further components.

Component $P^2$ in the context of formula (I) of the present invention preferably represents a cationic or polycationic peptide or protein or alternatively a cationic or polycationic polymer. Each component $P^2$ typically exhibits at least two -SH-moieties, capable to form a disulfide linkage upon condensation with further components $P^2$, component(s) $P^1$ and/or $P^3$ or alternatively with further components, e.g. amino acid components $(AA)_x$. Component $P^2$ typically occurs within the repetitive component $[\text{-S-}P^2\text{-S-}]_n$ of formula (I) of the present invention. The term "cationic or polycationic" typically refers to a charged molecule, which is positively charged (cation) at a pH value of about 1 to 9, preferably 4 to 9, 5 to 8 or even 6 to 8, more preferably of a pH value of or below 9, of or below 8, of or below 7, most preferably at physiological pH values, e.g. about 7.3 to 7.4. Accordingly, a cationic or polycationic peptide or protein as component $P^2$ or alternatively a cationic or polycationic polymer as component $P^2$ according to the present invention is positively charged under physiological conditions, particularly under physiological salt conditions of the cell *in vivo*.

In the specific case that component $P^2$ of formula (I) of the present invention is a cationic or polycationic peptide or protein the cationic properties of component $[\text{S-}P^2\text{-S}]_n$ or $\{[\text{S-}P^2\text{-S}]_a/[\text{S-}(AA)_x\text{-S}]_b$ (as defined below) may be determined upon its content of cationic amino acids in the entire component $[\text{S-}P^2\text{-S}]_n$ or $\{[\text{S-}P^2\text{-S}]_a/[\text{S-}(AA)_x\text{-S}]_b\}$. Preferably, the content of cationic amino acids in component $[\text{S-}P^2\text{-S}]_n$ or $\{[\text{S-}P^2\text{-S}]_a/[\text{S-}(AA)_x\text{-S}]_b\}$ is at least 10%, 20%, or 30%, preferably at least 40%, more preferably at least 50%, 60% or 70%, but also preferably at least 80%, 90%, or even 95%, 96%, 97%, 98%, 99% or 100%, most preferably at least 30%, 40%, 50%, 60%, 70%, 80%, 90%, 95%, 96%, 97%, 98%, 99% or 100%, or may be in the range of about 10% to 90%, more preferably in the range of about 15% to 75%, even more preferably in the range of about 20% to 50%, e.g. 20, 30, 40 or 50%, or in a range formed by any two of the afore mentioned values, provided, that the content of all amino acids, e.g. cationic, lipophilic, hydrophilic, aromatic and further amino acids, in the entire component $[\text{S-}P^2\text{-S}]_n$ or $\{[\text{S-}P^2\text{-S}]_a/[\text{S-}(AA)_x\text{-S}]_b\}$ is 100%.

In the specific case that component $P^2$ of formula (I) of the present invention is a cationic or polycationic polymer the cationic properties of component $[\text{S-}P^2\text{-S}]_n$ or $\{[\text{S-}P^2\text{-S}]_a/[\text{S-}(AA)X\text{-S}]_b\}$ may be determined upon its content of cationic charges in the entire component $[\text{S-}P^2\text{-S}]_n$ or $\{[\text{S-}P^2\text{-S}]_a/[\text{S-}(AA)_x\text{-S}]_b\}$ when compared to the overall charges of component $[\text{S-}P^2\text{-S}]_n$ or $\{[\text{S-}P^2\text{-S}]_a/[\text{S-}(AA)_x\text{-S}]_b\}$. Preferably, the content of cationic charges in component $[\text{S-}P^2\text{-S}]_n$ or $\{[\text{S-}P^2\text{-S}]_a/[\text{S-}(AA)_x\text{-S}]_b\}$ at a (physiological) pH as defined herein is at least 10%, 20%, or 30%, preferably at least 40%, more preferably at least 50%, 60% or 70%, but also preferably at least 80%, 90%, or even 95%, 96%, 97%, 98%, 99% or 100%, most preferably at least 30%, 40%, 50%, 60%, 70%, 80%, 90%, 95%, 96%, 97%, 98%, 99% or 100%, or may be in the range of about 10% to 90%, more preferably in the range of about 15% to 75%, even preferably in the range of about 20% to 50%, e.g. 20, 30, 40 or 50%, or in a range formed by any two of the afore mentioned values, provided, that the content of all charges, e.g. positive and negative charges at a (physiological) pH as defined herein, in the entire component $[\text{S-}P^2\text{-S}]_n$ or $\{[\text{S-}P^2\text{-S}]_a/[\text{S-}(AA)_x\text{-S}]_b\}$ is 100%.

In the context of the polymeric carrier cargo complex (=nanoparticle) formed by the nucleic acid cargo and a polymeric carrier molecule according to generic formula (I) $\text{L-}P^1\text{-S-}[\text{S-}P^2\text{-S}]_n\text{-S-}P^3\text{-L}$ as defined herein (or according to any of its subformulas herein) it is particularly preferred that at least 10% of all charges in the whole repetitive component $[\text{S-}P^2\text{-S}]_n$ or $\{[\text{S-}P^2\text{-S}]_a/[\text{S-}(AA)_x\text{-S}]_b\}$ are cationic to allow complexation of the negatively charged nucleic acid cargo.

The cationic or polycationic peptide or protein as component $P^2$, or the cationic or polycationic polymer as component $P^2$ according to generic formula (I), is preferably a linear molecule, however, branched cationic or polycationic peptides or proteins as component $P^2$ or branched cationic or polycationic polymers as component $P^2$ may also be used.

Typically, component $P^2$, e.g. the cationic or polycationic peptide or protein or the cationic or polycationic polymer as defined herein, is linked to its neighboring components, e.g. components $P^1$ and $P^3$, and/or as a part of repetitive component $[\text{S-}P^2\text{-S}]_n$ to further components $P^2$, via disulfide bonds (-S-S-) or to $(AA)_x$ components as part of $\{[\text{S-}P^2\text{-S}]_a/[\text{S-}(AA)_x\text{-S}]_b\}$- In this context, the sulfurs adjacent to component $P^2$ in the repetitive component $[\text{S-}P^2\text{-S}]_n$ and as defined in generic formula (I) $\text{L-}P^1\text{-S-}[\text{S-}P^2\text{-S}]_n\text{-S-}P^3\text{-L}$, necessary for providing a disulfide bond, may be provided by

component $P^2$ itself by a -SH moiety as defined herein or may be provided by modifying component $P^2$ accordingly to exhibit a -SH moiety within the above definition of repetitive component $[S-P^2-S]_n$. The -SH moieties for component $P^2$ are preferably as defined herein for components $P^1$ and $P^3$. If such -SH-moieties, necessary to form a disulfide bond (-S-S-) within the above meaning, are provided by component $P^2$ itself this may occur e.g. by at least two cysteines or any further (modified) amino acids or chemical compounds, which carry a -SH moiety, already occurring within the amino acid sequence of component $P^2$ at whatever position of the amino acid sequence of component $P^2$. Alternatively, component $P^2$ may be modified accordingly with a chemical compound, e.g. a cysteine or any further (modified) amino acid or chemical compound, which carries a (free) -SH moiety. Thereby, component $P^2$ preferably carries at least two -SH-moieties, which sulphurs atoms are capable to form a disulfide bond upon condensation with a-SH-moiety of components $P^1$ or $P^3$ as defined herein, or between a first component $P^2$ and a further component $P^2$, etc. Such - SH-moieties are preferably as defined herein. Preferably the at least two SH-moieties are located at the teminal ends or near to the terminal ends of component $P^2$

According to one specific aspect of the first embodiment of the present invention, component $P^2$ within repetitive component $[S-P^2-S]_n$ of generic formula (I) above may comprise a cysteine as a -SH moiety. In this context, repetitive component $[S-P^2-S]_n$ may thus be written as follows:

$$[Cys-P^2-Cys]_n$$

wherein n and $P^2$ are as defined herein and each Cys provides for the -SH-moiety for the disulfide bond. Cys is the amino acid cysteine in its three letter code. (For illustrative purposes, in the present description the disulfide bond -S-S- generally may also be written as -(Cys-S)-(S-Cys)-, wherein Cys-S represents a Cysteine with an naturally occurring -SH moiety, wherein this -SH moiety forms a disulfide bond with a -SH moiety of a second cysteine. Accordingly, repetitive component $[Cys-P^2-Cys]_n$ may also be written as $[(S-Cys)-P^2-(Cys-S)]_n$, which indicates that the -SH-moiety is provided by a cysteine and the Cysteine itself provides for the sulfur of the disulfide bond.)

In the context of the entire formula (I) above, this specific aspect of the first embodiment thus may be defined as follows:

$$L-P^1-S-[Cys-P^2-Cys]_n-S-P^3-L$$

wherein L, $P^1$, $P^2$, $P^3$ and n are as defined herein, S is sulphur and each Cys provides for one - SH-moiety for the disulfide bond.

In each case, the SH-moiety, e.g. of a cysteine or any further (modified) amino acid or further compound used for modification of component $P^2$, may be present in the cationic or polycationic peptide or protein or cationic or polycationic polymer as component $P^2$, internally or at one or both of its terminal ends, e.g. if a cationic or polycationic peptide or protein is used as component $P^2$ at the N-terminal end or at the C-terminal end, at both these terminal ends, and/or internally at any position of the cationic or polycationic peptide or protein as component $P^2$. Preferably, the -SH moiety may be present in component $P^2$ at least at one terminal end, more preferably at both terminal ends, e.g. at the N-terminal end and/or at the C-terminal end, more preferably at both the N-terminal and the C-terminal end of a cationic or polycationic peptide or protein as component $P^2$.

Due to its repetitive character component $[S-P^2-S]_n$ may represent a situation, wherein one of the at least two -SH-moieties of component $P^2$ was condensed with a -SH-moiety of a further component $P^2$ of generic formula (I) above, wherein both sulphurs of these -SH-moieties form a disulfide bond (-S-S-) between a first component $P^2$ and at least one further component $P^2$.

In this context, the number of repetitions of component $P^2$ in formula (I) according to the present invention is defined by integer n. n is an integer, typically selected from a range of about 1 to 50, preferably from a range of about 1, 2 or 3 to 30, more preferably from a range of about 1, 2, 3, 4, or 5 to 25, or a range of about 1, 2, 3, 4, or 5 to 20, or a range of about 1, 2, 3, 4, or 5 to 15, or a range of about 1, 2, 3, 4, or 5 to 10, including e.g. a range of about 3 to 20, 4 to 20, 5 to 20, or 10 to 20, or a range of about 3 to 15, 4 to 15, 5 to 15, or 10 to 15, or a range of about 6 to 11 or 7 to 10. If, for example, in repetitive component $[S-P^2-S]_n$ integer n is 5, repetitive component $[S-P^2-S]_n$ preferably reads as follows:

$$[S-P^2-S-S-P^2-S-S-P^2-S-S-P^2-S-S-P^2-S]$$

In the above example component $P^2$ occurs 5 times (preferably in a linear order), wherein each component $P^2$ is linked to its neighbor component by a disulfide bond within the above definition of repetitive component $[S-P^2-S]_n$. Any of components $P^2$ may be the same or different from each other.

According to one particular aspect of this embodiment, component $P^2$ represents a cationic or polycationic peptide or protein having a length of about 3 to about 100 amino acids, more preferably having a length of about 3 to about 50 amino acids, even more preferably having a length of about 3 to about 25 amino acids, e.g. having a length of about 3 to 10, 5 to 15, 10 to 20 or 15 to 25 amino acids, more preferably a length of about 5 to about 20 and even more preferably a length of about 10 to about 20.

The cationic or polycationic peptide or protein as component $P^2$ may be any protein or peptide suitable for this purpose and exhibiting at least two -SH-moieties, particular any cationic or polycationic peptide or protein capable to complex a nucleic acid as defined according to the present invention, and thereby preferably condensing the nucleic acid.

Particularly preferred, cationic or polycationic peptides or proteins as component $P^2$ exhibiting at least two -SH-moieties may be selected from protamine, nucleoline, spermine or spermidine, poly-L-lysine (PLL), basic polypeptides, poly-arginine, cell penetrating peptides (CPPs), chimeric CPPs, such as Transportan, or MPG peptides, HIV-binding peptides, Tat, HIV-1 Tat (HIV), Tat-derived peptides, oligoarginines, members of the penetratin family, e.g. Penetratin, Antennapedia-derived peptides (particularly from *Drosophila antennapedia*), pAntp, plsl, etc., antimicrobial-derived CPPs e.g. Buforin-2, Bac715-24, SynB, SynB(1), pVEC, hCT-derived peptides, SAP, MAP, KALA, PpTG20, Proline-rich peptides, Loligomere, Arginine-rich peptides, Calcitonin-peptides, FGF, Lactoferrin, histones, VP22 derived or analog peptides, HSV, VP22 (Herpes simplex), MAP, KALA or protein transduction domains (PTDs, PpT620, prolin-rich peptides, lysine-rich peptides, Pep-1, L-oligomers, Calcitonin peptide(s), etc.

According to one particular preferred aspect of the first embodiment of the present invention, cationic or polycationic peptides or proteins as component $P^2$ are selected from following cationic peptides having the following total sum formula (II), preferably under the provison that they exhibit additionally at least two -SH-moieties:

$$\{(Arg)_l;(Lys)_m;(His)_n;(Orn)_o;(Xaa)_x\}; \qquad \text{(formula (II))}$$

wherein $l + m + n + o + x = 8\text{-}15$, and $l$, $m$, $n$ or $o$ independently of each other may be any number selected from 0, 1, 2, 3, 4, 5, 6, 7, 8, 9, 10, 11, 12, 13, 14 or 15, provided that the overall content of Arg, Lys, His and Orn represents at least 10% of all amino acids of the oligopeptide; and Xaa may be any amino acid selected from native (= naturally occurring) or non-native amino acids except of Arg, Lys, His or Orn; and x may be any number selected from 0, 1, 2, 3, 4, 5, 6, 7, 8, 9, 10, 11, 12, 13 or 14, provided, that the overall content of Xaa does not exceed 90 % of all amino acids of the oligopeptide. Any of amino acids Arg, Lys, His, Orn and Xaa may be positioned at any place of the peptide. Particularly preferred peptides of this formula are oligoarginines such as e.g. $Arg_7$, $Arg_8$, $Arg_9$, $Arg_7$, $H_3R_9$, $R_9H_3$, $H_3R_9H_3$, $YSSR_9SSY$, $(RKH)_4$, $Y(RKH)_2R$, etc. (SEQ ID NOs: 1-9).

According to a particular preferred aspect of the first embodiment, cationic or polycationic peptides or proteins as component $P^2$, having the empirical formula (II) as shown above and additionally exhibiting at least two -SH-moieties, may be, without being restricted thereto, selected from the following subgroup of formulae:

$Arg_8$, $Arg_9$, $Arg_{10}$, $Arg_{11}$, $Arg_{12}$, $Arg_{13}$, $Arg_{14}$, $Arg_{15}$; (SEQ ID NOs: 2-3, 10-15);
$Lys_8$, $Lys_9$, $Lys_{10}$, $Lys_{11}$, $Lys_{12}$, $Lys_{13}$, $Lys_{14}$, $Lys_{15}$; (SEQ ID NOs: 16-23);
$His_8$, $His_9$, $His_{10}$, $His_{11}$, $His_{12}$, $His_{13}$, $His_{14}$, $His_{15}$; (SEQ ID NOs: 24-31);
$Orn_8$, $Orn_9$, $Orn_{10}$, $Orn_{11}$, $Orn_{12}$, $Orn_{13}$, $Orn_{14}$, $Orn_{15}$; (SEQ ID NOs: 32-39);

According to a further particularly preferred aspect of the first embodiment, cationic or polycationic peptides or proteins as component $P^2$, having the empirical formula (II) as shown above and additionally exhibiting at least two -SH-moieties, may be, without being restricted thereto, selected from the following subgroup of formulae, wherein the following formulae (as with empirical formula (II)) do not specify any amino acid order, but are intended to reflect empirical formulae by exclusively specifying the (number of) amino acids as components of the respective peptide. Accordingly, as an example, empirical formula $Arg_{(7-14)}Lys_1$ is intended to mean that peptides falling under this formula contain 7 to 14 Arg residues and 1 Lys residue of whatsoever order. If the peptides contain 7 Arg residues and 1 Lys residue, all variants having 7 Arg residues and 1 Lys residue are encompassed. The Lys residue may therefore be positioned anywhere in the e.g. 8 amino acid long sequence composed of 7 Arg and 1 Lys residues. The subgroup preferably comprises:

$Arg_{(7-14)}Lys_1$, $Arg_{(7-14)}His_1$, $Arg_{(7-14)}Orn_1$, $Lys_{(7-14)}His_1$, $Lys_{(7-14)}Orn_1$, $His_{(7-14)}Orn_1$,;

$Arg_{(6-13)}Lys_2$, $Arg_{(6-13)}His_2$, $Arg_{(6-13)}Orn_2$, $Lys_{(6-13)}His_2$, $Lys_{(6-13)}Orn_2$, $His_{(6-13)}Orn_2$,;

$Arg_{(5-12)}Lys_3$, $Arg_{(5-12)}His_3$, $Arg_{(5-12)}Orn_3$, $Lys_{(5-12)}His_3$, $Lys_{(5-12)}Orn_3$, $His_{(5-12)}Orn_3$,;

$Arg_{(4-11)}Lys_4$, $Arg_{(4-11)}His_4$, $Arg_{(4-11)}Orn_4$, $Lys_{(4-11)}His_4$, $Lys_{(4-11)}Orn_4$, $His_{(4-11)}Orn_4$,;

$Arg_{(3-10)}Lys_5$, $Arg_{(3-10)}His_5$, $Arg_{(3-10)}Orn_5$, $Lys_{(3-40)}His_5$, $Lys_{(3-10)}Orn_5$, $His_{(3-10)}Orn_5$,;

$Arg_{(2-9)}Lys_6$, $Arg_{(2-9)}His_6$, $Arg_{(2-9)}Orn_6$, $Lys_{(2-9)}His_6$, $Lys_{(2-9)}Orn_6$, $His_{(2-9)}Orn_6$,;

$Arg_{(1-8)}Lys_7$, $Arg_{(1-8)}His_7$, $Arg_{(1-8)}Orn_7$, $Lys_{(1-8)}His_7$, $Lys_{(1-8)}Orn_7$, $His_{(1-8)}Orn_7$,;

$Arg_{(6-13)}Lys_1His_1$, $Arg_{(6-13)}Lys_1Orn_1$, $Arg_{(6-13)}His_1Orn_1$, $Arg_1Lys_{(6-13)}His_1$, $Arg_1Lys_{(6-13)}Orn_1$, $Lys_{(6-13)}His_1Orn_1$, $Arg_1Lys_1His_{(6-13)}$, $Arg_1His_{(6-13)}Orn_1$, $Lys_1His_{(6-13)}Orn_1$;

$Arg_{(5-12)}Lys_2His_1$, $Arg_{(5-12)}Lys_1His_2$, $Arg_{(5-12)}Lys_2Orn_1$, $Arg_{(5-12)}Lys_1Orn_2$, $Arg_{(5-12)}His_2Orn_1$, $Arg_{(5-12)}HiS_1Orn_2$, $Arg_2Lys_{(5-12)}His_1$, $Arg_1Lys_{(5-12)}HiS_2$, $Arg_2Lys_{(5-12)}Orn_1$, $Arg_1Lys_{(5-12)}Orn_2$, $Lys_{(5-12)}His_2Orn_1$, $Lys_{(5-12)}His_1Orn_2$, $Arg_2Lys_1His_{(5-12)}$, $Arg_1Lys_2His_{(5-12)}$, $Arg_2His_{(5-12)}Orn_1$, $Arg_1His_{(5-12)}Orn_2$, $Lys_2His_{(5-12)}Orn_1$, $Lys_1His_{(5-12)}Orn_2$;

$Arg_{(4-11)}Lys_3His_1$, $Arg_{(4-11)}Lys_2His_2$, $Arg_{(4-11)}Lys_1His_3$, $Arg_{(4-11)}Lys_3Orn_1$, $Arg_{(4-1)}Lys_2Orn_2$, $Arg_{(4-11)}Lys_1Orn_3$, $Arg_{(4-11)}His_3Orn_1$, $Arg_{(4-11)}His_2Orn_2$, $Arg_{(4-11)}His_1Orn_3$, $Arg_3Lys_{(4-11)}His_1$, $Arg_2Lys_{(4-11)}His_2$, $Arg_1Lys_{(4-11)}His_3$, $Arg_3Lys_{(4-11)}Orn_1$, $Arg_2Lys_{(4-11)}Orn_2$, $Arg_1Lys_{(4-11)}Orn_3$, $Lys_{(4-11)}His_3Orn_1$, $Lys_{(4-11)}His_2Orn_2$, $Lys_{(4-11)}His_1Orn_3$, $Arg_3Lys_1His_{(4-11)}$, $Arg_2Lys_2His_{(4-11)}$, $Arg_1Lys_3His_{(4-11)}$, $Arg_3His_{(4-11)}Orn_1$, $Arg_2His_{(4-11)}Orn_2$, $Arg_1His_{(4-11)}Orn_3$, $Lys_3His_{(4-11)}Orn_1$, $Lys_2His_{(4-11)}Orn_2$, $Lys_1His_{(4-11)}Orn_3$;

$Arg_{(3-10)}Lys_4His_1$, $Arg_{(3-10)}Lys_3His_2$, $Arg_{(3-10)}Lys_2His_3$, $Arg_{(3-10)}Lys_1His_4$, $Arg_{(3-10)}Lys_4Orn_1$, $Arg_{(3-10)}Lys_3Orn_2$, $Arg_{(3-10)}Lys_2Orn_3$, $Arg_{(3-10)}Lys_1Orn_4$, $Arg_{(3-10)}His_4Orn_1$, $Arg_{(3-10)}His_3Orn_2$, $Arg_{(3-10)}His_2Orn_3$, $Arg_{(3-10)}His_1Orn_4$, $Arg_4Lys_{(3-10)}His_1$, $Arg_3Lys_{(3-10)}His_2$, $Arg_2Lys_{(3-10)}His_3$, $Arg_1Lys_{(3-10)}His_4$, $Arg_4Lys_{(3-10)}Orn_1$, $Arg_3Lys_{(3-10)}Orn_2$, $Arg_2Lys_{(3-10)}Orn_3$, $Arg_1Lys_{(3-10)}Orn_4$, $Lys_{(3-10)}His_4Orn_1$, $Lys_{(3-10)}His_3Orn_2$, $Lys_{(3-10)}His_2Orn_3$, $Lys_{(3-10)}His_1Orn_4$, $Arg_4Lys_1His_{(3-10)}$, $Arg_3Lys_2His_{(3-10)}$, $Arg_2Lys_3His_{(3-10)}$, $Arg_1Lys_4His_{(3-10)}$, $Arg_4His_{(3-10)}Orn_1$, $Arg_3His_{(3-10)}Orn_2$, $Arg_2His_{(3-10)}Orn_3$, $Arg_1His_{(3-10)}Orn_4$, $Lys_4His_{(3-10)}Orn_1$, $Lys_3His_{(3-10)}Orn_2$, $Lys_2His_{(3-10)}Orn_3$, $Lys_1His_{(3-10)}Orn_4$;

$Arg_{(2-9)}Lys_5His_1$, $Arg_{(2-9)}Lys_4His_2$, $Arg_{(2-9)}Lys_3His_3$, $Arg_{(2-9)}Lys_2His_4$, $Arg_{(2-9)}Lys_1His_5$, $Arg_{(2-9)}Lys_5Orn_1$, $Arg_{(2-9)}Lys_4Orn2$, $Arg_{(2-9)}Lys_3Orn_3$, $Arg_{(2-9)}Lys_2Orn_4$, $Arg_{(2-9)}Lys_1Orn_5$, $Arg_{(2-9)}His_5Orn_1$, $Arg_{(2-9)}His_4Orn_2$, $Arg_{(2-9)}His_3Orn_3$, $Arg_{(2-9)}His_2Orn_4$, $Arg_{(2-9)}His_1Orn_5$, $Arg_5Lys_{(2-9)}His_1$, $Arg_4Lys_{(2-9)}His_2$, $Arg_3Lys_{(2-9)}His_3$, $Arg_2Lys_{(2-9)}His_4$, $Arg_1Lys_{(2-9)}His_5$, $Arg_5Lys_{(2-9)}Orn_1$, $Arg_4Lys_{(2-9)}Orn_2$, $Arg_3Lys_{(2-9)}Orn_3$, $Arg_2Lys_{(2-9)}Orn_4$, $Arg_1Lys_{(2-9)}Orn_5$, $Lys_{(2-9)}His_5Orn_1$, $Lys_{(2-9)}His_4Orn_2$, $Lys_{(2-9)}His_3Orn_3$, $Lys_{(2-9)}His_2Orn_4$, $Lys_{(2-9)}His_1Orn_5$, $Arg_5Lys_1His_{(2-9)}$, $Arg_4Lys_2His_{(2-9)}$, $Arg_3Lys_3His_{(2-9)}$, $Arg_2Lys_4His_{(2-9)}$, $Arg_1Lys_5His_{(2-9)}$, $Arg_5His_{(2-9)}Orn_1$, $Arg_4His_{(2-9)}Orn_2$, $Arg_3His_{(2-9)}Orn_3$, $Arg_2His_{(2-9)}Orn_4$, $Arg_1His_{(2-9)}Orn_5$, $Lys_5His_{(2-9)}Orn_1$, $Lys_4His_{(2-9)}Orn_2$, $Lys_3His_{(2-9)}Orn_3$, $Lys_2His_{(2-9)}Orn_4$, $Lys_1His_{(2-9)}Orn_5$;

$Arg_{(1-8)}Lys_6His_1$, $Arg_{(1-8)}Lys_5His_2$, $Arg_{(1-8)}Lys_4His_3$, $Arg_{(1-8)}Lys_3His_4$, $Arg_{(1-8)}Lys_2His_5$, $Arg_{(1-8)}Lys_1His_6$, $Arg_{(1-8)}Lys_6Orn_1$, $Arg_{(1-8)}Lys_5Orn_2$, $Arg_{(1-8)}Lys_4Orn_3$, $Arg_{(1-8)}Lys_3Orn_4$, $Arg_{(1-8)}Lys_2Orn_5$, $Arg_{(1-8)}Lys_1Orn_6$, $Arg_{(1-8)}His_6Orn_1$, $Arg_{(1-8)}His_5Orn_2$, $Arg_{(1-8)}His_4Orn_3$, $Arg_{(1-8)}His_3Orn_4$, $Arg_{(1-8)}His_2Orn_5$, $Arg_{(1-8)}His_1Orn_6$, $Arg_6Lys_{(1-8)}His_1$, $Arg_5Lys_{(1-8)}His_2$, $Arg_4Lys_{(1-8)}His_3$, $Arg_3Lys_{(1-8)}His_4$, $Arg_2Lys_{(1-8)}His_5$, $Arg_1Lys_{(1-8)}His_6$, $Arg_6Lys_{(1-8)}Orn_1$, $Arg_5Lys_{(1-8)}Orn_2$, $Arg_4Lys_{(1-8)}Orn_3$, $Arg_3Lys_{(1-8)}Orn_4$, $Arg_2Lys_{(1-8)}Orn_5$, $Arg_1Lys_{(1-8)}Orn_6$, $Lys_{(1-8)}His_6Orn_1$, $Lys_{(1-8)}His_5Orn_2$, $Lys_{(1-8)}His_4Orn_3$, $Lys_{(1-8)}His_3Orn_4$, $Lys_{(1-8)}His_2Orn_5$, $Lys_{(1-8)}His_1Orn_6$, $Arg_6Lys_1His_{(1-8)}$, $Arg_5Lys_2His_{(1-8)}$, $Arg_4Lys_3His_{(1-8)}$, $Arg_3Lys_4His_{(1-8)}$, $Arg_2Lys_5His_{(1-8)}$, $Arg_1Lys_6His_{(1-8)}$, $Arg_6His_{(1-8)}Orn_1$, $Arg_5His_{(1-8)}Orn_2$, $Arg_4His_{(1-8)}Orn_3$, $Arg_3His_{(1-8)}Orn_4$, $Arg_2His_{(1-8)}Orn_5$, $Arg_1His_{(1-8)}Orn_6$, $Lys_6His_{(1-8)}Orn_1$, $Lys_5His_{(1-8)}Orn_2$, $Lys_4His_{(1-8)}Orn_3$, $Lys_3His_{(1-8)}Orn_4$, $Lys_2His_{(1-8)}Orn_5$, $Lys_1His_{(1-8)}Orn_6$;

$Arg_{(5-12)}Lys_1His_1Orn_1$, $Arg_1Lys_{(5-12)}His_1Orn_1$, $Arg_1Lys_1His_{(5-12)}Orn_1$, $Arg_1Lys_1His_1Orn_{(5-12)}$;

$Arg_{(4-11)}Lys_2His_1Orn_1$, $Arg_{(4-11)}Lys_1His_2Orn_1$, $Arg_{(4-11)}Lys_1His_1Orn_2$, $Arg_2Lys_{(4-11)}His_1Orn_1$, $Arg_1Lys_{(4-11)}His_2Orn_1$, $Arg_1Lys_{(4-11)}His_1Orn_2$, $Arg_2Lys_1His_{(4-11)}Orn_1$, $Arg_1Lys_2His_{(4-11)}Orn_1$, $Arg_1Lys_1His_{(4-11)}Orn_2$, $Arg_2Lys_1His_1Orn_{(4-11)}$, $Arg_1Lys_2His_1Orn_{(4-11)}$, $Arg_1Lys_1His_2Orn_{(4-11)}$;

$Arg_{(3-10)}Lys_3His_1Orn_1$, $Arg_{(3-10)}Lys_2His_2Orn_1$, $Arg_{(3-10)}Lys_2His_1Orn_2$, $Arg_{(3-10)}Lys_1His_2Orn_2$, $Arg_{(3-10)}Lys_1His_1Orn_3$, $Arg_3Lys_{(3-10)}His_1Orn_1$, $Arg_2Lys_{(3-10)}His_2Orn_1$, $Arg_2Lys_{(3-10)}His_1Orn_2$, $Arg_1Lys_{(3-10)}His_2Orn_2$, $Arg_1Lys_{(3-10)}His_1Orn_3$, $Arg_3Lys_1His_{(3-10)}Orn_1$, $Arg_2Lys_2His_{(3-10)}Orn_1$, $Arg_2Lys_1His_{(3-10)}Orn_2$, $Arg_1Lys_2His_{(3-10)}Orn_2$, $Arg_1Lys_1His_{(3-10)}Orn_3$, $Arg_3Lys_1His_1Orn_{(3-10)}$, $Arg_2Lys_2His_1Orn_{(3-10)}$, $Arg_2Lys_1His_2Orn_{(3-10)}$, $Arg_1Lys_2His_2Orn_{(3-10)}$, $Arg_1Lys_1His_3Orn_{(3-10)}$;

$Arg_{(2-9)}Lys_4His_1Orn_1$, $Arg_{(2-9)}Lys_1His_4Orn_1$, $Arg_{(2-9)}Lys_1His_1Orn_4$, $Arg_{(2-9)}Lys_3His_2Orn_1$, $Arg_{(2-9)}Lys_3His_1Orn_2$, $Arg_{(2-9)}Lys_2His_3Orn_1$, $Arg_{(2-9)}Lys_2His_1Orn_3$, $Arg_{(2-9)}Lys_1His_2Orn_3$, $Arg_{(2-9)}Lys_1His_3Orn_2$, $Arg_{(2-9)}Lys_2His_2Orn_2$, $Arg_4Lys_{(2-9)}His_1Orn_1$, $Arg_1Lys_{(2-9)}His_4Orn_1$, $Arg_1Lys_{(2-9)}His_1Orn_4$, $Arg_3Lys_{(2-9)}His_2Orn_1$, $Arg_3Lys_{(2-9)}His_1Orn_2$, $Arg_2Lys_{(2-9)}His_3Orn_1$, $Arg_2Lys_{(2-9)}His_1Orn_3$, $Arg_1Lys_{(2-9)}His_2Orn_3$, $Arg_1Lys_{(2-9)}His_3Orn_2$, $Arg_2Lys_{(2-9)}His_2Orn_2$,

$Arg_4Lys_1His_{(2-9)}Orn_1$, $Arg_1Lys_4His_{(2-9)}Orn_1$, $Arg_1Lys_1His_{(2-9)}Orn_4$, $Arg_3Lys_2His_{(2-9)}Orn_1$, $Arg_3Lys_1His_{(2-9)}Orn_2$, $Arg_2Lys_3His_{(2-9)}Orn_1$, $Arg_2Lys_1His_{(2-9)}Orn_3$, $Arg_1Lys_2His_{(2-9)}Orn_3$, $Arg_1Lys_3His_{(2-9)}Orn_2$; $Arg_2Lys_2His_{(2-9)}Orn_2$, $Arg_4Lys_1His_1Orn_{(2-9)}$, $Arg_1Lys_4His_1Orn_{(2-9)}$, $Arg_1Lys_1His_4Orn_{(2-9)}$, $Arg_3Lys_2His_1Orn_{(2-9)}$, $Arg_3Lys_1His_2Orn_{(2-9)}$, $Arg_2Lys_3His_1Orn_{(2-9)}$, $Arg_2Lys_1His_3Orn_{(2-9)}$, $Arg_1Lys_2His_3Orn_{(2-9)}$, $Arg_1Lys_3His_2Orn_{(2-9)}$, $Arg_2Lys_2His_2Orn_{(2-9)}$;

$Arg_{(1-8)}Lys_5His_1Orn_1$, $Arg_{(1-8)}Lys_1His_5Orn_1$, $Arg_{(1-8)}Lys_1His_1Orn_5$, $Arg_{(1-8)}Lys_4His_2Orn_1$, $Arg_{(1-8)}Lys_2His_4Orn_1$, $Arg_{(1-8)}Lys_2His_1Orn_4$, $Arg_{(1-8)}Lys_1His_2Orn_4$, $Arg_{(1-8)}Lys_1His_4Orn_2$, $Arg_{(1-8)}Lys_4His_1Orn_2$, $Arg_{(1-8)}Lys_3His_3Orn_1$, $Arg_{(1-8)}Lys_3His_1Orn_3$, $Arg_{(1-8)}Lys_1His_3Orn_3$, $Arg_5Lys_{(1-8)}His_1Orn_1$, $Arg_1Lys_{(1-8)}His_5Orn_1$, $Arg_1Lys_{(1-8)}His_1Orn_5$, $Arg_4Lys_{(1-8)}His_2Orn_1$, $Arg_2Lys_{(1-8)}His_4Orn_1$, $Arg_2Lys_{(1-8)}His_1Orn_4$, $Arg_1Lys_{(1-8)}His_2Orn_4$, $Arg_1Lys_{(1-8)}His_4Orn_2$, $Arg_4Lys_{(1-8)}His_1Orn_2$, $Arg_3Lys_{(1-8)}His_3Orn_1$, $Arg_3Lys_{(1-8)}His_1Orn_3$, $Arg_1Lys_{(1-8)}His_3Orn_3$, $Arg_5Lys_1His_{(1-8)}Orn_1$, $Arg_1Lys_5His_{(1-8)}Orn_1$, $Arg_1Lys_1His_{(1-8)}Orn_5$, $Arg_4Lys_2His_{(1-8)}Orn_1$, $Arg_2Lys_4His_{(1-8)}Orn_1$, $Arg_2Lys_1His_{(1-8)}Orn_4$, $Arg_1Lys_2His_{(1-8)}Orn_4$, $Arg_1Lys_4His_{(1-8)}Orn_2$, $Arg_4Lys_1His_{(1-8)}Orn_2$, $Arg_3Lys_3His_{(1-8)}Orn_1$, $Arg_3Lys_1His_{(1-8)}Orn_3$, $Arg_1Lys_3His_{(1-8)}Orn_3$, $Arg_5Lys_1His_1Orn_{(1-8)}$, $Arg_1Lys_5His_1Orn_{(1-8)}$, $Arg_1Lys_1His_5Orn_{(1-8)}$, $Arg_4Lys_2His_1Orn_{(1-8)}$, $Arg_2Lys_4His_1Orn_{(1-8)}$, $Arg_2Lys_1His_4Orn_{(1-8)}$, $Arg_1Lys_2His_4Orn_{(1-8)}$, $Arg_1Lys_4His_2Orn_{(1-8)}$, $Arg_4Lys_1His_2Orn_{(1-8)}$, $Arg_3Lys_3His_1Orn_{(1-8)}$, $Arg_3Lys_1His_3Orn_{(1-8)}$, $Arg_1Lys_3His_3Orn_{(1-8)}$;

According to another particular preferred aspect of the first embodiment, cationic or polycationic peptides or proteins as component $P^2$, having the empirical formula (II) as shown above, and additionally exhibiting at least two -SH-moieties may be, without being restricted thereto, selected from following formulae: $Arg_8$, $Arg_9$, $Arg_{10}$, $Arg_{11}$, $Arg_{12}$, $Arg_{13}$, $Arg_{14}$, $Arg_{15}$; $Lys_8$, $Lys_9$, $Lys_{10}$, $Lys_{11}$, $Lys_{12}$, $Lys_{13}$, $Lys_{14}$, $Lys_{15}$; $His_8$, $His_9$, $His_{10}$, $His_{11}$, $His_{12}$, $His_{13}$, $His_{14}$, $His_{15}$; $Orn_8$, $Orn_9$, $Orn_{10}$, $Orn_{11}$, $Orn_{12}$, $Orn_{13}$, $Orn_{14}$, $Orn_{15}$; (SEQ ID NOs: 2-3, 10-39, see above).

According to a further particular preferred aspect of the first embodiment, cationic or polycationic peptides or proteins as component $P^2$, having the empirical formula (II) as shown above, and additionally exhibiting at least two -SH-moieties may be, without being restricted thereto, selected from the subgroup consisting of generic formulas $Arg_9$ (also termed $R_9$), $Arg_9His_3$ (also termed $R_9H_3$), $His_3Arg_9His_3$ (also termed $H_3R_9H_3$), TyrSerSerArg9SerSerTyr (also termed $YSSR_9SSY$), $His_3Arg_9SerSerTyr$ (also termed $H_3R_9SSY$), $(ArgLysHis)_4$ (also termed $(RKH)_4$), Tyr(ArgLysHis)$_2$Arg (also termed $Y(RKH)_2R$); (SEQ ID NOs: 2, 5-9, 40, see above).

According to a one further particular preferred aspect of the first embodiment, the cationic or polycationic peptide or protein as component $P^2$, when defined according to formula $\{(Arg)_l;(Lys)_m;(His)_n;(Orn)_o;(Xaa)_x\}$ (formula (II)) as shown above, and additionally exhibiting at least two -SH-moieties may be, without being restricted thereto, selected from formula (IIa), preferably under the provision that at least one -SH-moiety is provided by a cysteine residue:

$$\{(Arg)_l;(Lys)_m;(His)_n;(Orn)_o;(Cys)_x\} \qquad \text{(formula (IIa))}$$

wherein $(Arg)_l;(Lys)_m;(His)_n;(Orn)_o$; and x are as defined herein,

Alternatively, the cationic or polycationic peptide or protein as component $P^2$, when defined according to formula $\{(Arg)_l;(Lys)_m;(His)_n;(Orn)_o;(Xaa)_x\}$ (formula (II)) as shown above, and additionally exhibiting at least two -SH-moieties may be, without being restricted thereto, selected from formula (IIa'), preferably under the provision that at least one -SH-moiety is provided by a cysteine residue:

$$\{(Arg)_l;(Lys)_m;(His)_n;(Orn)_o;(Xaa')_x;(Cys)_y\} \qquad \text{(formula (IIa'))}$$

wherein $(Arg)_l;(Lys)_m;(His)_n;(Orn)_o$; and x are as defined herein, Xaa' is any amino acid selected from native (= naturally occurring) or non-native amino acids except of Arg, Lys, His, Orn or Cys and y is any number selected from 1, 2, 3, 4, 5, 6, 7, 8, 9, 10, 11, 12, 13, 14, 15, 16, 17, 18, 19, 20, 21-30, 31-40, 41-50, 51-60, 61-70, 71-80 and 81-90, provided that the overall content of Arg (Arginine), Lys (Lysine), His (Histidine) and Orn (Ornithine) represents at least 10% of all amino acids of the oligopeptide.

These aspects of the first embodiment of the present invention may apply to situations, wherein component $P^2$ is selected from a cationic or polycationic peptide or protein according to empirical formula $(Arg)_l;(Lys)_m;(His)_n;(Orn)_o;(Xaa)_x$ (formula (II)) as shown above, which comprises or has been modified with at least one cysteine as -SH moiety in the above meaning such that the cationic or polycationic peptide as component $P^2$ carries at least one cysteine, which is capable to form a disulfide bond with other components of formula (I).

According to another particular preferred aspect of the first embodiment, the cationic or polycationic peptide or protein as component $P^2$, when defined according to formula $\{(Arg)_l;(Lys)_m;(His)_n;(Orn)_o;(Xaa)_x\}$ (formula (II)) as shown above, and preferably additionally exhibiting at least two -SH-moieties may be, without being restricted thereto, selected from formula (IIb), preferably under the provision that the at least two -SH-moieties are provided by two terminal cysteine residues:

Cys {(Arg)$_l$;(Lys)$_m$;(His)$_n$;(Orn)$_o$;(Xaa)$_x$} Cys         (formula (IIb))

wherein (Arg)$_l$;(Lys)$_m$;(His)$_n$;(Orn)$_o$;(Xaa)$_x$ are as defined herein and form a core of amino acids according to (semiempirical) formula (II). Exemplary examples may comprise any of the above sequences flanked by two Cys and following sequences:

Cys(Arg8)Cys, Cys(Arg$_9$)Cys, Cys(Arg$_{10}$)Cys, Cys(Arg$_{11}$)Cys, Cys(Arg$_{12}$)Cys, Cys(Arg$_{13}$)Cys, Cys(Arg$_{14}$)Cys, Cys(Arg$_{15}$)Cys; Cys(Lys$_8$)Cys, Cys(Lys$_9$)Cys, Cys(Lys$_{10}$)Cys, Cys(Lys$_{11}$)Cys, Cys(Lys$_{12}$)Cys, Cys(Lys$_{13}$)Cys, Cys(Lys$_{14}$)Cys, Cys(Lys$_{15}$)Cys; Cys(His$_8$)Cys, Cys(His$_9$)Cys, Cys(His$_{10}$)Cys, Cys(His$_{11}$)Cys, Cys(His$_{12}$)Cys, Cys(His$_{13}$)Cys, Cys(His$_{14}$)Cys, Cys(His$_{15}$)Cys; Cys(Orn$_8$)Cys, Cys(Orn$_9$)Cys, Cys(Orn$_{10}$)Cys, Cys(Orn$_{11}$)Cys, Cys(Orn$_{12}$)Cys, Cys(Orn$_{13}$)Cys, Cys(Orn$_{14}$)Cys, Cys(Orn$_{15}$)Cys, (SEQ ID NOs: 41 to 72) more preferably following exemplary sequences (SEQ ID NOs: 73 to 84):

| | |
|---|---|
| CysArg₉Cys: | Cys-Arg-Arg-Arg-Arg-Arg-Arg-Arg-Arg-Cys-Cys |
| CysArg₉His₃Cys: | Cys-Arg-Arg-Arg-Arg-Arg-Arg-Arg-Arg-His-His-His-Cys |
| CysHis₃Arg₉His₃Cys: | Cys-His-His-His-Arg-Arg-Arg-Arg-Arg-Arg-Arg-Arg-His-His-His-Cys |
| CysTyrSerSerArg₉SerSerTyrCys: | Cys-Tyr-Ser-Ser-Arg-Arg-Arg-Arg-Arg-Arg-Arg-Arg-Ser-Ser-Tyr-Cys |
| CysHis₃Arg₉SerSerTyrCys: | Cys-His-His-His-Arg-Arg-Arg-Arg-Arg-Arg-Arg-Arg-Ser-Ser-Tyr-Cys |
| Cys (ArgLysHis)₄Cys: | Cys-Arg-Lys-His-Arg-Lys-His-Arg-Lys-His-Arg-Lys-His-Cys |
| CysTyr(ArgLysHis)₂ArgCys: | Cys-Tyr-Arg-Lys-His-Arg-Lys-His-Arg-Cys |
| CysHis₃Arg₉His₃Cys: | Cys-His-His-His-Arg-Arg-Arg-His-His-His-Cys |
| CysHis₆Arg₉His₆Cys: | Cys-His-His-His-His-His-His-Arg-Arg-Arg-Arg-Arg-Arg-Arg-Arg-Arg-His-His-His-His-His-His-Cys |
| CysHis₃Arg₄His₃Cys: | Cys-H is-H i s-H is-Arg-Arg-Arg-Arg-H is-H i s-H i s-Cys |
| CysHis₆Arg₄His₆Cys | Cys-His-His-His-His-His-His-Arg-Arg-Arg-Arg-His-His-His-His-His-His-Cys |
| CysArg₁₂Cys: | Cys-Arg-Arg-Arg-Arg-Arg Arg-Arg-Arg-Arg-Arg-Arg-Arg -Cys |

This aspect of the first embodiment of the present invention may apply to situations, wherein the polycationic peptide or protein as component $P^2$, e.g. when defined according to empirical formula $(Arg)_l;(Lys)_m;(His)_n;(Orn)_o;(Xaa)_x$ (formula (II)) as shown above, has been modified with at least two (terminal) cysteines as -SH moieties in the above meaning such that component $P^2$ carries at least two (terminal) cysteines, which are capable to form a disulfide bond with other components of formula (I).

According to another aspect of the first embodiment, component $P^2$ represents a cationic or polycationic polymer, selected from e.g. any cationic polymer suitable in this context, provided that this cationic polymer exhibits at least two -SH-moieties, which provide for a disulfide bond linking component $P^2$ with component $P^1$ or $P^3$, or with further component(s) $P^2$ or amino acid components $(AA)_x$. Thus, likewise as defined herein, component $P^2$ may occur as a repetitive component as defined herein as represented by subformula $[S-P^2-S]_n$ or $\{[S-P^2-S]_a/[S-(AA)_x-S]_b\}$, wherein the same or different cationic or polycationic polymers $P^2$ may be used in said repetitive component.

Preferably, component $P^2$ represents a cationic or polycationic polymer, typically exhibiting a molecular weight of about 0.5 kDa to about 30 kDa, of about 1 kDa to about 30 kDa, of about 5 kDa to about 30 kDa, or a molecular weight of about 10 kDa to about 30 kDa, preferably of about 0.5 kDa to about 30 kDa, more preferably of about 1 kDa to about 20 kDa, and even more preferably of about 1.5 kDa to about 10 kDa. Additionally, the cationic or polycationic polymer as component $P^2$ typically exhibits at least two -SH moieties, which are capable to form a disulfide linkage upon condensation with either components $P^1$ or $P^3$ or with other components $P^2$ or amino acid components $(AA)_x$. as defined herein.

When component $P^2$ represents a cationic or polycationic polymer, such a polymer may be selected from acrylates, modified acrylates, such as pDMAEMA (poly(dimethylaminoethyl methylacrylate)), chitosanes, aziridines or 2-ethyl-2-oxazoline (forming oligo ethylenimines or modifed oligoethylenimines), polymers obtained by reaction of bisacrylates with amines forming oligo beta aminoesters or poly amido amines, or other polymers like polyesters, polycarbonates, etc. Each molecule of these cationic or polycationic polymers typically exhibits at least two -SH-moieties, wherein these at least two -SH-moieties may be introduced into the cationic or polycationic polymer by chemical modifications, e.g. using imonothiolan, 3-thio propionic acid or introduction of -SH-moieties containing amino acids, such as cystein, methionine or any further (modified) amino acid. Such -SH-moieties are preferably as already defined above for components $P^1$, $P^2$ or $P^3$.

Component $P^2$ of formula (I) of the present invention preferably occurs as repetitive component $[-S-P^2-S-]_n$. Such a repetitive component $[S-P^2-S]_n$ may be prepared using at least one or even more of the same or different of the above defined components $P^2$ and polymerizing same in a polymerization condensation reaction via their -SH-moieties.

According to one specific aspect of the first embodiment, such a repetitive component $[S-P^2-S]_n$ may be prepared using at least one or even more of the same or different of the above defined cationic or polycationic peptides or proteins, and polymerizing same in a polymerization condensation reaction via their -SH-moieties. Accordingly, such a repetitive component $[S-P^2-S]_n$ contains a number of at least one or even more of the same or different of the above defined cationic or polycationic proteins or peptides determined by integer n.

According to another specific aspect of the first embodiment, such a repetitive component $[S-P^2-S]_n$ may be prepared using at least one or even more of the same or different of the above defined cationic or polycationic polymers, and polymerizing same in a polymerization condensation reaction via their -SH-moieties. Accordingly, such a repetitive component $[S-P^2-S]_n$ contains a number of at least one or even more of the same or different of the above defined cationic or polycationic polymers determined by integer n.

According to a further specific aspect of the first embodiment, such a repetitive component $[S-P^2-S]_n$ may be prepared using at least one or even more of the same or different of the above defined cationic or polycationic polymers and at least one or even more of the same or different of the above defined cationic or polycationic proteins or peptides, and polymerizing same in a polymerization condensation reaction via their -SH-moieties. Accordingly, such a repetitive component $[S-P^2-S]_n$ contains a number of at least one or even more of the same or different of the above defined cationic or polycationic polymers and at least one or even more of the same or different of the above defined cationic or polycationic proteins or peptides , both together determined by integer n.

According to a further aspect of the first embodiment, the polymeric carrier according to formula (I) above, may comprise at least one amino acid component $(AA)_x$, wherein AA is preferably an amino acid as defined in the following, which, when occurring as amino acid component $(AA)_x$, allows to (substantially) modify the biophysical/biochemical properties of the polymeric carrier according to formula (I) as defined herein. According to the present invention, the number of amino acids in such an amino acid component $(AA)_x$ (repetitions) is defined by x. In the above context, x is preferably an integer and may be selected from a range of about 1 to 100, preferably from a range of about 1 to 50, more preferably 1 to 30, and even more preferably selected from a number comprising 1, 2, 3, 4, 5, 6, 7, 8, 9, 10, 11, 12, 13, 14 or 15-30, e.g. from a range of about 1 to 30, from a range of about 1 to 15, or from a number comprising 1, 2, 3, 4, 5, 6, 7, 8, 9, 10, 11, 12, 13, 14 or 15, or may be selected from a range formed by any two of the afore mentioned values.

Such components $(AA)_x$ may be contained in every parts of the polymeric carrier according to formula (I) above and therefore may be attached to all components of the polymeric carrier according to formula (I). It is particularly preferred that $(AA)_x$ is present as ligand or part of the repetitive component $[S-P^2-S]_n$.

In this contex it is particularly preferred that the amino acid component $(AA)_x$ contains or is flanked (e.g. terminally) by at least one -SH containing moiety, which allows introducing this component $(AA)_x$ via a disulfide bond into the polymeric carrier according to formula (I) as defined herein. In this context, the amino acid component $(AA)_x$ may also be read as a component $-S-(AA)_x-$ or $-S-(AA)_x-S-$, wherein S represents a -SH containing moiety (or, of course, one sulfur of a disulfide bond), e.g. a cysteine residue. In the specific case that the -SH containing moiety represents a cysteine, the amino acid component $(AA)_x$ may also be read as $-Cys-(AA)_x-$ or $-Cys-(AA)_x-Cys-$ wherein Cys represents Cysteine and provides for the necessary -SH-moiety for a disulfide bond. (Accordingly, $-Cys-(AA)_x-Cys-$ may also be written as $-(S-Cys)-(AA)_x-(Cys-S)-$ and $-Cys-(AA)_x-$ may also be written as $-(S-Cys)-(AA)_x-$).) The -SH containing moiety may be also introduced into the amino acid component $(AA)_x$ using any of modifications or reactions as shown above for components $P^1$, $P^2$ or $P^3$. In the specific case that the amino acid component $(AA)_x$ is linked to two components of the polymeric carrier according to formula (I) it is preferred that $(AA)_x$ contains at least two -SH-moieties, e.g. at least two Cysteines, preferably at its terminal ends. This is particularly preferred if $(AA)_x$ is part of the repetitive component $[S-P^2-S]_n$.

In an alternative the amino acid component $(AA)_x$ is introduced into the polymeric carrier according to formula (I) as defined herein via any chemical possible addition reaction. Therefore the amino acid component $(AA)_x$ contains at least one further functional moiety, which allows attaching same to a further component as defined herein, e.g. component $P^1$ or $P^3$, $P^2$, L, or a further amino acid component $(AA)_x$, etc. Such functional moieties may be selected from functionalities which allow the attachment of further components, e.g. functionalities as defined herein, e.g. by amide formation (e.g. carboxylic acids, sulphonic acids, amines, etc.), by Michael addition (e.g maleinimide moieties, $\alpha,\beta$ unsatured carbonyls, etc.), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow $S_n$-type substitution reactions (e.g halogenalkans, thiols, alcohols, amines, hydrazines, hydrazides, sulphonic acid esters, oxyphosphonium salts) or other chemical moieties which can be utilized in the attachment of further components.

The amino acid component $(AA)_x$ may also occur as a mixed repetitive amino acid component $[(AA)_x]_z$, wherein the number of amino acid components $(AA)_x$ is further defined by z. In this context, z is an integer and may be selected from a range of about 1 to 30, preferably from a range of about 1 to 15, more preferably 1 to 10 or 1 to 5 and even more preferably selected from a number selected from 1, 2, 3, 4, 5, 6, 7, 8, 9, 10, 11, 12, 13, 14 or 15, or may be selected from a range formed by any two of the afore mentioned values. Such a mixed repetitive amino acid component $[(AA)_x]_z$ may be used to integrate several of the same or different amino acid components $(AA)_x$ as defined herein in the polymeric carrier. Preferably, in the mixed repetitive amino acid component $[(AA)_x]_z$ the amino acid component $(AA)_x$ may contain or s may be flanked (e.g. terminally) by at least one -SH containing moiety, preferably at least two -SH containing moieties as already defined above, which allows coupling the different amino acid components $(AA)_x$ using a disulfide bond via a condensation polymerization. Likewise as above, the mixed repetitive amino acid component $[(AA)_x]_z$ may also be read as $[S-(AA)_x-S]_z$, wherein S represents a -SH containing moiety, e.g. a cysteine residue. In the specific case that the -SH containing moiety represents a cysteine, the mixed repetitive amino acid component $[(AA)_x]_z$ may also be read as $[Cys-(AA)_x-Cys]_z$, wherein Cys represents Cysteine and provides for the necessary -SH-moiety for a disulfide bond. The -SH containing moiety may be also introduced into the amino acid component $(AA)_x$ using any of modifications or reactions as shown above for components $P^1$, $P^2$ or $P^3$.

The amino acid component $(AA)_x$ or the mixed repetitive amino acid component $[(AA)_x]_z$ may be provided with at least one -SH-moiety, e.g. in a form represented by formula $(AA)_x-SH$. Then, the component $(AA)_x$ according to formula $(AA)_x-SH$ or the mixed repetitive amino acid component $[(AA)_x]_z$ according to formula $[(AA)_x]_z-SH$, may be bound to any of components L, $P^1$, $P^2$ and/or $P^3$ or another component $(AA)_x$ via a disulfide bond. If bound to component $P^1$ and/or component $P^3$, components $P^1$ and/or $P^3$ preferably exhibit at least two -SH-moieties to allow further binding of components $P^1$ and/or $P^3$ to a component $P^2$ via a -SH-moiety forming a disulfide bond (see above). The amino acid component $(AA)_x$ in a form represented by formula $(AA)_x-SH$ or the mixed repetitive amino acid component $[(AA)_x]_z$ according to formula $[(AA)_x]_z-SH$ may be also used to terminate a condensation reaction due to its single -SH moiety. In this case, the amino acid component $(AA)_x$ in a form represented by formula $(AA)_x-SH$ is preferably coupled terminally to components $P^1$ and/or $P^3$. The amino acid component $(AA)_x$ in a form represented by formula $(AA)_x-SH$ or the mixed repetitive amino acid component $[(AA)_x]_z$ according to formula $[(AA)_x]_z-SH$ may be also used to bind internally to any of components L, $P^1$, $P^2$ and/or $P^3$ or a further component $(AA)_x$ via a further internal -SH-moiety of any of components L, $P^1$, $P^2$ and/or $P^3$ or $(AA)_x$.

Furthermore, the amino acid component $(AA)_x$ may be provided with two -SH-moieties (or even more), e.g. in a form represented by formula $HS-(AA)_x-SH$. Additionally, the mixed repetitive amino acid component $[(AA)_x]_z$ may be provided with two -SH-moieties (or even more), e.g. in a form represented by formula $HS-[(AA)_x]_z-SH$, to allow binding to two functionalities via disulfide bonds, e.g. if the amino acid component $(AA)_x$ or the mixed repetitive amino acid

component $[(AA)_x]_z$ is used as a linker between two further components (e.g. as a linker between components L and $P^1$, between components $P^1$ and $P^2$, in or as a part of repetitive component $[S-P^2-S]_n$, between components $P^2$ and $P^3$ and/or between components $P^3$ and L). In this case, one -SH moiety is preferably protected in a first step using a protecting group as known in the art, leading to an amino acid component $(AA)_x$ of formula HS-$(AA)_x$-S-protecting group or to a mixed repetitive amino acid component $[(AA)_x]_z$ of formula HS-$[(AA)_x]_z$-S-protecting group. Then, the amino acid component $(AA)_x$ or the mixed repetitive amino acid component $[(AA)_x]_z$ may be bound to a component L, $P^1$, $P^2$ and/or $P^3$, to form a first disulfide bond via the non-protected -SH moiety. The protected-SH-moiety is then typically deprotected and bound to a further free -SH-moiety of a further component L, $P^1$, $P^2$ and/or $P^3$ to form a second disulfide bond. In the case that the amino acid component $(AA)_x$ or the mixed repetitive amino acid component $[(AA)_x]_z$ is part of the repetitive component $[S-P^2-S]_n$ it is preferred that the formation of the disulfide bonds between $(AA)_x$ and $P^2$ concurrently occurs with the polycondensation reaction of the repetitive component $[S-P^2-S]_n$ and therefore no protection of the at least two terminal -SH-moieties is not necessary.

Alternatively, the amino acid component $(AA)_x$ or the mixed repetitive amino acid component $[(AA)_x]_z$ may be provided with other functionalities as already described above for components $P^1$ and $P^2$ and/or $P^3$, which allow binding of the amino acid component $(AA)_x$ or binding of the mixed repetitive amino acid component $[(AA)_x]_z$ to any of components $P^1$, $P^2$ and/or $P^3$ or $(AA)_x$ and optionally to component L.

Thus, according to the present invention, the amino acid component $(AA)_x$ and/or the mixed repetitive amino acid component $[(AA)_x]_z$ may be bound to $P^1$, $P^2$, $P^3$, $(AA)_x$ and/or L with or without using a disulfide linkage. Binding without using a disulfide linkage may be accomplished by any of the reactions described above, preferably by binding the amino acid component $(AA)_x$ or the mixed repetitive amino acid component $[(AA)_x]_z$ to $P^1$, $P^2$, $P^3$, $(AA)_x$ and/or L using an amid-chemistry as defined herein. If desired or necessary, the other terminus of the amino acid component $(AA)_x$ or the mixed repetitive amino acid component $[(AA)_x]_z$, e.g. the N- or C-terminus, may be used to couple another component, e.g. a ligand L. For this purpose, the other terminus of the amino acid component $(AA)_x$ or the mixed repetitive amino acid component $[(AA)_x]_z$ preferably comprises or is modified to comprise a further functionality, e.g. an alkyn-species (see above), which may be used to add the other component via e.g. click-chemistry. Such a construct, e.g. L-$(AA)_x$-$P^1$-S- or L-$[(AA)_x]_z$-$P^1$-S-, may be used to terminate the polymerization condensation reaction of repetitive component $[S-P^2-S]_n$. If the ligand is bound via an acid-labile bond, the bond may be cleaved off in the endosome and the polymeric carrier presents amino acid component $(AA)_x$ or the mixed repetitive amino acid component $[(AA)_x]_z$ at its surface.

The amino acid component $(AA)_x$ or the mixed repetitive amino acid component $[(AA)_x]_z$ may occur as a further component of generic formula (I) above, e.g. as a linker between components $P^1$ or $P^3$ and $P^2$, as a linker between components L and $P^1$ or $P^2$ or as an additional component of the repetitive component $[S-P^2-S]_n$.

According to a first alternative, such an amino acid component $(AA)_x$ or the mixed repetitive amino acid component $[(AA)_x]_z$ may be present as a linker between components $P^1$ or $P^3$ and component $P^2$. This is preferably represented in the context of the entire polymeric carrier according to formula (I) by following formulae:

$$\text{L-}P^1\text{-S-S-}(AA)_x\text{-S-}[S\text{-}P^2\text{-S}]_n\text{-S-}(AA)_x\text{-S-S-}P^3\text{-L,}$$

or

$$\text{L-}P^1\text{-S-}[S\text{-}(AA)_x\text{-S}]_z\text{-}[S\text{-}P^2\text{-S}]_n\text{-}[S\text{-}(AA)_x\text{-S}]_z\text{-S-}P^3\text{-L,}$$

wherein n, x, z, S, L, AA, $P^1$, $P^2$ and $P^3$ are preferably as defined herein. In the above formulae, the term "-S-S-" represents a disulfide bond, wherein this at least one sulfur of the disulfide bond may also be provided by a cysteine. In this case, the term "-S-S-" in these formulae may also be written as "-S-Cys", as "-Cys-S" or as "-Cys-Cys-". In this context, the term "-Cys-Cys-" does not represent a peptide bond but a linkage of two cysteines via their -SH-moieties to form a disulfide bond. Accordingly, the term "-Cys-Cys-" may also be understood generally as "-(Cys-S)-(S-Cys)-", wherein in this specific case S indicates the sulfur of the -SH-moiety of cysteine. Likewise, the terms "-S-Cys" and "-Cys-S" indicate a disulfide bond between a -SH containing moiety and a cysteine, which may also be written as "-S-(S-Cys)" and "-(Cys-S)-S".

According to a second alternative, such an amino acid component $(AA)_x$ or the mixed repetitive amino acid component $[(AA)_x]_z$ may be present as a linker between components $P^1$ or $P^3$ and component L. This is preferably represented in the context of the entire polymeric carrier according to formula (I) by following formulae:

$$\text{L-}(AA)_x\text{-}P^1\text{-S-}[S\text{-}P^2\text{-S}]_n\text{-S-}P^3\text{-}(AA)_x\text{-L,}$$

or

$$L-[(AA)_x]_z-P^1-S-[S-P^2-S]_n-S-P^3-[(AA)_x]_z-L,$$

or alternatively

$$L-(AA)_x-S-S-P^1-S-[S-P^2-S]_n-S-P^3-S-S-(AA)_x-S-S-L,$$

or

$$L-S-S-(AA)_x-S-S-P^1-S-[S-P^2-S]_n-S-P^3-S-S-(AA)_x-S-S-L,$$

or

$$L-S-[S-(AA)_x-S]_z-S-P^1-S-[S-P^2-S]_n-S-P^3-S-[S-(AA)_x-S]_z-S-L,$$

etc. wherein n, x, z, S, L, AA, $P^1$, $P^2$ and $P^3$ are preferably as defined herein. In the above formulae, the term "-S-S-" represents a disulfide bond, as already defined above.

According to a third alternative, such an amino acid component $(AA)_x$ or the mixed repetitive amino acid component $[(AA)_x]_z$ may be present as a part of components $P^1$ and/or $P^3$, wherein the amino acid component $(AA)_x$ may be directly bound to (e.g. the terminus of) component $P^1$ and/or $P^3$ without a further ligand L. In this case the $(AA)_x$ component may be in the form of a ligand as defined above. This is preferably represented in the context of the entire polymeric carrier according to formula (I) by following formulae:

$$(AA)_x-P^1-S-[S-P^2-S]_n-S-P^3-(AA)_x,$$

or

$$[(AA)_x]_z-P^1-S-[S-P^2-S]_nS-P^3-[(AA)_x]_z,$$

or or alternatively

$$(AA)_x-S-S-P^1-S-[S-P^2-S]_n-S-P^3-S-S-(AA)_c,$$

or

$$H-[S-(AA)_x-S]_z-S-P^1-S-[S-P^2-S]_n-S-S-P^3-S-[S-(AA)_xS]_z-H,$$

wherein n, x, z, S, AA, $P^1$, $P^2$ and $P^3$ are preferably as defined herein. In the above formulae, the term "-S-S-" represents a disulfide bond, as already defined above. The free -SH moiety at the terminal ends in the last formula may also be terminated using a monothiol compound as defined herein.

According to a fourth and particularly preferred alternative, the amino acid component $(AA)_x$, preferably written as $S-(AA)_x-S$ or $[S-(AA)_x-S]$ may be used to modify component $P^2$, particularly the content of component $S-P^2-S$ in repetitive component $[S-P^2-S]_n$ of formula (I) above. This may be represented in the context of the entire polymeric carrier according to formula (I) e.g. by following formula (Ia):

$$L-P^1-S-\{[S-P^2-S]_a[S-(AA)_x-S]_b\}-S-P^3-L,$$

wherein x, S, L, AA, $P^1$, $P^2$ and $P^3$ are preferably as defined herein. In formula (Ia) above, any of the single components $[S-P^2-S]$ and $[S-(AA)_x-S]$ may occur in any order in the subformula $\{[S-P^2-S]_a[S-(AA)_x-S]_b\}$. The numbers of single components $[S-P^2-S]$ and $[S-(AA)_x-S]$ in the subformula $\{[S-P^2-S]_a[S-(AA)_x-S]_b\}$ are determined by integers a and b, wherein a + b = n. n is an integer and is defined as above for formula (I).

a is an integer, typically selected independent from integer b from a range of about 1 to 50, preferably from a range of about 1, 2 or 3 to 30, more preferably from a range of about 1, 2, 3, 4, or 5 to 25, or a range of about 1, 2, 3, 4, or 5 to 20, or a range of about 1, 2, 3, 4, or 5 to 15, or a range of about 1, 2, 3, 4, or 5 to 10, including e.g. a range of about 3 to 20, 4 to 20, 5 to 20, or 10 to 20, or a range of about 3 to 15, 4 to 15, 5 to 15, or 10 to 15, or a range of about 6 to 11 or 7 to 10. Most preferably, a is in a range of about 1, 2, 3, 4, or 5 to 10, more preferably in a range of about 1, 2, 3, or 4 to 9, in a range of about 1, 2, 3, or 4 to 8, or in a range of about 1, 2, or 3 to 7.

b is an integer, typically selected independent from integer a from a range of about 0 to 50 or 1 to 50, preferably from a range of about 0, 1, 2 or 3 to 30, more preferably from a range of about 0, 1, 2, 3, 4, or 5 to 25, or a range of about 0, 1, 2, 3, 4, or 5 to 20, or a range of about 0, 1, 2, 3, 4, or 5 to 15, or a range of about 0, 1, 2, 3, 4, or 5 to 10, including e.g. a range of about 3 to 20, 4 to 20, 5 to 20, or 10 to 20, or a range of about 3 to 15, 4 to 15, 5 to 15, or 10 to 15, or a range of about 6 to 11 or 7 to 10. Most preferably, b is in a range of about 1, 2, 3, 4, or 5 to 10, more preferably in a range of about 1, 2, 3, or 4 to 9, in a range of about 1, 2, 3, or 4 to 8, or in a range of about 1, 2, or 3 to 7.

In the above formula, the term "-S-S-" (the brackets are omitted for better readability) represents a disulfide bond as already defined above.

The modification of component $P^2$, particularly of component $S-P^2-S$ of repetitive component $[S-P^2-S]_n$, by "diluting" same with amino acid components $(AA)_x$ may be also realized in the context of any of the afore mentioned alternatives of the entire polymeric carrier according to formula (I),

$$L-P^1-S-S-(AA)_x-S-\{[S-P^2-S]_a[S-(AA)_x-S]_b\}-S-(AA)_x-S-S-P^3-L,$$

or

$$L-P^1-S-[S-(AA)_x-S]_z-\{[S-P^2-S]_a[S-(AA)_x-S]_b\}-[S-(AA)_x-S]_z-S-P^3-L,$$

or

$$L-(AA)_x-P^1-S-\{[S-P^2-S]_a[S-(AA)_x-S]_b\}-S-P^3-(AA)_x-L,$$

or

$$L-[(AA)_x]_z-P^1-S-\{[S-P^2-S]_a[S-(AA)_x-S]_b\}-S-P^3-[(AA)_x]_z-L,$$

or

$$L-(AA)_x-S-S-P^1-S-\{[S-P^2-S]_a[S-(AA)_x-S]_b\}-S-P^3-S-S-(AA)_x-S-S-L,$$

or

$$L-S-S-(AA)_x-S-S-P^1-S-\{[S-P^2-S]_a[S-(AA)_x-S]_b\}-S-P^3-S-S-(AA)_x-S-S-L,$$

or

$$L-S-[S-(AA)_x-S]_z-S-P^1-S-\{[S-P^2-S]_a[S-(AA)_x-S]_b\}-S-P^3-S-[S-(AA)_x-S]_z-S-L,$$

or

$$(AA)_x-P^1-S-\{[S-P^2-S]_a[S-(AA)_x-S]_b\}-S-P^3-(AA)_x,$$

or

$$[(AA)_x]_z-P^1-S-\{[S-P^2-S]_a[S-(AA)_x-S]_b\}-S-P^3-[(AA)_x]_z,$$

or

$$(AA)_x-S-S-P^1-S-\{[S-P^2-S]_a[S-(AA)_x-S]_b\}-S-P^3-S-S-(AA)_x,$$

or

$$H-[S-(AA)_x-S]_z-S-P^1-S-\{[S-P^2-S]_a[S-(AA)_x-S]_b\}-S-P^3-S-[S-(AA)_x-S]_z-H,$$

wherein n, x, z, a, b, S, L, AA, $P^1$, $P^2$ and $P^3$ are preferably as defined herein. Likewise, the term "-S-S-" represents a disulfide bond and is preferably as defined herein.

In the above alternatives, wherein the component $[S-P^2-S]$ is preferably "diluted" with amino acid components $[S-(AA)_x-S]$, the ratio is determined by integers a and b, wherein a + b = n. Preferably, integers a and b are selected such that the cationic binding properties of component $[S-P^2-S]$ are not lost but remain to a minimum extent in subformula/component $\{[S-P^2-S]_a[S-(AA)_x-S]_b\}$. This allows to weaken ("dilute") the cationic binding strength of component [S-

P$^2$-S] in repetitive component [S-P$^2$-S]$_n$ of polymeric carrier of formula (I) to a desired extent.

In this specific context the (desired) cationic binding strength of subformula/component {[S-P$^2$-S]$_a$[S-(AA)$_x$-S]$_b$} may be determined using different methods.

According to a first alternative, component P$^2$ of formula (I) of the present invention is particularly preferable a cationic or polycationic peptide as defined herein. Furthermore, the amino acid component (AA)$_x$, preferably written as [S-(AA)$_x$-S], typically resembles a peptide sequence. In this specific case, the cationic properties of subformula/component {[S-P$^2$-S]$_a$[S-(AA)$_x$-S]$_b$} may be determined upon their content of cationic amino acids in the entire subformula/component. Preferably, the content of cationic amino acids in subformula/component {[S-P$^2$-S]$_a$[S-(AA)$_x$-S]$_b$} is at least 10%, 20%, or 30%, preferably at least 40%, more preferably at least 50%, 60% or 70%, but also preferably at least 80%, 90%, or even 95%, 96%, 97%, 98%, 99% or 100%, most preferably at least 30%, 40%, 50%, 60%, 70%, 80%, 90%, 95%, 96%, 97%, 98%, 99% or 100%, or may be in the range of about 10% to 90%, more preferably in the range of about 15% to 75%, even preferably in the range of about 20% to 50%, e.g. 20, 30, 40 or 50%, or in a range formed by any two of the afore mentioned values, provided, that the content of all amino acids, e.g. cationic, lipophilic, hydrophilic, aromatic and further amino acids, in the entire subformula/component {[S-P$^2$-S]$_a$[S-{AA)$_x$-S]$_b$} is 100%.

According to a second alternative, component P$^2$ of formula (I) of the present invention is particularly preferable a cationic or polycationic polymer as defined herein. The amino acid component (AA)$_x$, preferably written as [S-(AA)$_x$-S], typically resembles a peptide sequence. In this specific case, the cationic properties of subformula/component {[S-P$^2$-S]$_a$[S-(AA)$_x$-S]$_b$} may be determined upon their content of cationic charges in the entire subformula/component. Preferably, the content of cationic charges in subformula/component {[S-P$^2$-S]$_a$[S-(AA)$_x$-S]$_b$} at a (physiological) pH as defined herein is at least 10%, 20%, or 30%, preferably at least 40%, more preferably at least 50%, 60% or 70%, but also preferably at least 80%, 90%, or even 95%, 96%, 97%, 98%, 99% or 100%, most preferably at least 30%, 40%, 50%, 60%, 70%, 80%, 90%, 95%, 96%, 97%, 98%, 99% or 100%, or may be in the range of about 10% to 90%, more preferably in the range of about 15% to 75%, even preferably in the range of about 20% to 50%, e.g. 20, 30, 40 or 50%, or in a range formed by any two of the afore mentioned values, provided, that the content of all charges, e.g. positive and negative charges at a (physiological) pH as defined herein, in the entire subformula/component {[S-P$^2$-S]$_a$[S-(AA)$_x$S]$_b$} is 100%.

Additionally, the polymeric carrier according to formula (I) above (or according to any of its subformulas herein), may comprise as an additional component, preferably as a ligand L or as an amino acid component (AA)$_x$ a signal peptide, a localization signal or sequence or a nuclear localization signal or sequence (NLS), which allows a translocalization of the polymeric carrier according to formula (I) above to a specific target, e.g. into the cell, into the nucleus, into the endosomal compartment, sequences for the mitochondrial matrix, localisation sequences for the plasma membrane, localisation sequences for the Golgi apparatus, the nucleus, the cytoplasm and the cytosceleton, etc. Such a signal peptide, localization signal or sequence or nuclear localization signal may be used for the transport of any of the herein defined nucleic acids, preferably an RNA or a DNA, more preferably an shRNA or a pDNA, e.g. into the nucleus. Without being limited thereto, such a signal peptide, localization signal or sequence or nuclear localization signal may comprise, e.g., localisation sequences for the endoplasmic reticulum. Particular localization signals or sequences or nuclear localization signals may include e.g. KDEL (SEQ ID NO: 85), DDEL (SEQ ID NO: 86), DEEL(SEQ ID NO: 87), QEDL (SEQ ID NO: 88), RDEL (SEQ ID NO: 89), and GQNLSTSN (SEQ ID NO: 90), nuclear localisation sequences, including PKKKRKV (SEQ ID NO: 91), PQKKIKS (SEQ ID NO: 92), QPKKP (SEQ ID NO: 93), RKKR (SEQ ID NO: 94), RKKRRQRRRAHQ (SEQ ID NO: 95), RQARRNRRRRWRERQR (SEQ ID NO: 96), MPLTRRRPAASQALAPPTP (SEQ ID NO: 97), GAALTILV (SEQ ID NO: 98), and GAALTLLG (SEQ ID NO: 99), localisation sequences for the endosomal compartment, including MDDQRDLISNNEQLP (SEQ ID NO: 100), localisation sequences for the mitochondrial matrix, including MLFNLRXXLNNAAFRHGHNFMVRNFRCGQPLX (SEQ ID NO: 101), localisation sequences for the plasma membrane: GCVCSSNP (SEQ ID NO: 102), GQTVTTPL (SEQ ID NO: 103), GQELSQHE (SEQ ID NO: 104), GNSPSYNP (SEQ ID NO: 105), GVSGSKGQ (SEQ ID NO: 106), GQTITTPL (SEQ ID NO: 107), GQTLTTPL (SEQ ID NO: 108), GQIFSRSA (SEQ ID NO: 109), GQIHGLSP (SEQ ID NO: 110), GARASVLS (SEQ ID NO: 111), and GCTLSAEE (SEQ ID NO: 112), localisation sequences for the endoplasmic reticulum and the nucleus, including GAQVSSQK (SEQ ID NO: 113), and GAQLSRNT (SEQ ID NO: 114), localisation sequences for the Golgi apparatus, the nucleus, the cytoplasm and the cytosceleton, including GNAAAAKK (SEQ ID NO: 115), localisation sequences for the cytoplasm and cytosceleton, including GNEASYPL (SEQ ID NO: 116), localisation sequences for the plasma membrane and cytosceleton, including GSSKSKPK (SEQ ID NO: 117), etc. Examples of secretory signal peptide sequences as defined herein include, without being limited thereto, signal sequences of classical or non-classical MHC-molecules (e.g. signal sequences of MHC I and II molecules, e.g. of the MHC class I molecule HLA-A*0201), signal sequences of cytokines or immunoglobulines as defined herein, signal sequences of the invariant chain of immunoglobulines or antibodies as defined herein, signal sequences of Lamp1, Tapasin, Erp57, Calretikulin, Calnexin, and further membrane associated proteins or of proteins associated with the endoplasmic reticulum (ER) or the endosomal-lysosomal compartment. Particularly preferably, signal sequences of MHC class I molecule HLA-A*0201 may be used according to the present invention. Most preferably such an additional component may occur as component L as defined herein. Alternatively, such an additional component may also be bound e.g. to a component L, $P^1$, $P^2$, $P^3$ or $(AA)_x$ as defined herein, e.g. to a side chain of any of components L, $P^1$, $P^2$, $P^3$ or $(AA)_x$, preferably via a side chain of component $P^2$, or optionally as a linker between components L and $P^1$ or $P^3$ and L. The binding to any of components L, $P^1$, $P^2$, or $P^3$ may also be accomplished using an acid-labile bond, preferably via a side chain of any of components L, $P^1$, $P^2$, $P^3$, which allows to detach or release the additional component at lower pH-values, e.g. at physiological pH-values as defined herein.

Additionally, the polymeric carrier according to formula (I) above (or according to any of its subformulas herein), may comprise further functional peptides or proteins preferably as ligand or amino acid component $(AA)_x$, which may modulate the functionality of the polymeric carrier accordingly. According to one alternative, such further functional peptides or proteins may comprise so called cell penetrating peptides (CPPs) or cationic peptides for transportation.

Particularly preferred are CPPs, which induce a pH-mediated conformational change in the endosome and lead to an improved release of the polymeric carrier (in complex with a nucleic acid) from the endosome by insertion into the lipid layer of the liposome. Such called cell penetrating peptides (CPPs) or cationic peptides for transportation, may include, without being limited thereto protamine, nucleoline, spermine or spermidine, poly-L-lysine (PLL), basic polypeptides, poly-arginine, cell penetrating peptides (CPPs), chimeric CPPs, such as Transportan, or MPG peptides, HIV-binding peptides, Tat, HIV-1 Tat (HIV), Tat-derived peptides, oligoarginines, members of the penetratin family, e.g. Penetratin, Antennapedia-derived peptides (particularly from *Drosophila antennapedia*), pAntp, pIsl, etc., antimicrobial-derived CPPs e.g. Buforin-2, Bac715-24, SynB, SynB(1), pVEC, hCT-derived peptides, SAP, MAP, KALA, PpTG20, Proline-rich peptides, Loligomers, Arginine-rich peptides, Calcitonin-peptides, FGF, Lactoferrin, , poly-L-Lysine, poly-Arginine, histones, VP22 derived or analog peptides, HSV, VP22 (Herpes simplex), MAP, KALA or protein transduction domains (PTDs, PpT620, prolin-rich peptides, arginine-rich peptides, lysine-rich peptides, Pep-1, L-oligomers, Calcitonin peptide(s), etc. Likewise, such an additional component may occur as component L or $(AA)_x$ as defined herein. Alternatively, such an additional component may also be bound to a component L, $P^1$, $P^2$, $P^3$ or $(AA)_x$ as defined herein, e.g. to a side chain of any of components L, $P^1$, $P^2$, $P^3$, or $(AA)_x$ preferably via a side chain of component $P^2$, or optionally as a linker between components L and $P^1$ or $P^3$ and L. The binding to any of components L, $P^1$, $P^2$, $P^3$ or $(AA)_x$ may also be accomplished using an acid-labile bond, preferably via a side chain of any of components L, $P^1$, $P^2$, $P^3$, or $(AA)_x$ which allows to detach or release the additional component at lower pH-values, e.g. at physiological pH-values as defined herein. In this context it is particularly preferred that this additional component occurs as ligand L or as amino acid component $(AA)_x$ of the repetitive component $[S-P^2-S]_n$ of formula (I).

According to a last alternative, the polymeric carrier according to formula (I) above (or according to any of its subformulas herein), may comprise as an additional component, preferably as amino acid component $(AA)_x$, any peptide or protein which can execute any favorable function in the cell. Particularly preferred are peptides or proteins selected from therapeutically active proteins or peptides, from antigens, e.g. tumour antigens, pathogenic antigens (animal antigens, viral antigens, protozoal antigens, bacterial antigens, allergic antigens), autoimmune antigens, or further antigens, from allergens, from antibodies, from immunostimulatory proteins or peptides, from antigen-specific T-cell receptors, or from any other protein or peptide suitable for a specific (therapeutic) application as defined below for coding nucleic acids. Likewise, such an additional component may occur preferably as $(AA)_x$ as defined herein. Alternatively, such an additional component may also be bound to a component L, $P^1$, $P^2$, $P^3$ or $(AA)_x$ as defined herein, e.g. to a side chain of any of components L, $P^1$, $P^2$, $P^3$, or $(AA)_x$ preferably via a side chain of component $P^2$, or optionally as a linker between components L and $P^1$ or $P^3$ and L. The binding to any of components L, $P^1$, $P^2$, $P^3$ or $(AA)_x$ may also be accomplished using an acid-labile bond, preferably via a side chain of any of components L, $P^1$, $P^2$, $P^3$, or $(AA)_x$ which allows to detach or release the additional component at lower pH-values, e.g. at physiological pH-values as defined herein. In this context it is particularly preferred that this additional component occurs as amino acid component $(AA)_x$ of the repetitive component $[S-P^2-S]_n$ of formula (I).

The polymeric carrier according to formula (I) may comprise at least one of the above mentioned cationic or polycationic peptides, proteins or polymers or further components, e.g. (AA), wherein any of the above alternatives may be combined with each other, and may be formed by polymerizing same in a polymerization condensation reaction via their -SH-moieties.

In the nucleic acid containing polymeric carrier cargo complex, the polymeric carrier molecule according to generic formula (I) $L-P^1-S-[S-P^2-S]_n-S-P^3-L$ as defined herein (or according to any of its subformulas herein) and the nucleic acid cargo are typically provided in a molar ratio of about 5 to 10000, preferably in a molar ratio of about 5 to 5000, more preferably in a molar ratio of about 5 to 2500, even more preferably in a molar ratio of about 5 to 2000, and most preferably in a molar ratio of about 5 to 1000 of polymeric carrier molecule : nucleic acid, or in a molar ratio of about 50 to 1000 of polymeric carrier molecule : nucleic acid, e.g. in a molar ratio of about 10 to 5000, in a molar ratio of about 20 to 2500, in a molar ratio of about 25 to 2000 of polymeric carrier molecule : nucleic acid.

Furthermore, in the polymeric carrier cargo complex, the polymeric carrier molecule according to generic formula (I) $L-P^1-S-[S-P^2-S]_n-S-P^3-L$ as defined herein (or according to any of its subformulas herein) and the nucleic acid cargo are preferably provided in an N/P-ratio of about 0.1 to 20, preferably in an N/P-ratio of about 0.2 to 12, and even more preferably in an N/P-ratio of about 0.4 to 10 or 0,6 to 5. In this context, an N/P-ratio is defined as the nitrogen/phosphate ratio (N/P-ratio) of the entire polymeric carrier cargo complex. This is typically illustrative for the content/amount of peptides, if peptides are used, in the polymeric carrier and characteristic for the content/amount of nucleic acids bound or complexed in the polymeric carrier cargo complex. It may be calculated on the basis that, for example, 1 μg RNA typically contains about 3 nmol phosphate residues, provided that the RNA exhibits a statistical distribution of bases. Additionally, 1 μg peptide typically contains about x*1 μg/M(peptide) nmol nitrogen residues, dependent on the molecular weight and the number x of its (cationic) amino acids.

In the context of the present invention such a nucleic acid cargo of the polymeric carrier cargo complex formed by the nucleic acid cargo and a polymeric carrier molecule according to generic formula (I) (or according to any of its subformulas herein) may be any suitable nucleic acid, selected e.g. from any DNA, preferably, without being limited thereto, e.g. genomic DNA, single-stranded DNA molecules, double-stranded DNA molecules, coding DNA, DNA primers, DNA probes, a pDNA, immunostimulating DNA or may be selected e.g. from any RNA, preferably, without being limited thereto, a coding RNA, a messenger RNA (mRNA), an siRNA, an shRNA, an antisense RNA, or riboswitches, immunostimulating RNA (isRNA) ribozymes or aptamers; etc. The nucleic acid may also be a ribosomal RNA (rRNA), a transfer RNA (tRNA), a messenger RNA (mRNA), or a viral RNA (vRNA). Preferably, the nucleic acid is RNA, more preferably a coding RNA. Even more preferably, the nucleic acid may be a (linear) single-stranded RNA, even more preferably an mRNA. In the context of the present invention, an mRNA is typically an RNA, which is composed of several structural elements, e.g. an optional 5'-UTR region, an upstream positioned ribosomal binding site followed by a coding region, an optional 3'-UTR region, which may be followed by a poly-A tail (and/or a poly-C-tail). An mRNA may occur as a mono-, di-, or even multicistronic RNA, i.e. an RNA which carries the coding sequences of one, two or more (identical or different) proteins or peptides as defined herein. Such coding sequences in di-, or even multicistronic mRNA may be separated by at least one IRES (internal ribosomal entry site) sequence.

Furthermore, the nucleic acid of the polymeric carrier cargo complex formed by the nucleic acid cargo and a polymeric carrier molecule according to generic formula (I) (or according to any of its subformulas herein) may be a single- or a double-stranded nucleic acid (molecule) (which may also be regarded as a nucleic acid (molecule) due to non-covalent association of two single-stranded nucleic acid(s) (molecules)) or a partially double-stranded or partially single stranded nucleic acid, which are at least partially self complementary (both of these partially double-stranded or partially single stranded nucleic acid molecules are typically formed by a longer and a shorter single-stranded nucleic acid molecule or by two single stranded nucleic acid molecules, which are about equal in length, wherein one single-stranded nucleic acid molecule is in part complementary to the other single-stranded nucleic acid molecule and both thus form a double-stranded nucleic acid molecule in this region, i.e. a partially double-stranded or partially single stranded nucleic acid (molecule). Preferably, the nucleic acid (molecule) may be a single-stranded nucleic acid molecule. Furthermore, the nucleic acid (molecule) may be a circular or linear nucleic acid molecule, preferably a linear nucleic acid molecule.

Coding nucleic acids:

The nucleic acid molecule of the polymeric carrier cargo complex may encode a protein or a peptide, which may be selected, without being restricted thereto, e.g. from therapeutically active proteins or peptides, selected e,g, from adjuvant proteins, from antigens, e.g. tumour antigens, pathogenic antigens (e.g. selected, from animal antigens, from viral antigens, from protozoal antigens, from bacterial antigens), allergenic antigens, autoimmune antigens, or further antigens, from allergens, from antibodies, from immunostimulatory proteins or peptides, from antigen-specific T-cell receptors, or from any other protein or peptide suitable for a specific (therapeutic) application, wherein the coding nucleic acid may be transported into a cell, a tissue or an organism and the protein may be expressed subsequently in this cell, tissue or organism.

The coding region of the nucleic acid molecule of the polymeric carrier cargo complex may occur as a mono-, di-, or even multicistronic nucleic acid, i.e. a nucleic acid which carries the coding sequences of one, two or more proteins or peptides. Such coding sequences in di-, or even multicistronic nucleic acids may be separated by at least one internal ribosome entry site (IRES) sequence, or by signal peptides which induce the cleavage of the resulting polypeptide which comprises several proteins or peptides.

In particular preferred aspects the encoded peptides or proteins are selected from human, viral, bacterial, protozoan proteins or peptides.

*a) Therapeutically active proteins*

In the context of the present invention, therapeutically active proteins or peptides may be encoded by the nucleic acid molecule of the herein defined polymeric carrier cargo complex. Therapeutically active proteins are defined herein as proteins which have an effect on healing, prevent prophylactically or treat therapeutically a disease, preferably as defined herein, or are proteins of which an individual is in need of. These may be selected from any naturally or synthetically designed occurring recombinant or isolated protein known to a skilled person from the prior art. Without being restricted thereto therapeutically active proteins may comprise proteins, capable of stimulating or inhibiting the signal transduction in the cell, e.g. cytokines, lymphokines, monokines, growth factors, receptors, signal transduction molecules, transcription factors, etc; anticoagulants; antithrombins; antiallergic proteins; apoptotic factors or apoptosis related proteins, therapeutic active enzymes and any protein connected with any acquired disease or any hereditary disease.

Particularly preferred in this context are therapeutically active proteins which are beneficial for the prevention of restenosis like e.g. thimidine kinase, cytosine deaminase, Fas ligand, CDK2, CDC3, cyclin B, CDK inhibitors p21 and p27, p16-p27, p53, hRAD 50, etc. or proteins which reduce intimal hyperplasia like PDGF receptor beta, TIMP-1, TIMP-3, eher t_PA /tissue plasminogen activator) etc. or VEGF, nitric oxide synthetases (eNOS and iNOS), thrombin inhibitor hirudun, TFPI, prostacyclin synthase (PGIS), COX-1, L-10, Il-4, Il-11, ADAM8, 9, 10, 12, 15, 17, 19, 28 and 33.

Furthermore, particularly preferred in this context are therapeutically active proteins which are beneficial for the prevention of inflammation, particularly in the context of the application of stents, artificial organs or joints.

A therapeutically active protein, which may be encoded by the nucleic acid molecule of the herein defined polymeric carrier cargo complex, may also be an adjuvant protein. In this context, an adjuvant protein is preferably to be understood as any protein, which is capable to elicit an innate immune response as defined herein. Preferably, such an innate immune response comprises activation of a pattern recognition receptor, such as e.g. a receptor selected from the Toll-like receptor (TLR) family, including e.g. a Toll like receptor selected from human TLR1 to TLR10 or from murine Toll like receptors TLR1 to TLR13. More preferably, the adjuvant protein is selected from human adjuvant proteins or from pathogenic adjuvant proteins, selected from the group consisting of, without being limited thereto, bacterial proteins, protozoan proteins, viral proteins, or fungal proteins, animal proteins, in particular from bacterial adjuvant proteins. In addition, nucleic acids encoding human proteins involved in adjuvant effects (e.g. ligands of pattern recognition receptors, pattern recoginition receptors, proteins of the signal transduction pathways, transcription factors or cytokines) may be used as well.

*b) Antigens*

The nucleic acid molecule of the herein defined polymeric carrier cargo complex may alternatively encode an antigen. According to the present invention, the term "antigen" refers to a substance which is recognized by the immune system and is capable of triggering an antigen-specific immune response, e.g. by formation of antibodies or antigen-specific T-cells as part of an adaptive immune response. In this context an antigenic epitope, fragment or peptide of a protein means particularly B cell and T cell epitopes which may be recognized by B cells, antibodies or T cells respectively.

In the context of the present invention, antigens as encoded by the nucleic acid molecule of the herein defined polymeric carrier cargo complex typically comprise any antigen, antigenic epitope or antigenic peptide, falling under the above definition, more preferably protein and peptide antigens, e.g. tumour antigens, allergenic antigens, auto-immune self-antigens, pathogenic antigens, etc. In particular antigens as encoded by the nucleic acid molecule of the herein defined polymeric carrier cargo complex may be antigens generated outside the cell, more typically antigens not derived from the host organism (e.g. a human) itself (i.e. non-self antigens) but rather derived from host cells outside the host organism, e.g. viral antigens, bacterial antigens, fungal antigens, protozoological antigens, animal antigens, allergenic antigens, etc. Allergenic antigens (allergy antigens) are typically antigens, which cause an allergy in a human and may be derived from either a human or other sources. Additionally, antigens as encoded by the nucleic acid molecule of the herein defined polymeric carrier cargo complex may be furthermore antigens generated inside the cell, the tissue or the body. Such antigens include antigens derived from the host organism (e.g. a human) itself, e.g. tumour antigens, self-antigens or auto-antigens, such as auto-immune self-antigens, etc., but also (non-self) antigens as defined herein, which have been originally been derived from host cells outside the host organism, but which are fragmented or degraded inside the body, tissue or cell, e.g. by (protease) degradation, metabolism, etc.

One class of antigens as encoded by the nucleic acid molecule of the herein defined polymeric carrier cargo complex comprises tumour antigens. "Tumour antigens" are preferably located on the surface of the (tumour) cell. Tumour antigens may also be selected from proteins, which are overexpressed in tumour cells compared to a normal cell. Furthermore, tumour antigens also include antigens expressed in cells which are (were) not themselves (or originally not themselves) degenerated but are associated with the supposed tumour. Antigens which are connected with tumour-supplying vessels or (re)formation thereof, in particular those antigens which are associated with neovascularization, e.g. growth factors, such as VEGF, bFGF etc., are also included herein. Antigens connected with a tumour furthermore include antigens from cells or tissues, typically embedding the tumour. Further, some substances (usually proteins or peptides) are expressed in patients suffering (knowingly or not-knowingly) from a cancer disease and they occur in increased concentrations in the body fluids of said patients. These substances are also referred to as "tumour antigens", however they are not antigens in the stringent meaning of an immune response inducing substance. The class of tumour antigens can be divided further into tumour-specific antigens (TSAs) and tumour-associated-antigens (TAAs). TSAs can only be presented by tumour cells and never by normal "healthy" cells. They typically result from a tumour specific mutation. TAAs, which are more common, are usually presented by both tumour and healthy cells. These antigens are recognized and the antigen-presenting cell can be destroyed by cytotoxic T cells. Additionally, tumour antigens can also occur on the surface of the tumour in the form of, e.g., a mutated receptor. In this case, they can be recognized by antibodies.

According to a preferred aspect, such tumor antigens as encoded by the nucleic acid of the polymeric carrier cargo complex are selected from the group consisting of 5T4, 707-AP, 9D7, AFP, AlbZIP HPG1, alpha-5-beta-1-integrin, alpha-5-beta-6-integrin, alpha-actinin-4/m, alpha-methylacyl-coenzyme A racemase, ART-4, ARTC1/m, B7H4, BAGE-1, BCL-2, bcr/abl, beta-catenin/m, BING-4, BRCA1/m, BRCA2/m, CA 15-3/CA 27-29, CA 19-9, CA72-4, CA125, calreticulin, CAMEL, CASP-8/m, cathepsin B, cathepsin L, CD19, CD20, CD22, CD25, CDE30, CD33, CD4, CD52, CD55, CD56, CD80, CDC27/m, CDK4/m, CDKN2A/m, CEA, CLCA2, CML28, CML66, COA-1/m, coactosin-like protein, collage XXIII, COX-2, CT-9/BRD6, Cten, cyclin B1, cyclin D1, cyp-B, CYPB1, DAM-10, DAM-6, DEK-CAN, EFTUD2/m, EGFR, ELF2/m, EMMPRIN, EpCam, EphA2, EphA3, ErbB3, ETV6-AML1, EZH2, FGF-5, FN, Frau-1, G250, GAGE-1, GAGE-2, GAGE-3, GAGE-4, GAGE-5, GAGE-6, GAGE7b, GAGE-8, GDEP, GnT-V, gp100, GPC3, GPNMB/m, HAGE, HAST-2, hepsin, Her2/neu, HERV-K-MEL, HLA-A*0201-R17I, HLA-A11/m, HLA-A2/m, HNE, homeobox NKX3.1, HOM-TES-14/SCP-1, HOM-TES-85, HPV-E6, HPV-E7, HSP70-2M, HST-2, hTERT, iCE, IGF-1R, IL-13Ra2, IL-2R, IL-5, immature laminin receptor, kallikrein-2, kallikrein-4, Ki67, KIAA0205, KIAA0205/m, KK-LC-1, K-Ras/m, LAGE-A1, LDLR-FUT, MAGE-A1, MAGE-A2, MAGE-A3, MAGE-A4, MAGE-A6, MAGE-A9, MAGE-A10, MAGE-A12, MAGE-B1, MAGE-B2, MAGE-B3, MAGE-B4, MAGE-B5, MAGE-B6, MAGE-B10, MAGE-B16, MAGE-B17, MAGE-C1, MAGE-C2, MAGE-C3, MAGE-D1, MAGE-D2, MAGE-D4, MAGE-E1, MAGE-E2, MAGE-F1, MAGE-H1, MAGEL2, mammaglobin A, MART-1/melan-A, MART-2, MART-2/m, matrix protein 22, MC1R, M-CSF, ME1/m, mesothelin, MG50/PXDN, MMP11, MN/CA IX-antigen, MRP-3, MUC-1, MUC-2, MUM-1/m, MUM-2/m, MUM-3/m, myosin class I/m, NA88-A, N-acetylglucosaminyltransferase-V, Neo-PAP, Neo-PAP/m, NFYC/m, NGEP, NMP22, NPM/ALK, N-Ras/m, NSE, NY-ESO-1, NY-ESO-B, OA1, OFA-iLRP, OGT, OGT/m, OS-9, OS-9/m, osteocalcin, osteopontin, p15, p190 minor bcr-abl, p53, p53/m, PAGE-4, PAI-1, PAI-2, PART-1, PATE, PDEF, Pim-1-Kinase, Pin-1, Pml/PARalpha, POTE, PRAME, PRDX5/m, prostein, proteinase-3, PSA, PSCA, PSGR, PSM, PSMA, PTPRK/m, RAGE-1, RBAF600/m, RHAMM/CD168, RU1, RU2, S-100, SAGE, SART-1, SART-2, SART-3, SCC, SIRT2/m, Sp17, SSX-1, SSX-2/HOM-MEL-40, SSX-4, STAMP-1, STEAP, survivin, survivin-2B, SYT-SSX-1, SYT-SSX-2, TA-90, TAG-72, TARP, TEL-AML1, TGFbeta, TGFbetaRII, TGM-4, TPI/m, TRAG-3, TRG, TRP-1, TRP-2/6b, TRP/INT2, TRP-p8, tyrosinase, UPA, VEGF, VEGFR-2/FLK-1, and WT1, or a fragment, variant or epitope thereof. Epitopes typically comprise 5 to 15, preferably 5 to 12, more preferably 6 to 9 amino acids of the antigen, preferably in its native form.

According to another alternative, one further class of antigens as encoded by the nucleic acid molecule of the herein defined polymeric carrier cargo complex comprises allergenic antigens. Such allergenic antigens may be selected from antigens derived from different sources, e.g. from animals, plants, fungi, bacteria, etc. Allergens in this context include e.g. grasses, pollens, molds, drugs, or numerous environmental triggers, etc. Allergenic antigens typically belong to different classes of compounds, such as nucleic acids and their fragments, proteins or peptides and their fragments, carbohydrates, polysaccharides, sugars, lipids, phospholipids, etc. Of particular interest in the context of the present invention are antigens, which may be encoded by the nucleic acid molecule of the polymeric carrier cargo complex, i.e. protein or peptide antigens and their fragments or epitopes, or nucleic acids and their fragments, particularly nucleic acids and their fragments, encoding such protein or peptide antigens and their fragments or epitopes.

c) Antibodies

According to a further alternative, the nucleic acid molecule of the herein defined polymeric carrier cargo complex may encode an antibody or an antibody fragment. According to the present invention, such an antibody may be selected from any antibody, e.g. any recombinantly produced or naturally occurring antibodies, known in the art, in particular antibodies suitable for therapeutic, diagnostic or scientific purposes, or antibodies which have been identified in relation to specific cancer diseases. Herein, the term "antibody" is used in its broadest sense and specifically covers monoclonal and polyclonal antibodies (including agonist, antagonist, and blocking or neutralizing antibodies) and antibody species with polyepitopic specificity. According to the invention, the term "antibody" typically comprises any antibody known in the art (e.g. IgM, IgD, IgG, IgA and IgE antibodies), such as naturally occurring antibodies, antibodies generated by immunization in a host organism, antibodies which were isolated and identified from naturally occurring antibodies or antibodies generated by immunization in a host organism and recombinantly produced by biomolecular methods known in the art, as well as chimeric antibodies, human antibodies, humanized antibodies, bispecific antibodies, intrabodies, i.e. antibodies expressed in cells and optionally localized in specific cell compartments, and fragments and variants of the aforementioned antibodies. In general, an antibody consists of a light chain and a heavy chain both having variable and constant domains. The light chain consists of an N-terminal variable domain, $V_L$, and a C-terminal constant domain, $C_L$. In contrast, the heavy chain of the IgG antibody, for example, is comprised of an N-terminal variable domain, $V_H$, and three constant domains, $C_H1$, $C_H2$ und $C_H3$.

In the context of the present invention, antibodies as encoded by the nucleic acid molecule of the herein defined polymeric carrier cargo complex may preferably comprise full-length antibodies, i.e. antibodies composed of the full heavy and full light chains, as described above. However, derivatives of antibodies such as antibody fragments, variants or adducts may also be encoded by the nucleic acid molecule of the herein defined polymeric carrier cargo complex. Antibody fragments are preferably selected from Fab, Fab', F(ab')$_2$, Fc, Facb, pFc', Fd and Fv fragments of the aforementioned (full-length) antibodies. In general, antibody fragments are known in the art. For example, a Fab ("fragment, antigen binding") fragment is composed of one constant and one variable domain of each of the heavy and the light chain. The two variable domains bind the epitope on specific antigens. The two chains are connected via a disulfide linkage. A scFv ("single chain variable fragment") fragment, for example, typically consists of the variable domains of the light and heavy chains. The domains are linked by an artificial linkage, in general a polypeptide linkage such as a peptide composed of 15-25 glycine, proline and/or serine residues.

In the present context it is preferable that the different chains of the antibody or antibody fragment are encoded by a multicistronic nucleic acid molecule. Alternatively, the different strains of the antibody or antibody fragment are encoded by several monocistronic nucleic acid(s) (sequences).

siRNA:

According to a further particularly preferred alternative, the nucleic acid of the polymeric carrier cargo complex formed by the nucleic acid cargo and a polymeric carrier molecule according to generic formula (I) (or according to any of its subformulas herein) may be in the form of dsRNA, preferably siRNA. A dsRNA, or a siRNA, is of interest particularly in connection with the phenomenon of RNA interference. The *in vitro* technique of RNA interference (RNAi) is based on double-stranded RNA molecules (dsRNA), which trigger the sequence-specific suppression of gene expression (Zamore (2001) Nat. Struct. Biol. 9: 746-750; Sharp (2001) Genes Dev. 5:485-490: Hannon (2002) Nature 41: 244-251). In the transfection of mammalian cells with long dsRNA, the activation of protein kinase R and RnaseL brings about unspecific effects, such as, for example, an interferon response (Stark et al. (1998) Annu. Rev. Biochem. 67: 227-264; He and Katze (2002) Viral Immunol. 15: 95-119). These unspecific effects are avoided when shorter, for example 21- to 23-mer, so-called siRNA (small interfering RNA), is used, because unspecific effects are not triggered by siRNA that is shorter than 30 bp (Elbashir et al. (2001) Nature 411: 494-498).

The nucleic acid of the polymeric carrier cargo complex may thus be a double-stranded RNA (dsRNA) having a length of from 17 to 29, preferably from 19 to 25, and preferably being at least 90%, more preferably 95% and especially 100% (of the nucleotides of a dsRNA) complementary to a section of the nucleic acid sequence of a (therapeutically relevant) protein or antigen described (as active ingredient) hereinbefore, either a coding or a non-coding section, preferably a coding section. 90% complementary means that with a length of a dsRNA described herein of, for example, 20 nucleotides, this contains not more than 2 nucleotides without corresponding complementarity with the corresponding section of the mRNA. The sequence of the double-stranded RNA used according to the invention as the nucleic acid of the polymeric carrier cargo complex is, however, preferably wholly complementary in its general structure with a section of the nucleic acid of a therapeutically relevant protein or antigen described hereinbefore. In this context the nucleic acid of the polymeric carrier cargo complex formed by the nucleic acid cargo and a polymeric carrier molecule according to generic formula (I) may be a dsRNA having the general structure 5'-(N$_{17-29}$)-3', preferably having the general structure 5'-(N$_{19-25}$)-3', more preferably having the general structure 5'-(N$_{19-24}$)-3', or yet more preferably having the general structure 5'-(N$_{21-23}$)-3', wherein for each general structure each N is a (preferably different) nucleotide of a section of the mRNA of a therapeutically relevant protein or antigen described hereinbefore, preferably being selected from a continuous number of 17 to 29 nucleotides of the mRNA of a therapeutically relevant protein or antigen and being present in the general structure 5'-(N$_{17-29}$)-3' in their natural order. In principle, all the sections having a length of from 17 to 29, preferably from 19 to 25, base pairs that occur in the coding region of the mRNA can serve as target sequence for a dsRNA herein. Equally, dsRNAs used as nucleic acid of the polymeric carrier cargo complex can also be directed against nucleotide sequences of a (therapeutically relevant) protein or antigen described (as active ingredient) hereinbefore that do not lie in the coding region, in particular in the 5' non-coding region of the mRNA, for example, therefore, against non-coding regions of the mRNA having a regulatory function. The target sequence of the dsRNA used as nucleic acid of the polymeric carrier cargo complex can therefore lie in the translated and untranslated region of the mRNA and/or in the region of the control elements of a protein or antigen described hereinbefore. The target sequence of a dsRNA used as nucleic acid of the polymeric carrier cargo complex can also lie in the overlapping region of untranslated and translated sequence; in particular, the target sequence can comprise at least one nucleotide upstream of the start triplet of the coding region of the mRNA.

In the context of the present invention siRNA directed against e.g. PDGF, VEGF, ICAM-1, VCAM-1, E-selektin, TNFa, IL-6 (in principle all pro inflammatory interleukins MMPs etc.) to prevent restenosis are particularly preferred.

Immunostimulatory nucleic acids:

a) Immunostimulatory CpG nucleic acids:

According to another alternative, the nucleic acid of the polymeric carrier cargo complex formed by the nucleic acid cargo and a polymeric carrier molecule according to generic formula (I) (or according to any of its subformulas herein) may be in the form of a a(n) (immunostimulatory) CpG nucleic acid, which is a CpG-RNA or CpG-DNA, which preferably induces an innate immune response. A CpG-RNA or CpG-DNA used according to the invention can be a single-stranded CpG-DNA (ss CpG-DNA), a double-stranded CpG-DNA (dsDNA), a single-stranded CpG-RNA (ss CpG-RNA) or a double-stranded CpG-RNA (ds CpG-RNA). The CpG nucleic acid used according to the invention is preferably in the form of CpG-RNA, more preferably in the form of single-stranded CpG-RNA (ss CpG-RNA). Also preferably, such CpG nucleic acids have a length as described above. Preferably the CpG motifs are unmethylated.

b) Immunostimulatory RNA (isRNA):

Likewise, according to a further alternative, the nucleic acid of the polymeric carrier cargo complex formed by the nucleic acid cargo and a polymeric carrier molecule according to generic formula (I) (or according to any of its subformulas herein) may be in the form of a of an immunostimulatory RNA (isRNA), which preferably elicits an innate immune response. Such an immunostimulatory RNA may be any (double-stranded or single-stranded) RNA, e.g. a coding RNA, as defined herein. Preferably, the immunostimulatory RNA may be a single-stranded, a double-stranded or a partially double-stranded RNA, more preferably a single-stranded RNA, and/or a circular or linear RNA, more preferably a linear RNA. More preferably, the immunostimulatory RNA may be a (linear) single-stranded RNA. Even more preferably, the immunostimulatory RNA may be a (long) (linear) single-stranded) non-coding RNA. In this context it is particular preferred that the isRNA carries a triphosphate at its 5'-end which is the case for *in vitro* transcribed RNA. An immunostimulatory RNA may also occur as a short RNA oligonucleotide as defined herein. An immunostimulatory RNA as used herein may furthermore be selected from any class of RNA molecules, found in nature or being prepared synthetically, and which can induce an innate immune response and may support an adaptive immune response induced by an antigen. In this context, an immune response may occur in various ways. A substantial factor for a suitable (adaptive) immune response is the stimulation of different T-cell sub-populations. T-lymphocytes are typically divided into two sub-populations, the T-helper 1 (Th1) cells and the T-helper 2 (Th2) cells, with which the immune system is capable of destroying intracellular (Th1) and extracellular (Th2) pathogens (e.g. antigens). The two Th cell populations differ in the pattern of the effector proteins (cytokines) produced by them. Thus, Th1 cells assist the cellular immune response by activation of macrophages and cytotoxic T-cells. Th2 cells, on the other hand, promote the humoral immune response by stimulation of B-cells for conversion into plasma cells and by formation of antibodies (e.g. against antigens). The Th1/Th2 ratio is therefore of great importance in the induction and maintenance of an adaptive immune response. In connection with the present invention, the Th1/Th2 ratio of the (adaptive) immune response is preferably shifted in the direction towards the cellular response (Th1 response) and a cellular immune response is thereby induced. According to one example, the innate immune system which may support an adaptive immune response, may be activated by ligands of Toll-like receptors (TLRs). TLRs are a family of highly conserved pattern recognition receptor (PRR) polypeptides that recognize pathogen-associated molecular patterns (PAMPs) and play a critical role in innate immunity in mammals. Currently at least thirteen family members, designated TLR1 - TLR13 (Toll-like receptors: TLR1, TLR2, TLR3, TLR4, TLR5, TLR6, TLR7, TLR8, TLR9, TLR10, TLR11, TLR12 or TLR13), have been identified. Furthermore, a number of specific TLR ligands have been identified. It was e.g. found that unmethylated bacterial DNA and synthetic analogs thereof (CpG DNA) are ligands for TLR9 (Hemmi H et al. (2000) Nature 408:740-5; Bauer S et al. (2001) Proc NatlAcadSci USA 98, 9237-42). Furthermore, it has been reported that ligands for certain TLRs include certain nucleic acid molecules and that certain types of RNA are immunostimulatory in a sequence-independent or sequence-dependent manner, wherein these various immunostimulatory RNAs may e.g. stimulate TLR3, TLR7, or TLR8, or intracellular receptors such as RIG-I, MDA-5, etc. E.g. Lipford *et al.* determined certain G,U-containing oligoribonucleotides as immunostimulatory by acting via TLR7 and TLR8 (see WO 03/086280). The immunostimulatory G,U-containing oligoribonucleotides described by Lipford *et al.* were believed to be derivable from RNA sources including ribosomal RNA, transfer RNA, messenger RNA, and viral RNA.

The immunostimulatory RNA (isRNA) used as the nucleic acid molecule of the polymeric carrier cargo complex formed by the nucleic acid cargo and a polymeric carrier molecule according to generic formula (I) (or according to any of its subformulas herein) may thus comprise any RNA sequence known to be immunostimulatory, including, without being limited thereto, RNA sequences representing and/or encoding ligands of TLRs, preferably selected from human family members TLR1 - TLR10 or murine family members TLR1 - TLR13, more preferably selected from (human) family members TLR1 - TLR10, even more preferably from TLR7 and TLR8, ligands for intracellular receptors for RNA (such as RIG-I or MDA-5, etc.) (see e.g. Meylan, E., Tschopp, J. (2006). Toll-like receptors and RNA helicases: two parallel ways to trigger antiviral responses. Mol. Cell 22, 561-569), or any other immunostimulatory RNA sequence. Furthermore, (classes of) immunostimulatory RNA molecules, used as the nucleic acid molecule of the polymeric carrier cargo complex may include any other RNA capable of eliciting an immune response. Without being limited thereto, such an immunostimulatory RNA may include ribosomal RNA (rRNA), transfer RNA (tRNA), messenger RNA (mRNA), and viral RNA (vRNA). Such an immunostimulatory RNA may comprise a length of 1000 to 5000, of 500 to 5000, of 5 to 5000, or of 5 to 1000, 5 to 500, 5 to 250, of 5 to 100, of 5 to 50 or of 5 to 30 nucleotides.

According to a particularly preferred aspect of this embodiment of the present invention, such immunostimulatory nucleic acid sequences, which are DNA or RNA sequences, particularly isRNA, consist of or comprise a nucleic acid of formula (III) or (IV):

$$G_l X_m G_n, \qquad \text{(formula (III))}$$

wherein:

G    is guanosine, uracil or an analogue of guanosine or uracil;
X    is guanosine, uracil, adenosine, thymidine, cytosine or an analogue of the above-mentioned nucleotides;
l    is an integer from 1 to 40,
    wherein
    when l = 1 G is guanosine or an analogue thereof,
    when l > 1 at least 50% of the nucleotides are guanosine or an analogue thereof;
m    is an integer and is at least 3;
    wherein
    when m = 3 X is uracil or an analogue thereof,
    when m > 3 at least 3 successive uracils or analogues of uracil occur;
n    is an integer from 1 to 40,
    wherein
    when n = 1 G is guanosine or an analogue thereof,
    when n > 1 at least 50% of the nucleotides are guanosine or an analogue thereof.

$$C_l X_m C_n, \qquad \text{(formula (IV))}$$

wherein:

C    is cytosine, uracil or an analogue of cytosine or uracil;
X    is guanosine, uracil, adenosine, thymidine, cytosine or an analogue of the above-mentioned nucleotides;
l    is an integer from 1 to 40,
    wherein
    when l = 1 C is cytosine or an analogue thereof,
    when l > 1 at least 50% of the nucleotides are cytosine or an analogue thereof;
m    is an integer and is at least 3;
    wherein
    when m = 3 X is uracil or an analogue thereof,
    when m > 3 at least 3 successive uracils or analogues of uracil occur;
n    is an integer from 1 to 40,
    wherei n
    when n = 1 C is cytosine or an analogue thereof,
    when n > 1 at least 50% of the nucleotides are cytosine or an analogue thereof.

The nucleic acids of formula (III) or (IV), which may be used as the nucleic acid cargo of the polymeric carrier cargo complex may be relatively short nucleic acid molecules with a typical length of approximately from 5 to 100 (but may also be longer than 100 nucleotides for specific embodiments, e.g. up to 200 nucleotides), from 5 to 90 or from 5 to 80 nucleotides, preferably a length of approximately from 5 to 70, more preferably a length of approximately from 8 to 60 and, more preferably a length of approximately from 15 to 60 nucleotides, more preferably from 20 to 60, most preferably from 30 to 60 nucleotides. If the nucleic acid of the nucleic acid cargo complex has a maximum length of e.g. 100 nucleotides, m will typically be <=98. The number of nucleotides G in the nucleic acid of formula (III) is determined by l or n. l and n, independently of one another, are each an integer from 1 to 40, wherein when l or n = 1 G is guanosine or an analogue thereof, and when l or n > 1 at least 50% of the nucleotides are guanosine or an analogue thereof. For example, without implying any limitation, when l or n = 4 $G_l$ or $G_n$ can be, for example, a GUGU, GGUU, UGUG, UUGG, GUUG, GGGU, GGUG, GUGG, UGGG or GGGG, etc.; when l or n = 5 $G_l$ or $G_n$ can be, for example, a GGGUU, GGUGU, GUGGU, UGGGU, UGGUG, UGUGG, UUGGG, GUGUG, GGGGU, GGGUG, GGUGG, GUGGG, UGGGG, or GGGGG, etc.; etc. A nucleotide adjacent to $X_m$ in the nucleic acid of formula (III) according to the invention is preferably not a uracil. Similarly, the number of nucleotides C in the nucleic acid of formula (IV) according to the invention is determined by l or n. l and n, independently of one another, are each an integer from 1 to 40, wherein when l or n = 1 C is cytosine or an analogue thereof, and when l or n > 1 at least 50% of the nucleotides are cytosine or an analogue thereof. For example, without implying any limitation, when l or n = 4, $C_l$ or $C_n$ can be, for example, a CUCU, CCUU, UCUC, UUCC, CUUC, CCCU, CCUC, CUCC, UCCC or CCCC, etc.; when l or n = 5 $C_l$ or $C_n$ can be, for example, a CCCUU, CCUCU, CUCCU, UCCCU, UCCUC, UCUCC, UUCCC, CUCUC, CCCCU, CCCUC, CCUCC, CUCCC, UCCCC, or CCCCC, etc.; etc. A nucleotide adjacent to $X_m$ in the nucleic acid of formula (IV) according to the invention is preferably not a uracil. Preferably, for formula (III), when l or n > 1, at least 60%, 70%, 80%, 90% or even 100% of the nucleotides are guanosine or an analogue thereof, as defined above. The remaining nucleotides to 100% (when guanosine constitutes less than 100% of the nucleotides) in the flanking sequences $G_1$ and/or $G_n$ are uracil or an analogue thereof, as defined hereinbefore. Also preferably, l and n, independently of one another, are each an integer from 2 to 30, more preferably an integer from 2 to 20 and yet more preferably an integer from 2 to 15. The lower limit of l or n can be varied if necessary and is at least 1, preferably at least 2, more preferably at least 3, 4, 5, 6, 7, 8, 9 or 10. This definition applies correspondingly to formula (IV).

According to a further particularly preferred aspect of this embodiment, such immunostimulatory nucleic acid sequences, which are DNA or RNA sequences, particularly isRNA, consist of or comprise a nucleic acid of formula (V) or (VI):

$$(N_uG_lX_mG_nN_v)_a, \qquad \text{(formula (V))}$$

wherein:

G is guanosine (guanine), uridine (uracil) or an analogue of guanosine (guanine) or uridine (uracil), preferably guanosine (guanine) or an analogue thereof;

X is guanosine (guanine), uridine (uracil), adenosine (adenine), thymidine (thymine), cytidine (cytosine), or an analogue of these nucleotides (nucleosides), preferably uridine (uracil) or an analogue thereof;

N is a nucleic acid sequence having a length of about 4 to 50, preferably of about 4 to 40, more preferably of about 4 to 30 or 4 to 20 nucleic acids, each N independently being selected from guanosine (guanine), uridine (uracil), adenosine (adenine), thymidine (thymine), cytidine (cytosine) or an analogue of these nucleotides (nucleosides);

a is an integer from 1 to 20, preferably from 1 to 15, most preferably from 1 to 10;

l is an integer from 1 to 40,
wherein when l = 1, G is guanosine (guanine) or an analogue thereof,
when l > 1, at least 50% of these nucleotides (nucleosides) are guanosine (guanine) or an analogue thereof;

m is an integer and is at least 3;
wherein when m = 3, X is uridine (uracil) or an analogue thereof, and
when m > 3, at least 3 successive uridines (uracils) or analogues of uridine (uracil) occur;

n is an integer from 1 to 40,
wherein when n = 1, G is guanosine (guanine) or an analogue thereof,
when n > 1, at least 50% of these nucleotides (nucleosides) are guanosine (guanine) or an analogue thereof;

u,v may be independently from each other an integer from 0 to 50,
preferably wherein when u = 0, v ≥ 1, or
when v = 0, u ≥ 1;

wherein the nucleic acid molecule of formula (V) has a length of at least 50 nucleotides, preferably of at least 100 nucleotides, more preferably of at least 150 nucleotides, even more preferably of at least 200 nucleotides and most preferably of at least 250 nucleotides.

$$(N_uC_lX_mC_nN_v)_a \qquad \text{(formula (VI))}$$

wherein:

C     is cytidine (cytosine), uridine (uracil) or an analogue of cytidine (cytosine) or uridine (uracil), preferably cytidine (cytosine) or an analogue thereof;

X     is guanosine (guanine), uridine (uracil), adenosine (adenine), thymidine (thymine), cytidine (cytosine) or an analogue of the above-mentioned nucleotides (nucleosides), preferably uridine (uracil) or an analogue thereof;

N     is each a nucleic acid sequence having independent from each other a length of about 4 to 50, preferably of about 4 to 40, more preferably of about 4 to 30 or 4 to 20 nucleic acids, each N independently being selected from guanosine (guanine), uridine (uracil), adenosine (adenine), thymidine (thymine), cytidine (cytosine) or an analogue of these nucleotides (nucleosides);

a     is an integer from 1 to 20, preferably from 1 to 15, most preferably from 1 to 10;

l     is an integer from 1 to 40,
wherein when $l = 1$, C is cytidine (cytosine) or an analogue thereof,
when $l > 1$, at least 50% of these nucleotides (nucleosides) are cytidine (cytosine) or an analogue thereof;

m     is an integer and is at least 3;
wherein when $m = 3$, X is uridine (uracil) or an analogue thereof,
when $m > 3$, at least 3 successive uridines (uracils) or analogues of uridine (uracil) occur;

n     is an integer from 1 to 40,
wherein when $n = 1$, C is cytidine (cytosine) or an analogue thereof,
when $n > 1$, at least 50% of these nucleotides (nucleosides) are cytidine (cytosine) or an analogue thereof.

u, v     may be independently from each other an integer from 0 to 50,
preferably wherein when $u = 0$, $v \geq 1$, or
when $v = 0$, $u \geq 1$;

wherein the nucleic acid molecule of formula (VI) according to the invention has a length of at least 50 nucleotides, preferably of at least 100 nucleotides, more preferably of at least 150 nucleotides, even more preferably of at least 200 nucleotides and most preferably of at least 250 nucleotides.

For formula (VI), any of the definitions given above for elements N (i.e. $N_u$ and $N_v$) and X ($X_m$), particularly the core structure as defined above, as well as for integers a, l, m, n, u and v, similarly apply to elements of formula (V) correspondingly, wherein in formula (VI) the core structure is defined by $C_l X_m C_n$. The definition of bordering elements $N_u$ and $N_v$ is identical to the definitions given above for $N_u$ and $N_v$.

According to a very particularly preferred aspect of this embodiment, the nucleic acid molecule according to formula (V), which is a DNA or RNA, may be selected from e.g. any of the following sequences:

UAGCGAAGCUCUUGGACCUAGGUUUUUUUUUUUUUUUGGGUGCGUUCCUAGAAG
UACACG (SEQ ID NO: 118)

UAGCGAAGCUCUUGGACCUAGGUUUUUUUUUUUUUUUGGGUGCGUUCCUAGAAG
UACACGAUCGCUUCGAGAACCUGGAUCCAAAAAAAAAAAAAAAACCCACGCAAGGAUCU
UCAUGUGC (SEQ ID NO: 119)

GGGAGAAAGCUCAAGCUUGGAGCAAUGCCCGCACAUUGAGGAAACCGAGUUGCAUA
UCUCAGAGUAUUGGCCCCCGUGUAGGUUAUUCUUGACAGACAGUGGAGCUUAUUCA
CUCCCAGGAUCCGAGUCGCAUACUACGGUACUGGUGACAGACCUAGGUCGUCAGUU

GACCAGUCCGCCACUAGACGUGAGUCCGUCAAAGCAGUUAGAUGUUACACUCUAUUA
GAUC (SEQ ID NO: 120)

GGGAGAAAGCUCAAGCUUGGAGCAAUGCCCGCACAUUGAGGAAACCGAGUUGCAUA
UCUCAGAGUAUUGGCCCCGUGUAGGUUAUUCUUGACAGACAGUGGAGCUUAUUCA
CUCCCAGGAUCCGAGUCGCAUACUACGGUACUGGUGACAGACCUAGGUCGUCAGUU
GACCAGUCCGCCACUAGACGUGAGUCCGUCAAAGCAGUUAGAUGUUACACUCUAUUA
GAUCUCGGAUUACAGCUGGAAGGAGCAGGAGUAGUGUUCUUGCUCUAAGUACCGAG
UGUGCCCAAUACCCGAUCAGCUUAUUAACGAACGGCUCCUCCUCUUAGACUGCAGCG
UAAGUGCGGAAUCUGGGGAUCAAAUUACUGACUGCCUGGAUUACCCUCGGACAUAU
AACCUUGUAGCACGCUGUUGCUGUAUAGGUGACCAACGCCCACUCGAGUAGACCAGC
UCUCUUAGUCCGGACAAUGAUAGGAGGCGCGGUCAAUCUACUUCUGGCUAGUUAAG
AAUAGGCUGCACCGACCUCUAUAAGUAGCGUGUCCUCUAG (SEQ ID NO: 121)

GGGAGAAAGCUCAAGCUUGGAGCAAUGCCCGCACAUUGAGGAAACCGAGUUGCAUA
UCUCAGAGUAUUGGCCCCGUGUAGGUUAUUCUUGACAGACAGUGGAGCUUAUUCA
CUCCCAGGAUCCGAGUCGCAUACUACGGUACUGGUGACAGACCUAGGUCGUCAGUU
GACCAGUCCGCCACUAGACGUGAGUCCGUCAAAGCAGUUAGAUGUUACACUCUAUUA
GAUCUCGGAUUACAGCUGGAAGGAGCAGGAGUAGUGUUCUUGCUCUAAGUACCGAG
UGUGCCCAAUACCCGAUCAGCUUAUUAACGAACGGCUCCUCCUCUUAGACUGCAGCG
UAAGUGCGGAAUCUGGGGAUCAAAUUACUGACUGCCUGGAUUACCCUCGGACAUAU
AACCUUGUAGCACGCUGUUGCUGUAUAGGUGACCAACGCCCACUCGAGUAGACCAGC
UCUCUUAGUCCGGACAAUGAUAGGAGGCGCGGUCAAUCUACUUCUGGCUAGUUAAG
AAUAGGCUGCACCGACCUCUAUAAGUAGCGUGUCCUCUAGAGCUACGCAGGUUCGC
AAUAAAAGCGUUGAUUAGUGUGCAUAGAACAGACCUCUUAUUCGGUGAAACGCCAG
AAUGCUAAAUUCCAAUAACUCUUCCCAAAACGCGUACGGCCGAAGACGCGCGCUUAU
CUUGUGUACGUUCUCGCACAUGGAAGAAUCAGCGGGCAUGGUGGUAGGGCAAUAG
GGGAGCUGGGUAGCAGCGAAAAAGGGCCCCUGCGCACGUAGCUUCGCUGUUCGUCU
GAAACAACCCGGCAUCCGUUGUAGCGAUCCCGUUAUCAGUGUUAUUCUUGUGCGCA
CUAAGAUUCAUGGUGUAGUCGACAAUAACAGCGUCUUGGCAGAUUCUGGUCACGUG
CCCUAUGCCCGGGCUUGUGCCUCUCAGGUGCACAGCGAUACUUAAAGCCUUCAAGG
UACUCGACGUGGGUACCGAUUCGUGACACUUCCUAAGAUUAUUCCACUGUGUUAGC
CCCGCACCGCCGACCUAAACUGGUCCAAUGUAUACGCAUUCGCUGAGCGGAUCGAUA
AUAAAAGCUUGAAUU (SEQ ID NO: 122)

GGGAGAAAGCUCAAGCUUAUCCAAGUAGGCUGGUCACCUGUACAACGUAGCCGGUA
UUUUUUUUUUUUUUUUUUUUUGACCGUCUCAAGGUCCAAGUUAGUCUGCCUAUA
AAGGUGCGGAUCCACAGCUGAUGAAAGACUUGUGCGGUACGGUUAAUCUCCCCUUU
UUUUUUUUUUUUUUUUUUAGUAAAUGCGUCUACUGAAUCCAGCGAUGAUGCUGGC
CCAGAUC (SEQ ID NO: 123)

GGGAGAAAGCUCAAGCUUAUCCAAGUAGGCUGGUCACCUGUACAACGUAGCCGGUA
UUUUUUUUUUUUUUUUUUUUUGACCGUCUCAAGGUCCAAGUUAGUCUGCCUAUA
AAGGUGCGGAUCCACAGCUGAUGAAAGACUUGUGCGGUACGGUUAAUCUCCCCUUU
UUUUUUUUUUUUUUUUUUAGUAAAUGCGUCUACUGAAUCCAGCGAUGAUGCUGGC
CCAGAUCUUCGACCACAAGUGCAUAUAGUAGUCAUCGAGGGUCGCCUUUUUUUUUU
UUUUUUUUUUUUUGGCCCAGUUCUGAGACUUCGCUAGAGACUACAGUUACAGCUG
CAGUAGUAACCACUGCGGCUAUUGCAGGAAAUCCCGUUCAGGUUUUUUUUUUUUU
UUUUUUUUCCGCUCACUAUGAUUAAGAACCAGGUGGAGUGUCACUGCUCUCGAGG
UCUCACGAGAGCGCUCGAUACAGUCCUUGGAAGAAUCUUUUUUUUUUUUUUUUUUU
UUUUGUGCGACGAUCACAGAGAACUUCUAUUCAUGCAGGUCUGCUCUA (R 722 SEQ
ID NO: 124)

GGGAGAAAGCUCAAGCUUAUCCAAGUAGGCUGGUCACCUGUACAACGUAGCCGGUA
UUUUUUUUUUUUUUUUUUUUUGACCGUCUCAAGGUCCAAGUUAGUCUGCCUAUA
AAGGUGCGGAUCCACAGCUGAUGAAAGACUUGUGCGGUACGGUUAAUCUCCCUUU
UUUUUUUUUUUUUUUUUUUAGUAAAUGCGUCUACUGAAUCCAGCGAUGAUGCUGGC
CCAGAUCUUCGACCACAAGUGCAUAUAGUAGUCAUCGAGGGUCGCCUUUUUUUUUU
UUUUUUUUUUUUUGGCCCAGUUCGAGACUUCGCUAGAGACUACAGUUACAGCUG
CAGUAGUAACCACUGCGGCUAUUGCAGGAAAUCCCGUUCAGGUUUUUUUUUUUUU
UUUUUUUUCCGCUCACUAUGAUUAAGAACCAGGUGGAGUGUCACUGCUCUCGAGG
UCUCACGAGAGCGCUCGAUACAGUCCUUGGAAGAAUCUUUUUUUUUUUUUUUUUUU
UUUUGUGCGACGAUCACAGAGAACUUCUAUUCAUGCAGGUCUGCUCUAGAACGAAC
UGACCUGACGCCUGAACUUAUGAGCGUGCGUAUUUUUUUUUUUUUUUUUUUUUUUU
UCCUCCCAACAAAUGUCGAUCAAUAGCUGGGCUGUUGGAGACGCGUCAGCAAAUGCC
GUGGCUCCAUAGGACGUGUAGACUUCUAUUUUUUUUUUUUUUUUUUUUUUUCCCGG
GACCACAAAUAAUAUUCUUGCUUGGUUGGGCGCAAGGGCCCCGUAUCAGGUCAUAA
ACGGGUACAUGUUGCACAGGCUCCUUUUUUUUUUUUUUUUUUUUUUUUUCGCUGAG
UUAUUCCGGUCUCAAAAGACGGCAGACGUCAGUCGACAACACGGUCUAAAGCAGUGC
UACAAUCUGCCGUGUUCGUGUUUUUUUUUUUUUUUUUUUUGUGAACCUACACGGC
GUGCACUGUAGUUCGCAAUUCAUAGGGUACCGGCUCAGAGUUAUGCCUUGGUUGA
AAACUGCCCAGCAUACUUUUUUUUUUUUUUUUUUUUUUUCAUAUUCCCAUGCUAAGCAA
GGGAUGCCGCGAGUCAUGUUAAGCUUGAAUU (SEQ ID NO: 125)

According to another very particularly preferred embodiment, the nucleic acid molecule according to formula (VI), which is a DNA or RNA, may be selected from e.g. any of the following sequences:

UAGCGAAGCUCUUGGACCUACCUUUUUUUUUUUUUUUCCCUGCGUUCCUAGAAGUA
CACG (SEQ ID NO: 126)

or

UAGCGAAGCUCUUGGACCUACCUUUUUUUUUUUUUUUUCCCUGCGUUCCUAGAAGU
ACACGAUCGCUUCGAGAACCUGGAUGGAAAAAAAAAAAAAAAGGGACGCAAGGAUCU
UCAUGUGC (SEQ ID NO: 127)

In a further preferred embodiment the nucleic acid molecule of the herein defined polymeric carrier cargo complex, which is a DNA or RNA, may also occur in the form of a modified nucleic acid.

According to a further aspect, the nucleic acid molecule of the herein defined polymeric carrier cargo complex, which is a DNA or RNA, may be provided as a "stabilized nucleic acid", e.g. a stabilized RNA or DNA, more preferably as a RNA that is essentially resistant to *in vivo* degradation (e.g. by an exo- or endo-nuclease).

In this context, the nucleic acid molecule of the herein defined polymeric carrier cargo complex, which is a DNA or RNA, may contain backbone modifications, sugar modifications or base modifications. A backbone modification in connection with the present invention is a modification in which phosphates of the backbone of the nucleotides contained in the nucleic acid molecule of the polymeric carrier cargo complex are chemically modified. A sugar modification in connection with the present invention is a chemical modification of the sugar of the nucleotides of the nucleic acid molecule of the polymeric carrier cargo complex. Furthermore, a base modification in connection with the present invention is a chemical modification of the base moiety of the nucleotides of the nucleic acid molecule of the polymeric carrier cargo complex.

According to a further aspect, the nucleic acid molecule of the herein defined polymeric carrier cargo complex, which is a DNA or RNA, can contain a lipid modification.

The nucleic acid of the polymeric carrier cargo complex as defined herein, which is a DNA or RNA, may also be in the form of a modified nucleic acid, wherein any modification, as defined herein, may be introduced into the nucleic acid. Modifications as defined herein preferably lead to a further stabilized nucleic acid.

According to one aspect, the nucleic acid of the polymeric carrier cargo complex as defined herein, which is a DNA or RNA, may thus be provided as a "stabilized nucleic acid", preferably as a stabilized mRNA, more preferably as an mRNA that is essentially resistant to *in vivo* degradation (e.g. by an exo- or endo-nuclease). Such stabilization can be effected, for example, by a modified phosphate in which phosphates of the backbone of the nucleotides contained in the nucleic acid are chemically modified. The nucleic acid of the polymeric carrier cargo complex may additionally or alternatively also contain sugar or base modifications. The nucleic acid of the polymeric carrier cargo complex, particularly if provided as an mRNA, can also be stabilized against degradation by RNases by the addition of a so-called "5' cap" structure. Particular preference is given in this connection to an m7G(5')ppp (5'(A,G(5')ppp(5')A or G(5')ppp(5')G as the 5' cap" structure. According to a further aspect, the nucleic acid of the polymeric carrier cargo complex may contain, especially if the nucleic acid is in the form of an mRNA, a poly-A tail on the 3' terminus of typically about 10 to 200 adenosine nucleotides, preferably about 10 to 100 adenosine nucleotides, more preferably about 20 to 100 adenosine nucleotides or even more preferably about 40 to 80 adenosine nucleotides. According to a further aspect, the nucleic acid of the polymeric carrier cargo complex may contain, especially if the nucleic acid is in the form of an mRNA, a poly-C tail on the 3' terminus of typically about 10 to 200 cytosine nucleotides, preferably about 10 to 100 cytosine nucleotides, more preferably about 20 to 70 cytosine nucleotides or even more preferably about 20 to 60 or even 10 to 40 cytosine nucleotides. According to another aspect, the nucleic acid of the polymeric carrier cargo complex may be modified, and thus stabilized, especially if the nucleic acid is in the form of an mRNA, by modifying the G/C content of the nucleic acid, particularly an mRNA, preferably of the coding region thereof.

In a particularly preferred aspect of the present invention, the G/C content of the coding region of the nucleic acid of the polymeric carrier cargo complex, especially if the nucleic acid is in the form of an mRNA, is modified, particularly increased, compared to the G/C content of the coding region of its particular wild-type mRNA, i.e. the unmodified mRNA. The encoded amino acid sequence of the at least one mRNA is preferably not modified compared to the coded amino acid sequence of the particular wild-type mRNA. Preferably, the G/C content of the coding region of nucleic acid of the polymeric carrier cargo complex, especially if the nucleic acid is in the form of an mRNA, is increased by at least 7%, more preferably by at least 15%, particularly preferably by at least 20%, compared to the G/C content of the coded region of the wild-type mRNA which codes for an antigen, antigenic protein or antigenic peptide as deinined herein or its fragment or variant thereof. According to a specific embodiment at least 5%, 10%, 20%, 30%, 40%, 50%, 60%, more preferably at least 70 %, even more preferably at least 80% and most preferably at least 90%, 95% or even 100% of the substitutable codons in the region coding for a protein or peptide as defined herein or its fragment or variant thereof or the whole sequence of the wild type mRNA sequence are substituted, thereby increasing the GC/content of said sequence. In this context, it is particularly preferable to increase the G/C content of the nucleic acid of the polymeric carrier cargo complex, especially if the nucleic acid is in the form of an mRNA, to the maximum (i.e. 100% of the substitutable codons), in particular in the region coding for a protein, compared to the wild-type sequence. According to the invention, a further preferred modification of the nucleic acid of the polymeric carrier cargo complex, especially if the nucleic acid is in the form of an mRNA, the region which codes for the adjuvant protein is modified compared to the corresponding region of the wild-type mRNA such that at least one codon of the wild-type sequence which codes for a tRNA which is relatively rare in the cell is exchanged for a codon which codes for a tRNA which is relatively frequent in the cell and carries the same amino acid as the relatively rare tRNA. By this modification, the sequences of the nucleic acid, especially if the nucleic acid is in the form of an mRNA, is modified such that codons for which frequently occurring tRNAs are available are inserted. In other words, according to the invention, by this modification all codons of the wild-type sequence which code for a tRNA which is relatively rare in the cell can in each case be exchanged for a codon which codes for a tRNA which is relatively frequent in the cell and which, in each case, carries the same amino acid as the relatively rare tRNA.

Which tRNAs occur relatively frequently in the cell and which, in contrast, occur relatively rarely is known to a person skilled in the art; cf. e.g. Akashi, Curr. Opin. Genet. Dev. 2001, 11(6): 660-666. The codons which use for the particular amino acid the tRNA which occurs the most frequently, e.g. the Gly codon, which uses the tRNA which occurs the most frequently in the (human) cell, are particularly preferred.

Nucleic acid molecules used according to the present invention as defined herein may be prepared using any method known in the art, including synthetic methods such as e.g. solid phase synthesis, as well as *in vitro* methods, such as *in vitro* transcription reactions or *in vivo* reactions, such as *in vivo* propagation of DNA plasmids in bacteria.

According to another particularly preferred embodiment, the nucleic acid of the polymeric carrier cargo complex, which is a DNA or RNA, especially if the nucleic acid is in the form of a coding nucleic acid, preferably an mRNA, may additionally or alternatively encode a secretory signal peptide. Such signal peptides are sequences, which typically exhibit a length of about 15 to 30 amino acids and are preferably located at the N-terminus of the encoded peptide, without being limited thereto. Signal peptides as defined herein preferably allow the transport of the protein or peptide as encoded by the nucleic acid of the present invention, especially if the nucleic acid is in the form of an mRNA, into a defined cellular compartment, preferably the cell surface, the endoplasmic reticulum (ER) or the endosomal-lysosomal compartment.

Any of the above modifications may be applied to the nucleic acid of the polymeric carrier cargo complex, which is a DNA or RNA, especially if the nucleic acid is in the form of an mRNA, and further to any nucleic acid as used in the context of the present invention and may be, if suitable or necessary, be combined with each other in any combination, provided, these combinations of modifications do not interfere with each other in the respective nucleic acid. A person skilled in the art will be able to take his choice accordingly.

Proteins or peptides as encoded by the nucleic acid of the polymeric carrier cargo complex as defined herein, may comprise fragments or variants of those sequences. Additionally, the nucleic acid of the polymeric carrier cargo complex may comprise fragments or variants of those coding sequences. Such fragments or variants may typically comprise a sequence having a sequence identity with one of the above mentioned proteins or peptides or sequences of their encoding nucleic acid sequences of at least 5%, 10%, 20%, 30%, 40%, 50%, 60%, preferably at least 70%, more preferably at least 80%, equally more preferably at least 85%, even more preferably at least 90% and most preferably at least 95% or even 97%, to the entire wild-type sequence, either on nucleic acid level or on amino acid level.

"Fragments" of proteins or peptides in the context of the present invention may comprise a sequence of an protein or peptide as defined herein, which is, with regard to its amino acid sequence (or its encoded nucleic acid sequence), N-terminally, C-terminally and/or intrasequentially truncated compared to the amino acid sequence of the original (native) protein (or its encoded nucleic acid sequence). Such truncation may thus occur either on the amino acid level or correspondingly on the nucleic acid level. A sequence identity with respect to such a fragment as defined herein may therefore preferably refer to the entire protein or peptide as defined herein or to the entire (coding) nucleic acid sequence of such a protein or peptide. The same applies accordingly to nucleic acids.

Such fragments of proteins or peptides in the context of the present invention may furthermore comprise a sequence of a protein or peptide as defined herein, which has a length of about 6 to about 20 or even more amino acids, e.g. fragments as processed and presented by MHC class I molecules, preferably having a length of about 8 to about 10 amino acids, e.g. 8, 9, or 10, (or even 6, 7, 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T-cells in form of a complex consisting of the peptide fragment and an MHC molecule, i.e. the fragments are typically not recognized in their native form.

The fragments of proteins or peptides as defined herein may also comprise epitopes of those proteins or peptides. Epitopes (also called "antigen determinants") in the context of the present invention are typically fragments located on the outer surface of (native) proteins or peptides as defined herein, preferably having 5 to 15 amino acids, more preferably having 5 to 12 amino acids, even more preferably having 6 to 9 amino acids, which may be recognized by antibodies or B-cell receptors, i.e. in their native form. Such epitopes of proteins or peptides may furthermore be selected from any of the herein mentioned variants of such proteins or peptides. In this context antigenic determinants can be conformational or discontinous epitopes which are composed of segments of the proteins or peptides as defined herein that are discontinuous in the amino acid sequence of the proteins or peptides as defined herein but are brought together in the three-dimensional structure or continuous or linear epitopes which are composed of a single polypeptide chain.

"Variants" of proteins or peptides as defined herein may be encoded by the nucleic acid of the polymeric carrier cargo complex, wherein nucleotides of the nucleic acid, encoding the protein or peptide as defined herein, are exchanged. Thereby, a protein or peptide may be generated, having an amino acid sequence which differs from the original sequence in one or more mutation(s), such as one or more substituted, inserted and/or deleted amino acid(s). Preferably, these fragments and/or variants have the same biological function or specific activity compared to the full-length native protein, e.g. its specific antigenic property.

It is also within the scope of invention if nanoparticles (= polymeric carrier cargo complexes) of different origin (e.g. differing in the type of polymeric carrier and/or nucleic acid) are together embedded in a coating of biodegradable polymer.

According to the present invention, the nanoparticle comprising or consisting of a complex of a nucleic acid and a polymeric carrier molecule as defined herein is coated with a biodegradable polymer, wherein the biodegradable polymer is a PLGA polymer.

According to claimed and non-claimed embodiments disclosed herein, (e.g. biodegradable) polymers are used for coating of the nucleic acid comprising nanoparticles (= polymeric carrier cargo complexes). These (biodegradable) polymers can be selected from all (biodegradable) polymers known in the art for such purposes. Particularly preferred are those (biodegradable) polymers which are water-insoluble but which are soluble in organic solvents, in particular in organic solvents such as ethanol, acetone and/or THF. Particularly preferred in this context are polyesters (e.g. polylactic acid (PLA), polyglycolic acid (PGA)), co-polymers (e.g. poly(lactic-co-glycolic acid) (PLGA), with different ratios of lactic acid and glycolic acid), polyamides, lactame (e.g. caprolactam), polyether, etc. According to a non-claimed embodiment, the polymer need not necessarily be biodegradable. However, for the medical applications contemplated herein it is of advantage if the polymer is biodegradable.

In this context particularly preferred are PLGA polymers with an average molecular weight in the range of 4 kDa-210 kDa, more preferably in the range of 10 kDa to 110 kDa, even more preferably in the range of 20 kDa to 80 kDa. The proportion of Lactic acid in the PLGA polymer is preferably in the range of 25 to 100%, more preferably in the range of 25 to 85%.

The nanoparticles may also be coated with mixtures of two or more different (biodegradable) polymers. If thus herein reference is made to coating with "a" (biodegradable) polymer, this coating is not necessarily, but preferably, limited to only one type of (biodegradable) polymer.

It is also understood that preferably the coating with the (biodegradable) polymer is a direct coating of the nanoparticles, i.e. the nanoparticles are not separated from the (biodegradable) polymer via a further intermediate coating or coatings, but are in direct contact with the (biodegradable) polymer. As used herein, "coated with a (biodegradable) polymer" is intended to refer to situation wherein individual nanoparticles are coated by a layer of (biodegradable) polymer as well as to situations where a plurality of nanoparticles are embedded (e.g. evenly or unevenly distributed) in a (biodegradable) polymer composition.

According to a further embodiment, the present invention also provides a (pharmaceutical) composition comprising the inventive nucleic acid comprising nanoparticles coated with a biodegradable polymer as defined herein and optionally a pharmaceutically acceptable carrier and/or vehicle.

As a first ingredient, the inventive pharmaceutical composition comprises the inventive (nucleic acid comprising) nanoparticles coated with a biodegradable polymer as defined herein.

As a second ingredient the inventive pharmaceutical composition may comprise at least one additional pharmaceutically active component. A pharmaceutically active component in this connection is a compound that has a therapeutic effect to heal, ameliorate or prevent a particular indication, preferably cancer diseases, autoimmune disease, allergies or infectious diseases. Such compounds include, without implying any limitation, peptides or proteins, preferably as defined herein for coding nucleic acids, nucleic acids, preferably as defined herein, (therapeutically active) low molecular weight organic or inorganic compounds (molecular weight less than 5000, preferably less than 1000), sugars, antigens or antibodies, preferably as defined herein, therapeutic agents already known in the prior art, antigenic cells, antigenic cellular fragments, cellular fractions; cell wall components (e.g. polysaccharides), modified, attenuated or deactivated (e.g. chemically or by irradiation) pathogens (virus, bacteria etc.), adjuvants, preferably as defined herein, etc.

Furthermore, the inventive pharmaceutical composition may comprise a pharmaceutically acceptable carrier and/or vehicle. In the context of the present invention, a pharmaceutically acceptable carrier typically includes the liquid or non-liquid basis of the inventive pharmaceutical composition. If the inventive pharmaceutical composition is provided in liquid form, the carrier will typically be pyrogen-free water; isotonic saline or buffered (aqueous) solutions, e.g phosphate, citrate etc. buffered solutions. Particularly for injection of the inventive pharmaceutical composition, water or preferably a buffer, more preferably an aqueous buffer, may be used, containing a sodium salt, preferably at least 50 mM of a sodium salt, a calcium salt, preferably at least 0.01 mM of a calcium salt, and optionally a potassium salt, preferably at least 3 mM of a potassium salt. According to a preferred embodiment, the sodium, calcium and, optionally, potassium salts may occur in the form of their halogenides, e.g. chlorides, iodides, or bromides, in the form of their hydroxides, carbonates, hydrogen carbonates, or sulfates, etc. Without being limited thereto, examples of sodium salts include e.g. $NaCl$, $NaI$, $NaBr$, $Na_2CO_3$, $NaHCO_3$, $Na_2SO_4$, examples of the optional potassium salts include e.g. $KCl$, $KI$, $KBr$, $K_2CO_3$, $KHCO_3$, $K_2SO_4$, and examples of calcium salts include e.g. $CaCl_2$, $CaI_2$, $CaBr_2$, $CaCO_3$, $CaSO_4$, $Ca(OH)_2$. Furthermore, organic anions of the aforementioned cations may be contained in the buffer. According to a more preferred embodiment, the buffer suitable for injection purposes as defined herein, may contain salts selected from sodium chloride ($NaCl$), calcium chloride ($CaCl_2$) and optionally potassium chloride ($KCl$), wherein further anions may be present additional to the chlorides. $CaCl_2$ can also be replaced by another salt like $KCl$. Typically, the salts in the injection buffer are present in a concentration of at least 50 mM sodium chloride ($NaCl$), at least 3 mM potassium chloride ($KCl$) and at least 0,01 mM calcium chloride ($CaCl_2$). The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of cells due to osmosis or other concentration effects. Reference media are e.g. liquids occurring in *"in vivo"* methods, such as blood, lymph, cytosolic liquids, or other body liquids, or e.g. liquids, which may be used as reference media in *"in vitro"* methods, such as common buffers or liquids. Such common buffers or liquids are known to a skilled person. Ringer-Lactate solution is particularly preferred as a liquid basis.

According to another aspect, the inventive pharmaceutical composition may comprise an adjuvant. In this context, an adjuvant may be understood as any compound, which is suitable to initiate or increase an immune response of the innate immune system, i.e. a non-specific immune response. With other words, when administered, the inventive pharmaceutical composition typically elicits an innate immune response due to the adjuvant, optionally contained therein. Such an adjuvant may be selected from any adjuvant known to a skilled person and suitable for the present case, i.e. supporting the induction of an innate immune response in a mammal.

The inventive pharmaceutical composition may be administered orally, parenterally, by inhalation spray, topically, rectally, nasally, buccally, vaginally or via an implanted reservoir. The term parenteral as used herein includes subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, and sublingual injection or infusion techniques.

The inventive pharmaceutical composition may be used for human and also for veterinary medical purposes, preferably for human medical purposes, as a pharmaceutical composition in general or as a vaccine.

According to a particular preferred aspect, the inventive pharmaceutical composition may be provided or used as an immunostimulating agent. In this context, the inventive pharmaceutical composition is preferably as defined above. More preferably, the nucleic acid comprised in the polymeric carrier cargo complex, contained in the inventive pharmaceutical composition, is typically an immunostimulatory nucleic acid as defined herein, e.g. a CpG-DNA or an immunostimulatory RNA (isRNA). Alternatively or additionally, the nucleic acid of the polymeric carrier cargo complex, contained in the pharmaceutical composition, is a coding nucleic acid as defined herein, preferably a cDNA or an mRNA, more preferably encoding an adjuvant protein or an antigen preferably as defined herein.

In a specific aspect of this embodiment in this context it is preferred that an adjuvant protein or an antigen is a component of the polymeric carrier, preferably as $(AA)_x$, component.

According to an even more preferred aspect, the inventive pharmaceutical composition (or the inventive nucleic acid comprising nanoparticles coated with a biodegradable polymer for reversible immobilization and/or controlled drug release) may be provided or used as an adjuvant. In this context, the adjuvant is preferably defined as the inventive pharmaceutical composition above. More preferably, the nucleic acid of the polymeric carrier cargo complex, preferably contained in the adjuvant, is typically an immunostimulatory nucleic acid as defined herein, e.g. a CpG-DNA or an immunostimulatory RNA (isRNA). Alternatively or additionally, the nucleic acid of the polymeric carrier cargo complex, preferably contained in the adjuvant, is a coding nucleic acid as defined herein, preferably a cDNA or an mRNA, more preferably encoding an adjuvant protein or an antigen, preferably as defined herein. The inventive nucleic acid comprising nanoparticles coated with a (biodegradable) polymer for reversible immobilization and/or controlled drug release, preferably contained in the adjuvant, typically initiates an innate immune response in the patient to be treated. Such an adjuvant may be utilized in any accompanying therapy, with any known vaccine or any further (known) therapeutic agent, preferably prior to, concurrent with or subsequent to administration of the main therapy, prior to, concurrent with or subsequent to administration of a further (known) vaccine or a (known) further therapeutic agent.

The inventive nucleic acid comprising nanoparticles coated with a biodegradable polymer for reversible immobilization and/or controlled drug release or the inventive pharmaceutical composition as defined herein provided or used as an adjuvant or immunostimulating agent is preferably capable of triggering a non-antigen-specific, (innate) immune reaction (as provided by the innate immune system), preferably in an immunostimulating manner. An immune reaction can generally be brought about in various ways. An important factor for a suitable immune response is the stimulation of different T-cell sub-populations. T-lymphocytes typically differentiate into two sub-populations, the T-helper 1 (Th1) cells and the T-helper 2 (Th2) cells, with which the immune system is capable of destroying intracellular (Th1) and extracellular (Th2) pathogens (e.g. antigens). The two Th cell populations differ in the pattern of effector proteins (cytokines) produced by them. Thus, Th1 cells assist the cellular immune response by activation of macrophages and cytotoxic T-cells. Th2 cells, on the other hand, promote the humoral immune response by stimulation of B-cells for conversion into plasma cells and by formation of antibodies (e.g. against antigens). The Th1/Th2 ratio is therefore of great importance in the immune response. In connection with the present invention, the Th1/Th2 ratio of the immune response is preferably displaced by the immune-stimulating agent, namely the inventive nucleic acid comprising nanoparticles coated with a biodegradable polymer for reversible immobilization and/or controlled drug release in the direction towards the cellular response, that is to say the Th1 response, and a predominantly cellular immune response is thereby induced. As defined above, the inventive nucleic acid comprising nanoparticles coated with a biodegradable polymer for reversible immobilization and/or controlled drug release exerts by itself an unspecific innate immune response, which allows the inventive nucleic acid comprising nanoparticles coated with a biodegradable polymer for reversible immobilization and/or controlled drug release be used as such (without adding another pharmaceutically active component) as an immunostimulating agent. If administered together with another pharmaceutically active component, preferably a specifically immunogenic component, preferably an antigen, the nucleic acid comprised in the inventive nucleic acid comprising nanoparticles coated with a biodegradable polymer for reversible immobilization and/or controlled drug release serves as an adjuvant supporting the specific adaptive immune response elicited by the other pharmaceutically active component e.g. an antigen.

According to another particularly preferred embodiment, the inventive pharmaceutical composition (or the inventive nucleic acid comprising nanoparticles coated with a biodegradable polymer for reversible immobilization and/or controlled drug release) may be provided or used as a vaccine.

Such an inventive vaccine is typically composed like the inventive pharmaceutical composition and preferably supports or elicits an immune response of the immune system of a patient to be treated, e.g. an innate immune response, if an RNA or mRNA is used as the nucleic acid molecule of the polymeric carrier cargo complex formed by the nucleic acid cargo and a polymeric carrier molecule according to generic formula (I) or (Ia) or according to any of subformulas thereof as defined herein. Furthermore or alternatively, the inventive vaccine may elicit an adaptive immune response, preferably, if the nucleic acid of the polymeric carrier cargo complex formed by the nucleic acid cargo and a polymeric carrier molecule according to generic formula (I) or (Ia) or according to any of subformulas thereof as defined herein encodes any of the above mentioned antigens or proteins, which elicit an adaptive immune response.

In this context, the vaccine is preferably defined as an adjuvant or as an inventive pharmaceutical composition as disclosed above. More preferably, the nucleic acid of the polymeric carrier cargo complex, contained in such a vaccine, may be any nucleic acid as defined above, preferably an immunostimulatory nucleic acid as defined herein, e.g. a CpG-DNA or an immunostimulatory RNA (isRNA). Alternatively or additionally, the nucleic acid of the polymeric carrier cargo complex, preferably contained in the vaccine, is a coding nucleic acid as defined herein, preferably a cDNA or an mRNA, more preferably encoding an adjuvant protein, preferably as defined herein. Alternatively or additionally, the nucleic acid of the polymeric carrier cargo complex, preferably contained in the vaccine, is a coding nucleic acid as defined herein, preferably a cDNA or an mRNA, more preferably encoding an antigen, preferably as defined herein. Furthermore, particularly, if the nucleic acid of the polymeric carrier cargo complex does not encode an antigen, the inventive vaccine may contain an antigen, preferably as defined above, either as a protein or peptide or encoded by a nucleic acid, or antigenic cells, antigenic cellular fragments, cellular fractions; cell wall components (e.g. polysaccharides), modified, attenuated or de-activated (e.g. chemically or by irradiation) pathogens (virus, bacteria etc.).

According to a further aspect the inventive vaccine may contain a peptide or protein antigen as $(AA)_x$ component of the polymeric carrier as defined herein, preferably as part of the repetitive component $[S-P^2-S]_n$.

The inventive vaccine may also comprise a pharmaceutically acceptable carrier, adjuvant, and/or vehicle as defined herein for the inventive pharmaceutical composition.

The inventive vaccine can additionally contain one or more auxiliary substances in order to increase its immunogenicity, if desired. A synergistic action of the inventive nucleic acid comprising nanoparticles coated with a biodegradable polymer for reversible immobilization and/or controlled drug release formed by the polymer coating, the nucleic acid cargo and the polymeric carrier molecule according to generic formula (I) or (Ia) or according to any of subformulas thereof as defined herein and of an auxiliary substance, which may be optionally contained in the inventive vaccine as defined herein, is preferably achieved thereby. Depending on the various types of auxiliary substances, various mechanisms can come into consideration in this respect. For example, compounds that permit the maturation of dendritic cells (DCs), for example lipopolysaccharides, TNF-alpha or CD40 ligand, form a first class of suitable auxiliary substances. In general, it is possible to use as auxiliary substance any agent that influences the immune system in the manner of a "danger signal" (LPS, GP96, etc.) or cytokines, such as GM-CFS, which allow an immune response to be enhanced and/or influenced in a targeted manner. Particularly preferred auxiliary substances are cytokines, such as monokines, lymphokines, interleukins or chemokines, that further promote the innate immune response, such as IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta or TNF-alpha, growth factors, such as hGH.

Further additives which may be included in the inventive vaccine are emulsifiers, such as, for example, Tween®; wetting agents, such as, for example, sodium lauryl sulfate; colouring agents; taste-imparting agents, pharmaceutical carriers; tablet-forming agents; stabilizers; antioxidants; preservatives.

The inventive vaccine can also additionally or alternatively contain any further compound, which is known to be immune-stimulating due to its binding affinity (as ligands) to human Toll-like receptors TLR1, TLR2, TLR3, TLR4, TLR5, TLR6, TLR7, TLR8, TLR9, TLR10, or due to its binding affinity (as ligands) to murine Toll-like receptors TLR1, TLR2, TLR3, TLR4, TLR5, TLR6, TLR7, TLR8, TLR9, TLR10, TLR11, TLR12 or TLR13.

Another class of compounds, which may be added to an inventive vaccine in this context, may be CpG nucleic acids, in particular CpG-RNA or CpG-DNA. A CpG-RNA or CpG-DNA can be a single-stranded CpG-DNA (ss CpG-DNA), a double-stranded CpG-DNA (dsDNA), a single-stranded CpG-RNA (ss CpG-RNA) or a double-stranded CpG-RNA (ds CpG-RNA). The CpG nucleic acid is preferably in the form of CpG-RNA, more preferably in the form of single-stranded CpG-RNA (ss CpG-RNA). The CpG nucleic acid preferably contains at least one or more (mitogenic) cytosine/guanine dinucleotide sequence(s) (CpG motif(s)). According to a first preferred alternative, at least one CpG motif contained in these sequences, that is to say the C (cytosine) and the G (guanine) of the CpG motif, is unmethylated. All further cytosines or guanines optionally contained in these sequences can be either methylated or unmethylated. According to a further preferred alternative, however, the C (cytosine) and the G (guanine) of the CpG motif can also be present in methylated form.

The inventive vaccine can also additionally or alternatively contain an immunostimulatory RNA, i.e. an RNA derived from an immunostimulatory RNA, which triggers or increases an (innate) immune response. Preferably, such an RNA may be in general as defined herein for RNAs. In this context, those classes of RNA molecules, which can induce an innate immune response, may be selected e.g. from ligands of Toll-like receptors (TLRs), particularly from RNA sequences representing and/or encoding ligands of TLRs, preferably selected from human family members TLR1 - TLR10 or murine family members TLR1 - TLR13, more preferably from TLR7 and TLR8, ligands for intracellular receptors for RNA (such as RIG-I or MDA-5, etc.) (see e.g. Meylan, E., Tschopp, J. (2006). Toll-like receptors and RNA helicases: two parallel ways to trigger antiviral responses. Mol. Cell 22, 561-569), or any other immunostimulatory RNA sequence. Such an immunostimulatory RNA may comprise a length of 1000 to 5000, of 500 to 5000, of 5 to 5000, or of 5 to 1000, 5 to 500, 5 to 250, of 5 to 100, of 5 to 50 or of 5 to 30 nucleotides.

The present invention further provides as an embodiment medical or diagnostic devices exhibiting a coating, the coating comprising said nanoparticles embedded in a biodegradable polymer.

In this context medical devices are defined as all devices or implants used for medical purposes in or on a patient. In general a medical device is a product which is used for medical purposes in patients, in diagnosis, therapy or surgery. Such medical devices or implants can be chosen from: artificial joints (hip, knee etc.), artificial heart valves, artificial anus, devices for fixing broken bones, etc, but particularly preferred in this context are coronary stents and all medical devices for (intra)vascular applications like balloon catheters, vascular prostheses, coils, etc.

Additionally preferred are all kind of patches, heart or vascular patches.

Diagnostic devices include all devices including research tools which may be used in the context of transfection of cells with nucleic acids. Particularly preferred in this context is plastic ware used for cell culture, e.g. cell culture plates, or glass ware, e.g. glass slides.

Also disclosed herein is the use of a (biodegradable) polymer for coating a polymeric carrier cargo complex (i.e. nanoparticles) as defined herein for reversible immobilization and/or controlled release.

Further disclosed herein is the use of a (biodegradable) polymer for coating a polymeric carrier cargo complex (i.e. nanoparticles) as defined herein on medical or diagnostic devices for reversible immobilization and/or controlled release.

The present disclosure further comprises a method of preparing the inventive nucleic acid comprising nanoparticles coated with a (biodegradable) polymer, e.g. for reversible immobilization and/or drug release, and a method for providing medical or diagnostic devices or implants with a coating comprising a (biodegradable) polymer with the nucleic acid comprising nanoparticles embedded therein. Preferably, the method is carried out for reversible immobilization and/or drug release of said nanoparticle(s) on said medical or diagnostic devices or implants. The disclosure also comprises the product obtained or obtainable by such methods (product by process).

The inventive method of preparing the inventive, coated nanoparticles preferably comprises the following steps:

a) providing a nanoparticle comprising or consisting of a complex of a nucleic acid, which is DNA or RNA a polymeric carrier molecule according to generic formula (I):

$$L-P^1-S-[S-P^2-S]_n-S-P^3-L \qquad \text{(formula I)}$$

wherein

$P^1$ and $P^3$ are different or identical to each other and represent a linear or branched hydrophilic polymer chain, each $P^1$ and $P^3$ exhibiting at least one -SH-moiety, capable to form a disulfide linkage upon condensation with component $P^2$, the linear or branched hydrophilic polymer chain selected independent from each other from polyethylene glycol (PEG), poly-*N*-(2-hydroxypropyl)methacrylamide, poly-2-(methacryloyloxy)ethyl phosphorylcholines, poly(hydroxyalkyl L-asparagine), poly(2-(methacryloyloxy)ethyl phosphorylcholine), hydroxyethylstarch or poly(hydroxyalkyl L-glutamine), wherein the hydrophilic polymer chain exhibits a molecular weight of about 1 kDa to about 100 kDa,

$P^2$ is a cationic or polycationic peptide or protein, having a length of 3 to 100 amino acids, or is a cationic or polycationic polymer, having a molecular weight of 0.5 kDa to 30 kDa, each $P^2$ exhibiting at least two -SH-moieties, capable to form a disulfide linkage upon condensation with further components $P^2$ or component(s) $P^1$ and/or $P^3$;

-S-S- is a (reversible) disulfide bond;

L is an optional ligand, which may be present or not, and may be selected independent from the other from RGD, Transferrin, Folate, a signal peptide or signal sequence, a localization signal or sequence, a nuclear localization signal or sequence (NLS), an antibody, a cell penetrating peptide, TAT, a ligand of a receptor, cytokines, hormones, growth factors, small molecules, carbohydrates, mannose, galactose, synthetic ligands, small molecule agonists, inhibitors or antagonists of receptors, or RGD peptidomimetic analogues; and

n is an integer, selected from a range of 1 to 50, preferably in a range of 1, 2, 3, 4, or 5 to 10, more preferably in a range of 1, 2, 3, or 4 to 9; and

b) contacting, preferably by mixing, the nanoparticle of a) with a biodegradable PLGA polymer in an organic solvent containing solution, and

c) optionally removing, e.g. by drying, the organic solvent and where appropriate any other solvent in the organic solvent containing solution.

There are several constellations conceivable to carry out said method. The nanoparticles may be provided without solvent (e.g. lyophilized) or in solution, e.g. in water or in an organic solvent containing solution. The nanoparticles may then be contacted with the (biodegradable) polymer. The polymer may for this purpose already be dissolved in an organic solvent containing solution (preferably 100% organic solvent) and either the dry nanoparticles are then dissolved in said solution or the nanoparticle containing solution is mixed with said (biodegradable) polymer solution. In the alternative, the nanoparticles may for example be present in an organic solvent containing solution (preferably 100% organic solvent) and the (biodegradable) polymer is then dissolved in said organic solvent containing solution (provided the organic solvent content is sufficiently high to do so). Likewise, nanoparticles as well as (biodegradable) polymer may be present in dry form, are then joined together and subsequently the organic solvent containing solution is added to solve the nanoparticles and (biodegradable) polymer in said solution.

In more detail, the method of preparing the inventive, coated nanoparticles may comprise preferably the following steps:

1. Preparing of the polymeric carrier according to formula (I) (or any of its subformula):

a) providing at least one cationic or polycationic protein or peptide as component $P^2$ as defined herein and/or at least one cationic or polycationic polymer as component $P^2$ as defined herein, and optionally at least one further component (e.g. $(AA)_x$, $[(AA_x)]_z$, etc.)., preferably in the ratios indicated above by formula (I), mixing these components, preferably in a basic milieu as defined herein, preferably in the presence of oxygen or a further starter as defined herein which leads to mild oxidation conditions, preferably at a pH, at a temperature and at time as defined herein, and thereby condensing and thus polymerizing these components with each other via disulfide bonds (in a polymerization condensation or polycondensation) to obtain a repetitive component $H\text{-}[S\text{-}P^2\text{-}S]_n\text{-}H$ or $H\{[S\text{-}P^2\text{-}S]_a[S\text{-}(AA)_x\text{-}S]_b\}H$, etc.;

b) providing a hydrophilic polymer $P^1$ and/or $P^3$ as defined herein, optionally modified with a ligand L and/or an amino acid component $(AA)_x$ as defined herein;

c) mixing the hydrophilic polymer $P^1$ and/or $P^3$ provided according to step b) with the repetitive component $H\text{-}[S\text{-}P^2\text{-}S]_n\text{-}H$ or $H\{[S\text{-}P^2\text{-}S]_a[S\text{-}(AA)_x\text{-}S]_b\}H$, etc. obtained according to step a), typically in a ratio of about 2 : 1, (and thereby typically terminating the polymerization condensation or polycondensation reaction) and obtaining the polymeric carrier, preferably according to formula (I) as defined herein or according to any subformula thereof as defined herein;

d) optionally purifying the polymeric carrier obtained according to step c), preferably using a method as defined herein;

2. Complexation of the polymeric carrier with the nucleic acid cargo:

a) adding a nucleic acid as defined herein to the polymeric carrier obtained according to step 1 c) or 1 d), preferably in the above mentioned ratios, and complexing the nucleic acid with the polymeric carrier obtained according to step 1 c) or 1 d) to obtain a polymeric carrier cargo complex as defined herein.

3. Optional lyophilization of the polymeric carrier cargo complex:

a) Optionally the polymeric carrier cargo complex as obtained from step 2 can be lyophilized. This allows the reconstitution of the lyophilized nucleic acid comprising nanoparticles in an organic solvent containing solution with a high percentage of organic solvent (preferably 100% of an organic solvent).

b) Prior to the coating with the biodegradable polymer, which is a PLGA polymer, the polymeric carrier cargo complexes are reconstituted in a solvent, preferably in an organic solvent containing solution as defined herein.

4. Mixing of the resulting solution from step 2 or 3 with the biodegradable polymer dissolved in an organic solvent containing solution as defined herein.

5. Optionally removing, e.g. by drying, the organic solvent (and/or where appropriate any other solvent in the resulting solution of step 4).

If the polymeric carrier cargo complexes are lyophilized, the lyophilized polymeric carrier cargo complexes can be directly reconstituted in the organic solvent containing solution comprising the biodegradable polymer. Prior to mixing with the biodegradable polymer dissolved in an organic solvent containing solution, the resulting solution from step 2 or 3 comprising the polymeric carrier cargo complexes can be mixed with/dissolved in an organic solvent containing solution.

The disclosed method of providing medical or diagnostic devices or implants with a coating comprising a (biodegradable) polymer with the (nucleic acid comprising) nanoparticles embedded therein, e.g. for reversible immobilization and/or drug release of said nanoparticle(s) on said medical or diagnostic devices or implants, preferably comprises following steps:

a) providing an organic solvent containing solution comprising dissolved in said solution i) nanoparticles comprising or consisting of a complex of a nucleic acid and a polymeric carrier molecule according to generic formula (I) as defined herein (polymeric carrier cargo complex), and ii) a (biodegradable) polymer,
b) applying the organic solvent containing solution on (e.g. a surface of) the medical or diagnostic device or an implant as defined herein; and
c) optionally removing, e.g. by drying, the organic solvent (and/or where appropriate any other solvent).

In particular, said method may comprise the following steps:

1. Preparing of the polymeric carrier according to formula (I) (or any of its subformula): as defined above for the method of preparing the inventive nucleic acid comprising nanoparticles coated with a (biodegradable) polymer for reversible immobilization and/or drug release

2. Complexation of the polymeric carrier with the nucleic acid cargo: as defined above for the method of preparing the inventive nucleic acid comprising nanoparticles coated with a (biodegradable) polymer for reversible immobilization and/or drug release

3. Optional lyophilization of the polymeric carrier cargo complex: as defined above for the method of preparing the inventive nucleic acid comprising nanoparticles coated with a (biodegradable) polymer for reversible immobilization and/or drug release

4. Mixing of the resulting solution from step 2 or 3 with the (biodegradable) polymer dissolved in an organic solvent containing solution as defined herein. If the polymeric carrier cargo complexes were lyophilized, the lyophilized polymeric carrier cargo complexes can be directly reconstituted in the organic solvent containing solution comprising the (biodegradable) polymer.

5. Application of the resulting solution of step 4 on a medical or diagnostic device or an implant as defined herein, particularly by dip coating, spray drying, flow coating or spin coating.

6. Optionally removing, e.g. by drying, the organic solvent (and/or where appropriate any other solvent).

The method of preparing the polymeric carrier according to formula (I) as defined herein in step 1 represents a multi-step condensation polymerization or polycondensation reaction via - SH moieties of the educts, e.g. component(s) $P^2$ as defined herein, further components $P^1$ and/or $P^3$ and optionally further components $(AA)_x$. The condensation polymerization or polycondensation reaction preferably leads to the polymeric carrier as a condensation polymer, wherein the single components are linked by disulfide bonds. This condensation polymerization leads to the polymeric carrier according to formula (I) preparing in a first step 1 a) of the condensation reaction the repetitive component $H-[S-P^2-S]_n-H$ or a variant thereof as a sort of a "core" or "central motif" of the polymeric carrier. In a second step 1 b) components $P^1$ and/or $P^3$ are provided, which allow to terminate or to somehow "coat" the repetitive component $H-[S-P^2-S]_n-H$ or a variant thereof in a third step c) by adding components $P^1$ and/or $P^3$ as defined herein (optionally modified with a ligand L and/or an amino acid component $(AA)_x$ as defined herein) to the condensation product obtained according to step 1 a). In subsequent step d), this product may be purified and further used to complex a nucleic acid cargo as defined herein to obtain a complex.

It is important to understand that the method is based on an equibrility reaction under mild oxidation conditions in steps 1 a), 1 (b)) and 1 c), which, upon balancing the equilibirity state, allows to obtain the polymeric carrier according to formula (I) above or according to any of its subformulas comprising the selected components in the desired molar ratios. For this purpose, long reaction times are envisaged to achieve an equibrility state in steps 1 a), 1 (b)) and 1 c). If for example a condensation polymerization is to be carried out using a molar ratio of 5 components $P^2$ in step a), the equilibrium is surprisingly settled at a polymer length of about 5 after sufficient time, preferably e.g. > 12 hours. However, due to the equilibrium the polymer length (as defined by n) is not fixed at a specific value, e.g. 5, but may vary accordingly within the equibrility reaction. Accordingly, about 5 may mean about 4 to 6, or even about 3 to 7. Preferably, the polymer length and thus the integer n (and thus a, b and a + b) varies within a limit of about $\pm 1$, or $\pm 2$.

As defined herein in step 1 a) of the method of preparing the polymeric carrier according to formula (I) at least one cationic or polycationic protein or peptide as component $P^2$ as defined herein and/or at least one cationic or polycationic polymer as component $P^2$ as defined herein are provided, preferably in the ratios indicated above by formula (I). These components are mixed, preferably in a basic milieu as defined herein, preferably in the presence of oxygen or a further starter as defined herein which leads to mild oxidation conditions, preferably at a pH, and at a temperature and at a time as defined herein, and thereby condensing and thus polymerizing these components with each other via disulfide bonds (in a polymerization condensation or polycondensation) to obtain a repetitive component $H\text{-}[S\text{-}P^2\text{-}S]_n\text{-}H$.

In all cases, step 1 a) is based on an equibrility reaction under mild oxidation conditions which, upon balancing the equilibirity state, allows to obtain either inventive repetitive component $H\text{-}[S\text{-}P^2\text{-}S]_n\text{-}H$ or inventive repetitive component $H\text{-}\{[S\text{-}P^2\text{-}S]_a[S\text{-}(AA)_x\text{-}S]_b\}\text{-}H$ in the desired molar ratios. In the equibrity state, n is preferably 1, 2, 3, 4, or 5 to 10, more preferably 4 to 9, and a + b = n is as defined above, preferably a + b = 1, 2, 3, 4, or 5 to 10, more preferably 4 to 9. For this purpose, long reaction times are envisaged to achieve an equibrility state in step a), most preferably e.g. > 12 hours. Accordingly, step a) of the method of preparing a polymeric carrier typically requires at least about 5 hours, even more preferably at least about 7.5 hours or even 10 hours, most preferably at least about 12 hours, e.g. a reaction time of about 12 to 60 hours, a reaction time of about 12 to 48 hours, a reaction time of about 12 to 36 hours, or a reaction time of about 12 to 24 hours, etc, wherein the lower border of 12 hours of the latter ranges may also be adjusted to 10, 7.5, or even 5 hours.

In step 1 a), the at least one cationic or polycationic protein or peptide as component $P^2$ as defined herein and/or at least one cationic or polycationic polymer as component $P^2$ as defined herein, and optionally at least one amino acid component $(AA)_x$ as defined herein, are preferably contained in a basic milieu in the step a) of the method of preparing the polymeric carrier according to formula (I) (or any of its subformulas, e.g. (Ia)). Such a basic milieu typically exhibits a pH range of about 6 to about 12, preferably a pH range of about 7 to about 10, more preferably a pH range of about 8 to about 10, e.g. about 8, 8.5, 9, 9.5, or 10 or any range selected from any two of these or the aforementioned values.

Furthermore, the temperature of the solution in step 1 a) is preferably in a range of about 5°C to about 60°C, more preferably in a range of about 15°C to about 40°C, even more preferably in a range of about 20°C to about 30°C, and most preferably in a range of about 20°C to about 25°C, e.g. about 25°C.

In step 1 a) of the method of preparing the polymeric carrier according to formula (I) (or any of its subformulas, e.g. (Ia)) as defined herein (hydrophilic) buffers may be used as suitable. Preferred buffers may comprise, but are not limited to carbonate buffers, borate buffers, Bicine buffer, CHES buffer, CAPS buffer, Ethanolamine containing buffers, HEPES, MOPS buffer, Phosphate buffer, PIPES buffer, Tris buffer, Tricine buffer, TAPS buffer, and/or TES buffer as buffering agents. Particularly preferred is a carbonate buffer.

Upon mixing the components in step 1 a), preferably in the presence of oxygen, preferably in the presence of a basic milieu as defined herein, the condensation polymerization or polycondensation reaction is started. For this purpose, the mixture in step 1 a) is preferably exposed to oxygen or may be started using a further starter, e.g. a catalytical amount of an oxidizing agent, e.g. DMSO, etc. To determine the desired polymer chain length the condensation reaction has to be carried out under mild oxidation conditions, preferably in the presence of less than 30% DMSO, more preferably in the presence of less than 20% DMSO and most preferably in the presence of less than 10% DMSO. Upon start of the condensation polymerization or polycondensation reaction the at least one cationic or polycationic protein or peptide and/or at least one cationic or polycationic polymer as component $P^2$ and optionally at least one amino acid component $(AA)_x$ as defined herein, are condensed and thus polymerized with each other via disulfide bonds (polymerization condensation or polycondensation). In this reaction step 1 a) preferably linear polymers are created using monomers with at least two reactive -SH moieties, i.e. at least one cationic or polycationic protein or peptide and/or at least one cationic or polycationic polymer as component $P^2$ as defined herein, each component $P^2$ exhibiting at least two free -SH-moieties as defined herein, e.g. at their terminal ends. However, components $P^2$ with more than two free -SH-moieties may be used, which may lead to branched polymers.

According to a second step 1 b) of the method of preparing the polymeric carrier according to formula (I) as defined herein (or according to any of its subformulas), a hydrophilic polymer $P^1$ and/or $P^3$ as defined herein is added to the condensation product obtained according to step a). In this context, the hydrophilic polymers $P^1$ and/or $P^3$ as defined herein, preferably exhibit at least one -SH-moiety, more preferably only one -SH-moiety per hydrophilic polymers $P^1$ and/or $P^3$ as defined herein, thereby terminally stopping the polymerization condensation or polycondensation according to step a) in step c). Hydrophilic polymers $P^1$ and/or $P^3$ as defined herein may be the same or different, wherein these polymers may be selected according to the desired properties. Typically, hydrophilic polymers $P^1$ and/or $P^3$ as a whole may be added to the condensation product obtained according to step a) in a ratio of about 2 : 1 hydrophilic polymer $P^1$ and/or $P^3$ : condensation product obtained according to step 1 a).

According to one alternative, the hydrophilic polymer(s) $P^1$ and/or $P^3$ additionally may be modified with either a component L (ligand) as defined herein or a component $(AA)_x$ or $[(AA)_x]_z$, as defined herein or both a component L (ligand) as defined herein and a component $(AA)_x$ or $[(AA)_x]_z$ as defined herein.

According to a further step 2 of the inventive methods, the nucleic acid as defined herein is added to the polymeric carrier according to formula (I) or to any of its subformulas as defined herein obtained according to step 1 c) or 1 d), preferably in the above mentioned ratios.

In general, the polymeric carrier cargo complexes are manufactured in a non-organic solvent (e.g. water, salt-containing buffers, or sugar containing solutions).

In a further step 3 of the inventive methods the polymeric carrier cargo complexes are optionally lyophilized to remove the solvent. Therefore the lyophilized polymeric carrier cargo complexes can be reconstituted directly in the organic solvent containing solution comprising the (biodegradable) polymer. Furthermore the lyophilization of the polymeric carrier cargo complexes can be used for storage of the polymeric carrier cargo complexes prior coating with the (biodegradable) polymer or application on a medical or diagnostic device by coating with the (biodegradable) polymer.

In a subsequent step 4, the polymeric carrier cargo complexes are dissolved in or mixed with a solvent, preferably an organic solvent containing solution which allows for mixing with (biodegradable) polymers only soluble in organic solvents. This resulting solution from step 4 (i.e. comprising the nanoparticles as well as the (biodegradable) polymer) is also termed as coating solution.

Due to the poor solubility and the instability of such (biodegradable) polymers in water or rather non-organic solvents and the poor or low solubility of highly hydrophilic nucleic acid in organic solvents, it was a technical demanding task to develop a polymeric carrier for the complexation of nucleic acids which allows solution in an organic solvent.

Additionally such a polymeric carrier must ensure the integrity of the nucleic acid cargo without inducing irreversible agglomeration or loss of biological activity.

The organic solvent which can be utilized to coat the nuceic acid comprising nanoparticles as such, or to coat the nucleic acid comprising nanoparticles on a surface of medical or diagnostic devices by different coating techniques (e.g. dip coating, spray drying, flow coating, or spin coating) can be chosen from all organic solvents known to a skilled person which are suitable for such purposes. Particularly preferred are solvents comprising alcohols (e.g. methanol, ethanol, i-propanol), ethers (e.g diethyl ether, methyl-t- butyl ether, tetrahydrofuran) and acetone.

Furthermore, the organic solvent must allow that the nanoparticles can be homogenously distributed in the dissolved biodegradable polymer at a high concentration.

An "organic solvent containing solution", as used herein; comprises an organic solvent. These comprised organic solvents can be chosen from all organic solvents known to a skilled person which are suitable for such purposes, in particular for dissolving the (biodegradable) polymer. Particularly preferred as organic solvents are alcohols (e.g. methanol, ethanol, i-propanol), ethers (e.g diethyl ether, methyl-t-butyl ether, tetrahydrofuran), esters (e.g. ethyl acetate, methyl acetate) and acetone. The organic solvent containing solution may also comprise a mixture of organic solvents. The organic solvent containing solution comprises preferably in the range of about 50 to about 100%, more preferably in the range of about 80 to about 100%, even more preferably in the range of about 90 to about 100% of organic solvent. A higher content of organic solvent is in particular preferred where dissolution of the (biodegradable) polymer is required. If the (biodegradable) polymer is already dissolved, the content of organic solvent may be lower, but should preferably not fall below the solubility limit for the (biodegradable) polymer.

In particular in the coating solution comprising the nanoparticles as well as the biodegradable polymer (e.g. resulting solution in step 4) the final concentration of the organic solvent content in the solution amounts preferably to a proportion in the range of 50% to 100%, more preferably to a proportion in the range of 70% to 95%, and even more preferably to a proportion in the range of 80% to 95%. If necessary or beneficial the non-organic solvent in which the polymeric carrier cargo complexes are dissolved resulting from step 2 or 3 can be replaced totally by the organic solvent. In such cases the non-organic solvent can be removed by lyophilization prior reconstitution in the organic solvent.

The coating solution resulting from step 4 of the method of coating of medical or diagnostic devices or implants by (biodegradable) polymers with the nucleic acid comprising nanoparticles for reversible immobilization and/or drug release may then be used for coating of a medical or diagnostic surface or device as defined herein by common processes known in the art (e.g. dip coating, spray drying, flow coating and spin coating).

During this step the polymeric carrier cargo complexes are reversibly immobilized in a matrix of the (biodegradable) polymer coated onto the surface of the medical or diagnostic device.

In this context, a skilled person would not have expected that it would be possible to generate a homogenous coating solution containing an organic solvent and low water content, the homogenous coating solution comprising stable (nucleic acid comprising) nanoparticles based on hydrophilic cationic protein/peptides or polymers on the one hand and hydrophobic (biodegradable) polymers on the other hand, without loss of integrity, activity or stability of the (nucleic acid comprising) nanoparticles. Such coating solutions can be used for the reversible immobilization and/or controlled drug release of such nucleic acid particles, particularly on medical or diagnostic devices as defined herein.

According to one embodiment, the present invention is directed to the first medical use of the inventive nucleic acid comprising nanoparticles coated with a biodegradable polymer for reversible immobilization and/or controlled drug release as defined herein, as a medicament, preferably for gene therapy or treatment of a disease as defined herein. The medicament may be in the form of a pharmaceutical composition or in the form of an adjuvant or a vaccine as a specific form of pharmaceutical compositions. A pharmaceutical composition in the context of the present invention typically comprises the inventive nucleic acid comprising nanoparticles coated with a (biodegradable) polymer for reversible immobilization and/or controlled drug release and optionally further ingredients as defined herein, and optionally a pharmaceutically acceptable carrier and/or vehicle, preferably as defined herein.

According to one further embodiment, the present invention is directed to the inventive nucleic acid comprising nanoparticles coated with a biodegradable polymer for reversible immobilization and/or controlled drug release or the inventive medical and diagnostic devices as defined herein, for use in the prophylaxis, treatment and/or amelioration of diseases as defined herein. Preferably, diseases as mentioned herein are selected from cancer or tumour diseases, infectious diseases, preferably (viral, bacterial or protozoological) infectious diseases, autoimmune diseases, allergies or allergic diseases, monogenetic diseases, i.e. (hereditary) diseases, or genetic diseases in general, diseases which have a genetic inherited background and which are typically caused by a defined gene defect and are inherited according to Mendel's laws, cardiovascular diseases, neuronal diseases, diseases of the respiratory system, diseases of the digestive system, diseases of the skin, musculoskeletal disorders, disorders of the connective tissue, neoplasms, immune deficiencies, endocrine, nutritional and metabolic diseases, eye diseases, ear diseases and any disease which can be influenced by the present invention. Particularly preferred in this context is the treatment of patients treated by medical devices, like coronary stents or implants to prevent particularly restenosis, calcification, foreign body reaction or inflammation.

Particularly preferred in this context is the prophylactic or therapeutic treatment of patients who are treated by medical devices or implants for the prevention of restenosis, calcification, foreign body reaction or inflammation.

According to one further embodiment, the present invention is directed to the inventive nucleic acid comprising nanoparticles coated with a (biodegradable) polymer for reversible immobilization and/or controlled drug release or the inventive medical or diagnostic devices for use in immunotherapy, gene therapy, vaccination, or for use as an adjuvant.

According to a further embodiment, the present invention is directed to the inventive nucleic acid comprising nanoparticles coated with a biodegradable polymer for reversible immobilization and/or controlled drug release, the inventive pharmaceutical composition or vaccine, or the inventive medical or diagnostic devices as defined herein for use in the treatment of diseases as defined herein, particularly prophylaxis, treatment and/or amelioration of various diseases as defined herein, which preferably comprises using or administering to a patient in need thereof the inventive nucleic acid comprising nanoparticles coated with a (biodegradable) polymer for reversible immobilization and/or controlled drug release, the inventive pharmaceutical composition or vaccine, or of the inventive medical or diagnostic devices as defined herein.

The present invention also allows treatment of diseases, which have not been inherited, or which may not be summarized under the above categories. Such diseases may include e.g. the treatment of patients, which are in need of a specific protein factor, e.g. a specific therapeutically active protein as mentioned above. This may e.g. include dialysis patients, e.g. patients which undergo a (regular) a kidney or renal dialysis, and which may be in need of specific therapeutically active proteins as defined herein, e.g. erythropoietin (EPO), etc.

According to a final embodiment, the present disclosure also comprises kits, particularly kits of parts, comprising as components alone or in combination with further ingredients the inventive nucleic acid comprising nanoparticles coated with a (biodegradable) polymer for reversible immobilization and/or controlled drug release, at least one pharmaceutical composition comprising same and/or kits comprising same, and optionally technical instructions with information on the administration and dosage of the inventive nucleic acid comprising nanoparticles coated with a (biodegradable) polymer for reversible immobilization and/or controlled drug release, and/or the inventive pharmaceutical composition. Such kits, preferably kits of parts, may be applied, e.g., for any of the above mentioned applications or uses. Such kits, when occurring as a kit of parts, may further contain each component in a different part of the kit. These kits or kits of parts may also be used as research tool or for diagnostic purposes.

## Figures

The following Figures are intended to illustrate the invention further. They are not intended to limit the subject matter of the invention thereto.

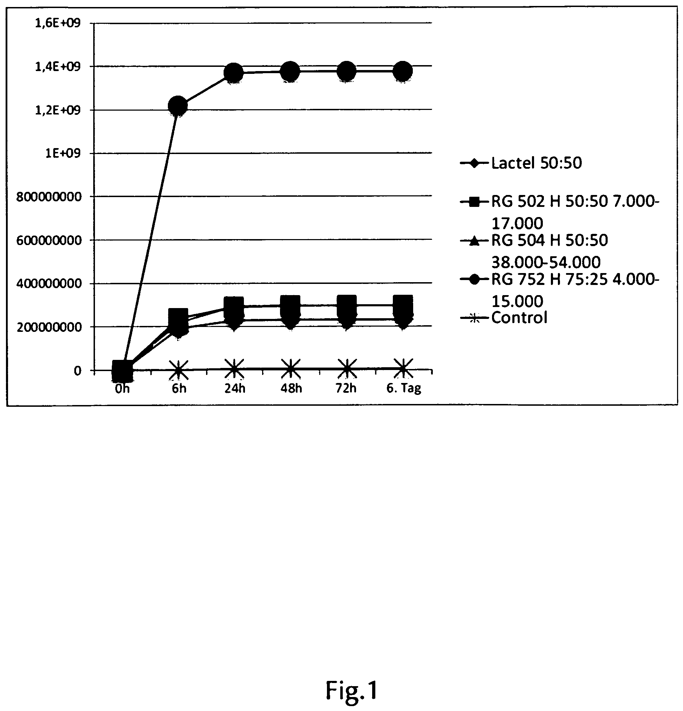

**Figure 1:** Expression of Luciferase in medium supernatant of endothelial cells. 100,000 EA.hy 926 cells were cultured on cover slips coated by PLGA with mRNA containing nanoparticles. Different PLGAs were used. The supernatant of the cells was used to measure Luciferase expression after 0, 6 hours, 24 hours, 48 hours and 72 hours and 6 days. After each measure point cells were washed excessively with PBS for several times to remove luciferase. The RLU of the luciferase assay was always added to the previous measurement.

The control was a cell cultured coverslips without coating.

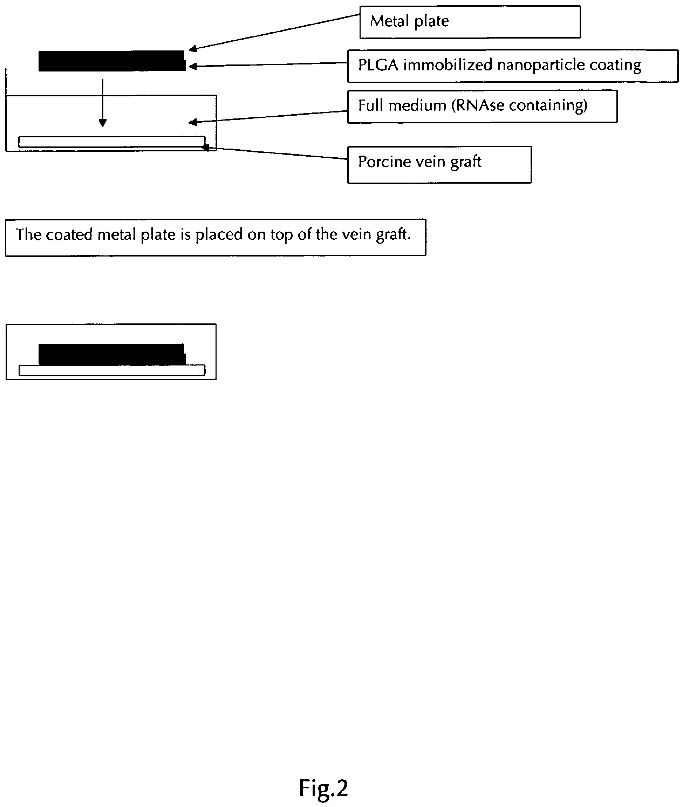

**Figure 2:** Schematic diagram of the application of metallic plates coated with mRNA containing nanoparticles by PLGA in porcine vein grafts. MRNA containing nanoparticles were coated by PLGA on phynox plates. Therefore mRNA comprising nanoparticles were together with PLGA pipetted in stainless steel plates (phynos plates). The solution was again dried over night. Freshly excised porcine vein of the jugularis were cut longitudinal. The veins were placed into one 6-well with 2 ml endothelial medium (Vasculife, endothelial growth medium). Afterwards the coated phynox plate was laid with the coated side on the endothel of the vein.

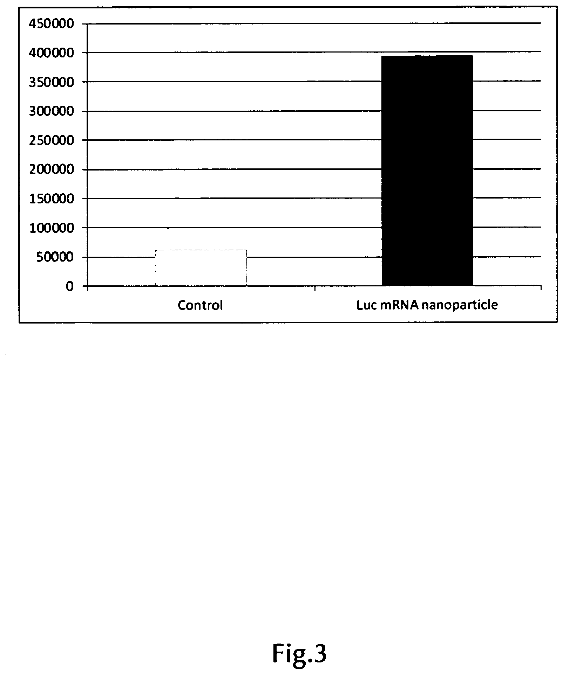

**Figure 3:** Expression of Luciferase in medium supernatant after 24 hours of transfection of porcine vein grafts with immobilized Luciferase mRNA containing nanoparticles (20 μg/plate) coated on phynox plates with the help of Lactel-PLGA (40 μg/plate). The procedure corresponds to figure 2. The supernatant of the medium was measured as described in Example 5.

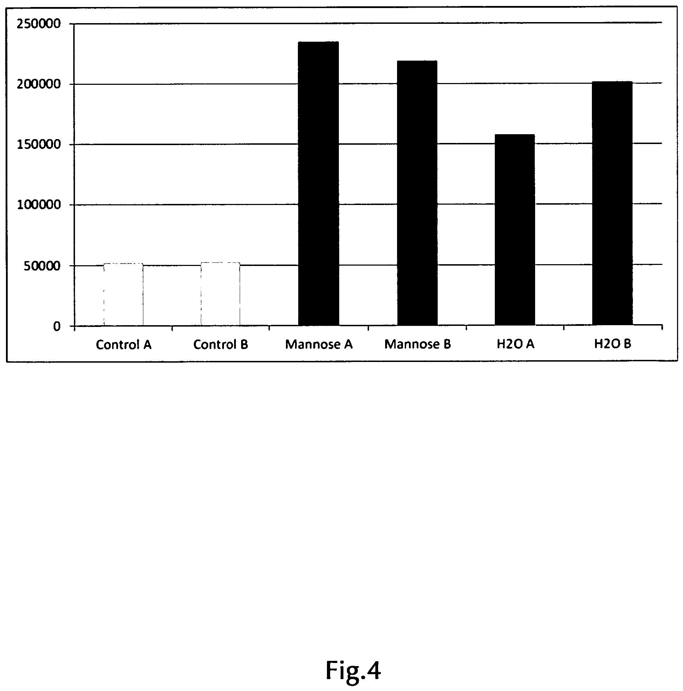

**Figure 4:** Expression of Luciferase in medium supernatant after 24 hours of transfection of porcine vein grafts with immobilized Luciferase mRNA containing nanoparticles (20 μg/plate) coated on phynox plates with the help of Lactel-PLGA (40 μg/plate). In this experiment the mRNA containing nanoparticles were prepared in different solutions; mannose containing solution or $H_2O$.

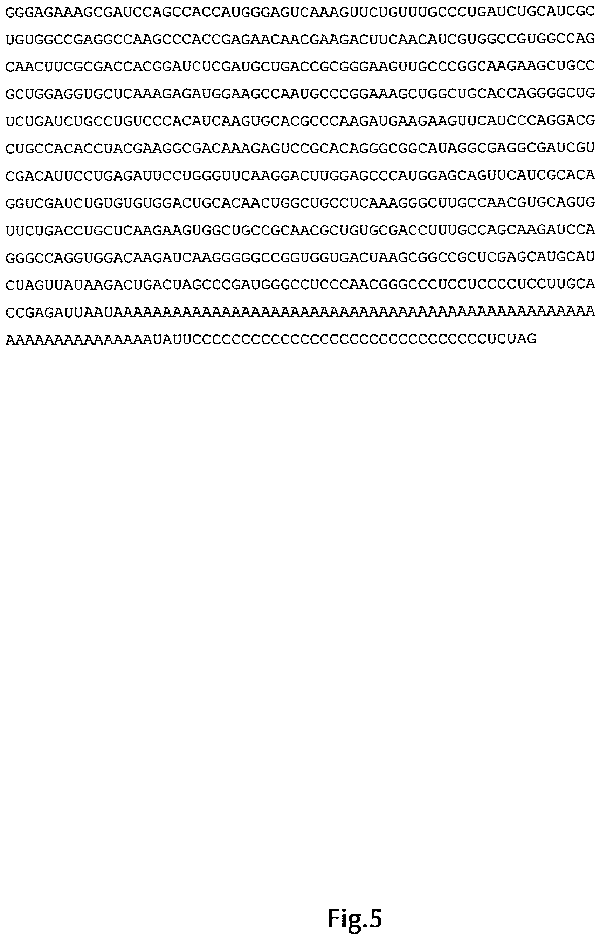

**Figure 5:** shows the mRNA sequence encoding *Gaussia* luciferase (SEQ ID NO: 128). The mRNA sequence contains following sequence elements:

•the coding sequence encoding *Gaussia* luciferase;
•stabilizing sequences derived from alpha-globin-3'-UTR (muag (mutated alpha-globin-3'-UTR));
•70 × adenosine at the 3'-terminal end (poly-A-tail);
•30 × cytosine at the 3'- terminal end (poly-C-tail).

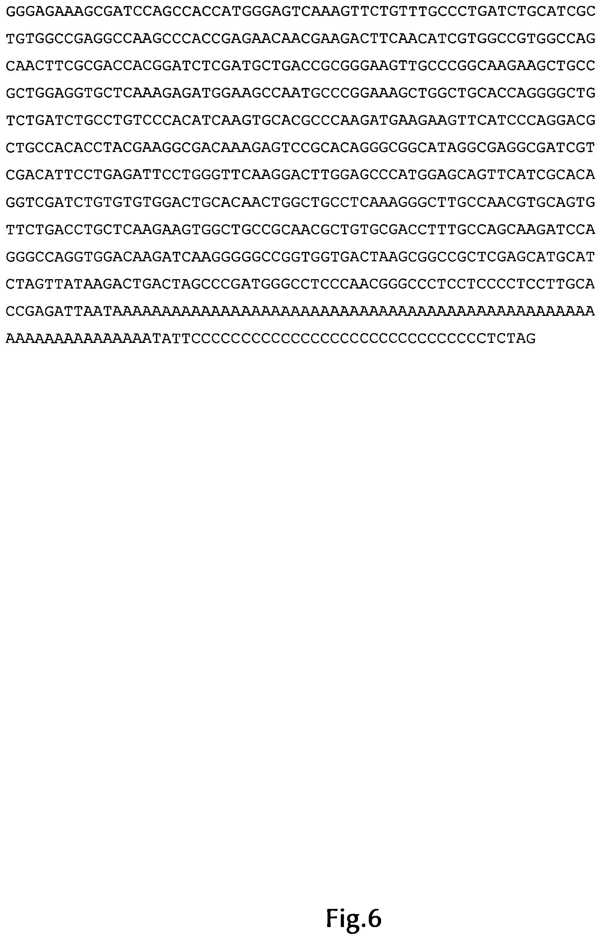

**Figure 6:** shows the corresponding DNA sequence encoding *Gaussia* luciferase (SEQ ID NO: 129).

**Examples**

The following examples are intended to illustrate the invention further. They are not intended to limit the subject matter of the invention thereto.

**1. Preparation of DNA and mRNA constructs encoding Gaussia luciferase *(Gaussia)***

For the present examples DNA sequences, encoding *Gaussia* luciferase, were prepared and used for subsequent *in vitro* transcription reactions.
According to a first preparation, the DNA sequence was prepared, which corresponds to the *Gaussia* luciferase coding sequence. Additionally sequences derived from alpha-globin-3'-UTR (muag (mutated alpha-globin-3'-UTR)), a histone stem-loop sequence, a stretch of 70 × adenosine at the 3'-terminal end (poly-A-tail) and a stretch of 30 × cytosine at the 3'- terminal end (poly-C-tail) were introduced 3' of the coding sequence.
The sequence contains following sequence elements:

- the coding sequence encoding *Gaussia* luciferase;
- stabilizing sequences derived from alpha-globin-3'-UTR (muag (mutated alpha-globin-3'-UTR));
- 70 × adenosine at the 3'-terminal end (poly-A-tail);
- 30 × cytosine at the 3'- terminal end (poly-C-tail).

**2. *In vitro* transcription:**

The respective DNA plasmid prepared according to Example 1 was transcribed *in vitro* using T7-Polymerase. Subsequently the mRNA was purified using PureMessenger® (CureVac, Tübingen, Germany).
In SEQ ID NO: 129 (see Figure 6) the sequence of the DNA corresponding to the mRNA is shown.

### 3. Reagents:

Peptides: The peptides used in the present experiments were as follows:
PB83: HO-PEG$_{5000}$-S-(S-CHHHHHHRRRRHHHHHC-S-)$_7$-S-PEG$_{5000}$-OH

### 4. Synthesis of the polymeric carrier:

The condensation reaction was performed with the calculated amount of peptide (component P$^2$) which is dissolved in a mixture of a buffered aqueous solution at pH 8,5 with an optional additive of 5% (v/v) Dimethylsulfoxide (DMSO) (which are mild oxidation conditions and therefore allow the establishment of an equilibrium) and stirred for 18h at ambient temperature. Afterwards the calculated amount of a thiol group containing PEG derivative (alpha-Methoxy-omega-mercapto poly(ethylene glycol)) (component P$^1$) (dissolved in water) is added and the resulting solution is stirred for another 18h. Subsequent lyophilisation and purification yield the desired polymer. The ratio between PEG component P$^1$ to peptide component P$^2$ defines the chain length of the P$^2$ polymer.

The condensation reaction in this reaction environment is reversible, therefore the chain length of the polymer is determined by the amount of the monothiol compound which terminates the polymerisation reaction. In summary the length of the polymer chain is determined by the ratio of oligo-peptide and monothiol component. This reaction is supported by the chosen mild oxidation conditions. With more stringent oxidation conditions (30% DMSO) the generation of high molecular (long chain) polymers is induced.

4.1. 1. Step: Exemplary polymerization reaction:

n HS-CHHHRRRRHHHC-SH → H-(S-CHHHRRRRHHHC-S)$_n$-H

4.2. 2. Step: Exemplary stop reaction:

H-(S-CHHHRRRRHHHC-S)$_n$-H + 2 PEG-SH → PEG-S-(S-CHHHRRRRHHHC-S)$_n$-S-PEG

4.3. Exemplary synthesis reaction:

step 1)

5 x HS-CHHHRRRRHHHC-SH → H-(S-CHHHRRRRHHHC-S)$_5$-H

step 2)

H-(S-CHHHRRRRHHHC-S)$_5$-H + 2x PEG$_{5000}$-SH
→ PEG$_{000}$-S-(S-CHHHRRRRHHHC-S)$_5$-S-PEG$_{5000}$

To achieve a polymer length of 5 (n = 5), a molar ratio of peptide:PEG of 5:2 was used.

Some variations of the synthesis reaction were done to show the the effect of the PEGchains and the effects of the reversible attachment of the PEG chains.

4.4. Synthesis reaction for polymeric carriers without PEG chains:

The reaction conditions are the same as mentioned above, but the step of the addition of a sulfhydryl containing PEG derivative is not performed/skipped.

4.5. Synthesis reaction for irreversible attached PEG chains:

The reaction conditions are the same as mentioned above, but instead of a sulfhydryl containing PEG derivative a maleimide containing PEG derivative is utilized. The maleimide mojety reacts rapidly with free sulfhydryl groups forming a covalent bond. Therefore the termination of the polymerization is not under the dynamic equilibria conditions as for sulfhyrdyl containing PEG derivatives but under irreversible conditions which results in a "frozen" polymerization pattern of high polydiversity and not the defined reaction products of the dynamic equilibria reaction.

### 5. Complexation of RNA:

The mRNA construct defined above in Example 1 and prepared according to Example 2, were complexed for the purposes of the present invention with the polymeric carrier, preferably as defined in Example 4. Therefore, 4 $\mu$g RNA coding for *Gaussia* luciferase according to SEQ ID NO: 128 were mixed in molar ratios as indicated with the polymeric carrier (according to formula I) thereby forming a complex. Afterwards the resulting solution was adjusted with water to a final volume of 50 $\mu$l und incubated for 30 minutes at room temperature.

Following ratio of polymeric carrier/RNA were used in the experiments:.

| Präfix | Polymer | Ratio | Cationi c AS | N/P |
|---|---|---|---|---|
| PB83 | HO- PEG$_{5000}$-S-(S-CHHHHHHHRRRRHHHHHHC-S-)$_7$-S-PEG$_{5000}$-OH | 250 | 28 | 2,8 |
| Ratio = molar ratio of RNA:peptide<br>cationic AS = cationic amino acids, which are positively charged at a physiological pH (i.e. not histidine (H) but e.g. arginine (R))<br>N/P = is the ratio of basic nitrogen atoms in the polymeric carrier to phosphate residues in the nucleic acid, considering that nitrogen atoms confer to positive charges and phosphate of the phosphate backbone of the nucleic acid confers to the negative charge. Histidine residues are counted neutral, because complex formation is done at physiological pH, therefore the imidazole residue is uncharged.<br>N/P is calculated by the following formula:<br><br>$$N/P = \frac{pmol\ [RNA]*\ ratio*cationic\ AS}{\mu g\ RNA*3*1000}$$<br><br>For the calculations mRNA coding for *Gaussia* luciferase according to SEQ ID NO: 128 was applied, which has a molecular weight of kDa. Therefore 1 $\mu$g mRNA according to SEQ ID NO: 128 confers to pmol mRNA according to SEQ ID NO: 128 | | | | |

### 6. Coating of the polymeric carrier cargo complexes with the biodegradable polymer PLGA:

Different PLGAs used in the experiments:

1. Lactel, PLGA, no indication of the molecular weight,, ratio 50:50, ester terminated
2. Sigma Aldrich, Resomer RG 752 H, catno. 719919, Poly(DL-lactide-co-glycolide) acid terminated, (72:25), MW 4000-15000
3. Sigma Aldrich, Resomer RG 502 H, catno. 719897, Poly(DL-lactide-co-glycolide) acid terminated, (50:50), MW 7000-17000
4. Sigma Aldrich, Resomer RG 504 H, catno. 719900, Poly(DL-lactide-co-glycolide) acid terminated, (50:50), MW 38000-54000

100 $\mu$l of the solution comprising the mRNA containing nanoparticles prepared according to example 5 (basal medium, 18 $\mu$g mRNA containing nanoparticles and 7 $\mu$l Interferin) were mixed with 100 $\mu$l of 0,05 % PLGA solution (solved in 100 % acetone).

### 7. Transfection of endothelial cells:

The solution as prepared according to example 6 was pipetted in duplets on coverslips (Thermanox Plastic Coverslips with 15 mm diameter) in one or in a second repeat to prevent solution from floating away from the cover slip. The solution on the coverslips was dried overnight at RT under sterile conditions and corresponds to 18 $\mu$g mRNA containing nanoparticles coated with 50 $\mu$g PLGA on one slide.

At the next day EA.hy 926 cells which are immortalized endothelial cells were cultured on the coverslips with 1 ml medium (DMEM high glucose, supplemented with L-Glutamine and antibiotics). Around 100,000 cells were cultured on one coverslip.

The measurement of secreted luciferase was measured 6h, 24 h, 48 h, 72h and 6 days after culturing. After each measurement cells were washed excessively with PBS to remove luciferase enzyme. For each measurement 20 $\mu$l of cell supernatant was pipetted into one well of a 96-well plate. The measurement buffer was consisting of PBS without Ca and Mg, supplemented with 5 mM NaCl and 0.04 mg Coelenterazien. With the Mithras LB 940 (Berthold) 100 $\mu$l of the measurement buffer was injected to the 20 $\mu$l supernatant. The luminescence detection (RLU) was performed for 10 sec by the Mithras apparatus. For calculating the luciferase expression the RLU of each measurement was added to the previous one.

### 8. <u>Expression of luciferase ex vivo:</u>

8.1. <u>Caoting of Phynoxplates (stainless steal plates):</u>

Nanoparticles containing mRNA coding for *Gaussia* Luciferase were formulated at a concentration of 0.1 g/l. 200 $\mu$l of this solution was mixed with an equal volume solution containing 40 $\mu$g PLGA in acetone. The coating solution was applied to the plates and after drying at RT over night the plates were ready for use in transfection studies.

8.2. <u>Transfection of vein grafts:</u>

Freshly prepared lumen tissue of Vena jugularis externa (pig) were incubated in 6 well plates in 2 ml medium. The coated metal plates were placed on top of the tissue (endothelium), therefore enabling direct contact of the PLGA matrix to the cells. After 24h the amount of expressed *Gaussia* luciferase was quantified in the supernatant (see Example 7).

### 11. <u>Results:</u>

### 11.1. **Expression of luciferase in EA.hy926 cells:**

The results show that all cells growing on coverslips with PLGA immobilized mRNA containing nanoparticles secrete luciferase, compared to control cells growing on slips without mRNA containing nanoparticles coding for *Gaussia* luciferase. Furthermore, cells grown on the PLGA resomer 752 with a ratio of 75:25 show the highest luciferase expression compared to the 50:50 resomers. The RLU of each measurement point was added to the previous one, resulting in a curve that shows a burst release between 0-24h, by the dissociation of the mRNA out of the PLGA coating.. Afterwards the expression of luciferase increases slow, which indicates the hydrolyzation of the PLGA.

### 11.5. **Expression of luciferase *ex vivo*:**

Expression of luciferase in porcine vein grafts was determined 24 hours after application of metallic plates coated by PLGA with polymeric carrier cargo complexes comprising 20 $\mu$g mRNA coding for *Gaussia* luciferase. The secreted luciferase was again measured in the surrounding endothelial cell medium. As can be seen in Fig. 3, PLGA coating of the metallic plates with the mRNA containing nanoparticles leads to a high luciferase expression in the porcine vein grafts.

Furthermore, expression of luciferase in porcine vein grafts 24 hours after application of metallic plates coated by PLGA with polymeric carrier cargo complexes is independent of the polymeric carrier medium which was containing mannose or H2O as shown in Figure 4.

### Claims

1. Nanoparticle comprising or consisting of a complex of a nucleic acid, which is a DNA or RNA, and a polymeric carrier molecule according to generic formula (I):

$$\text{L-P}^1\text{-S-[S-P}^2\text{-S]}_n\text{-S-P}^3\text{-L} \qquad \text{(formula I)}$$

   wherein,

   $P^1$ and $P^3$ are different or identical to each other and represent a linear or branched hydrophilic polymer chain, each $P^1$ and $P^3$ exhibiting at least one -SH-moiety, capable to form a disulfide linkage upon condensation with component $P^2$, the linear or branched hydrophilic polymer chain selected independent from each other from polyethylene glycol (PEG), poly-*N*-(2-hydroxypropyl)methacrylamide, poly-2-(methacryloyloxy)ethyl phosphorylcholines, poly(hydroxyalkyl L-asparagine), poly(2-(methacryloyloxy)ethyl phosphorylcholine), hydroxyethylstarch or poly(hydroxyalkyl L-glutamine), wherein the hydrophilic polymer chain exhibits a molecular weight of 1 kDa to 100 kDa,

$P^2$ is a cationic or polycationic peptide or protein, having a length of 3 to 100 amino acids, or is a cationic or polycationic polymer, having a molecular weight of 0.5 kDa to 30 kDa,

each $P^2$ exhibiting at least two -SH-moieties, capable to form a disulfide linkage upon condensation with further components $P^2$ or component(s) $P^1$ and/or $P^3$;

-S-S- is a (reversible) disulfide bond;

L is an optional ligand, which may be present or not, and may be selected independent from the other from RGD, Transferrin, Folate, a signal peptide or signal sequence, a localization signal or sequence, a nuclear localization signal or sequence (NLS), an antibody, a cell penetrating peptide, TAT, a ligand of a receptor, cytokines, hormones, growth factors, small molecules, carbohydrates, mannose, galactose, synthetic ligands, small molecule agonists, inhibitors or antagonists of receptors, or RGD peptidomimetic analogues; and

n is an integer, selected from a range of 1 to 50, preferably in a range of 1, 2, 3, 4, or 5 to 10, more preferably in a range of 1, 2, 3, or 4 to 9,

wherein the nanoparticle is coated with a biodegradable polymer and wherein the biodegradable polymer is a PLGA polymer.

2. The nanoparticle according to claim 1, wherein the polymeric carrier molecule additionally contains an amino acid component $(AA)_x$, wherein x is an integer selected from a range of 1 to 100.

3. The nanoparticle according to any of claims 1 to 2, wherein the -SH-moiety of component(s) $P^2$ of the polymeric carrier is provided by a cysteine.

4. The nanoparticle according to any of claims 1 to 3, wherein component $P^2$ of the polymeric carrier is selected from a peptide comprising a cationic peptide of formula (IIb):

$$Cys\{(Arg)_l;(Lys)_m;(His)_n;(Orn)_o;(Xaa)_x\}Cys, \qquad \text{(formula IIb)}$$

wherein

l + m + n + o + x = 8-15, and l, m, n or o independently of each other may be any number selected from 0, 1, 2, 3, 4, 5, 6, 7, 8, 9, 10, 11, 12, 13, 14 or 15, provided that the overall content of Arg, Lys, His and Orn represents at least 10% of all amino acids of the oligopeptide; and Xaa may be any amino acid selected from native or non-native amino acids except of Arg, Lys, His or Orn; and x may be any number selected from 0, 1, 2, 3, 4, 5, 6, 7, 8, 9, 10, 11, 12, 13 or 14, provided, that the overall content of Xaa does not exceed 90 % of all amino acids of the oligopeptide.

5. The nanoparticle according to any of claims 1 to 4, wherein the nucleic acid is provided in a molar ratio of 5 to 10000 of polymeric carrier molecule : nucleic acid.

6. The nanoparticle according to claim 1 to 5, wherein the DNA or RNA is a coding DNA, a coding RNA, a coding mRNA, an siRNA, an immunostimulatory nucleic acid, an immunostimulatory CpG nucleic acid, or an immunostimulatory RNA (isRNA).

7. The nanoparticle according to any of claims 1 to 6, wherein the nucleic acid encodes a therapeutically active protein or peptide, an antigen, including tumor antigens, pathogenic antigens, animal antigens, viral antigens, protozoal antigens, bacterial antigens, allergic antigens, autoimmune antigens, allergens, antibodies, immunostimulatory proteins or peptides, or antigen-specific T-cell receptors.

8. The nanoparticle according to any of claims 1 to 7, wherein the PLGA polymer is defined by an average molecular weight in the range of 4 kDa to 210 kDa, more preferably in the range of 10 kDa to 110 kDa, even more preferably in the range of 20 kDa to 80 kDa.

9. The nanoparticle according to any of claims 1 to 8, wherein the proportion of lactic acid in the PLGA polymer is in the range of 25 to 100%, and preferably in the range of 25 to 85%.

10. Composition comprising a nanoparticle according to any of claims 1 to 9 and optionally a pharmaceutically acceptable carrier and/or vehicle.

11. The composition according to claim 10, wherein the composition is a pharmaceutical composition and/or vaccine.

**12.** Method for preparing a coated nanoparticle as defined in any of claims 1 to 9, the method comprising the following steps:

a) providing a nanoparticle comprising or consisting of a complex of a nucleic acid, which is a DNA or RNA, and a polymeric carrier molecule according to generic formula (I):

$$\text{L-P}^1\text{-S-[S-P}^2\text{-S]}_n\text{-S-P}^3\text{-L} \qquad \text{(formula I)}$$

wherein

$P^1$ and $P^3$ are different or identical to each other and represent a linear or branched hydrophilic polymer chain, each $P^1$ and $P^3$ exhibiting at least one -SH-moiety, capable to form a disulfide linkage upon condensation with component $P^2$, the linear or branched hydrophilic polymer chain selected independent from each other from polyethylene glycol (PEG), poly-*N*-(2-hydroxypropyl)methacrylamide, poly-2-(methacryloyloxy)ethyl phosphorylcholines, poly(hydroxyalkyl L-asparagine), poly(2-(methacryloyloxy)ethyl phosphorylcholine), hydroxyethylstarch or poly(hydroxyalkyl L-glutamine), wherein the hydrophilic polymer chain exhibits a molecular weight of about 1 kDa to about 100 kDa,
$P^2$ is a cationic or polycationic peptide or protein, having a length of 3 to 100 amino acids, or is a cationic or polycationic polymer, having a molecular weight of 0.5 kDa to 30 kDa, each $P^2$ exhibiting at least two -SH-moieties, capable to form a disulfide linkage upon condensation with further components $P^2$ or component(s) $P^1$ and/or $P^3$;
-S-S- is a (reversible) disulfide bond;
L is an optional ligand, which may be present or not, and may be selected independent from the other from RGD, Transferrin, Folate, a signal peptide or signal sequence, a localization signal or sequence, a nuclear localization signal or sequence (NLS), an antibody, a cell penetrating peptide, TAT, a ligand of a receptor, cytokines, hormones, growth factors, small molecules, carbohydrates, mannose, galactose, synthetic ligands, small molecule agonists, inhibitors or antagonists of receptors, or RGD peptidomimetic analogues; and
n is an integer, selected from a range of 1 to 50, preferably in a range of 1, 2, 3, 4, or 5 to 10 , more preferably in a range of 1, 2, 3, or 4 to 9; and

b) contacting, preferably by mixing, the nanoparticle of a) with a biodegradable PLGA polymer in an organic solvent containing solution, and
c) optionally removing, e.g. by drying, the organic solvent and where appropriate any other solvent in the organic solvent containing solution.

**13.** The method according to claim 12, the method comprising the following steps:

I. Preparing the polymeric carrier according to formula (I) (or any of its subformula) by:

a) providing at least one cationic or polycationic protein or peptide as component $P^2$ and/or at least one cationic or polycationic polymer as component $P^2$, and optionally at least one further component $(AA)_x$, mixing these components, preferably in a basic milieu, preferably in the presence of oxygen or a further starter which leads to mild oxidation conditions, thereby condensing and thus polymerizing these components with each other via disulfide bonds in a polymerization condensation or polycondensation to obtain a repetitive component $\text{H-[S-P}^2\text{-S]}_n\text{-H}$ or $\text{H\{[S-P}^2\text{-S]}_a[\text{S-(AA)}_x\text{-S]}_b\}\text{H}$; etc.;
b) providing a hydrophilic polymer $P^1$ and/or $P^3$ as defined according to claim 1, optionally modified with a ligand L and/or an amino acid component $(AA)_x$ as defined according to claim 1 or claim 2, respectively;
c) mixing the hydrophilic polymer $P^1$ and/or $P^3$ according to step b) with the repetitive component $\text{H-[S-P}^2\text{-S]}_n\text{-H}$ or $\text{H\{[S-P}^2\text{-S]}_a[\text{S-(AA)}_x\text{-S]}_b\}\text{H}$ obtained according to step a) in a ratio of 2 : 1, thereby typically terminating the polymerization condensation or polycondensation reaction and obtaining the polymeric carrier molecule according to formula (I) or (Ia);
d) optionally purifying the polymeric carrier molecule obtained according to step c);

II. Complexation of the polymeric carrier with the nucleic acid cargo:
adding a nucleic acid, which is a DNA or RNA, to the polymeric carrier obtained according to step I.c) or I.d), and complexing the nucleic acid with the polymeric carrier obtained according to step I.c) or I.d) to obtain a polymeric carrier cargo complex;
III. Optional lyophilization of the polymeric carrier cargo complex:

a) optionally lyophilizing the polymeric carrier cargo complex as obtained from step II;
b) prior to the coating with the biodegradable PLGA polymer reconstituting the polymeric carrier cargo complexes in a solvent, preferably in an organic solvent containing solution;

IV. Mixing of the resulting solution from step II. or III. with the biodegradable PLGA polymer dissolved in an organic solvent containing solution; and
V. Optionally removing, e.g. by drying, the organic solvent and/or, where appropriate, any other solvent in the resulting solution of step IV..

**14.** Method according to claim 12 or 13, wherein the organic solvent containing solution comprises or consists of acetone, ethanol and/or THF.

**15.** The coated nanoparticle according to any of claims 1 to 9 for use as a medicament.

**16.** The coated nanoparticle according to any of claims 1 to 9 or the pharmaceutical composition or vaccine according to claim 10 or claim 11 for use in the prophylaxis, treatment and/or amelioration of diseases selected from cancer or tumour diseases; infectious diseases, preferably (viral, bacterial or protozoological) infectious diseases; autoimmune diseases; allergies or allergic diseases; monogenetic diseases, i.e. (hereditary) diseases, or genetic diseases in general; diseases which have a genetic inherited background and which are typically caused by a defined gene defect and are inherited according to Mendel's laws; cardiovascular diseases; neuronal diseases; diseases of the respiratory system; diseases of the digestive system; diseases of the skin; musculoskeletal disorders; disorders of the connective tissue; neoplasms; immune deficiencies; endocrine, nutritional and metabolic diseases; eye diseases and ear diseases.

**17.** The coated nanoparticle according to any of claims 1 to 9 or the composition according to claim 10 or claim 11 for use in the prevention or treatment of restenosis; calcification; foreign body reaction; and/or inflammation.

**18.** Medical or diagnostic device, particularly a stent or an implant, comprising a coated nanoparticle according to any of claims 1 to 9.

**19.** The device according to claim 18, wherein the nanoparticle is comprised in a coating on a surface of the device.

**Patentansprüche**

**1.** Nanopartikel, welches einen Komplex aus einer Nukleinsäure, bei der es sich um eine DNA oder RNA handelt, und einem polymeren Trägermolekül gemäß der generischen Formel (I) umfasst oder aus diesem besteht:

$$\text{L-P}^1\text{-S-[S-P}^2\text{-S]}_n\text{-S-P}^3\text{-L} \qquad \text{(Formel I)},$$

wobei

$P^1$ und $P^3$ verschieden voneinander oder identisch sind und eine lineare oder verzweigte hydrophile Polymerkette darstellen, wobei $P^1$ und $P^3$ wenigstens eine -SH-Gruppe aufweisen, die eine Disulfidverknüpfung nach Kondensation mit Komponente $P^2$ ausbilden kann, wobei die lineare oder verzweigte hydrophile Polymerkette unabhängig voneinander aus Polyethylenglycol (PEG), Poly-*N*-(2-hydroxypropyl)methacrylamid, Poly-2-(methacryloyloxy)ethylphosphorylcholinen, Poly(hydroxyalkyl-L-asparagin), Poly(2-(methacryloyloxy)ethylphosphorylcholin), Hydroxyethylstärke oder Poly(hydroxyalkyl-L-glutamin) ausgewählt ist, wobei die hydrophile Polymerkette ein Molekulargewicht von 1 kDa bis 100 kDa aufweist,
$P^2$ ein kationisches oder polykationisches Peptid oder Protein mit einer Länge von 3 bis 100 Aminosäuren ist oder ein kationisches oder polykationisches Polymer mit einem Molekulargewicht von 0,5 kDa bis 30 kDa ist, wobei $P^2$ jeweils wenigstens zwei -SH-Gruppen aufweist, die eine Disulfidverknüpfung nach Kondensation mit weiteren Komponenten $P^2$ oder Komponente(n) $P^1$ und/oder $P^3$ ausbilden können,
-S-S- eine (reversible) Disulfidbindung ist;
L ein optionaler Ligand ist, der vorliegen kann oder nicht, und unabhängig von den anderen ausgewählt sein kann aus RGD, Transferrin, Folat, einem Signalpeptid oder einer Signalsequenz, einem Lokalisationssignal oder einer Lokalisationssequenz, einem Kernlokalisationssignal oder einer Kernlokalisationssequenz (NLS), einem Antikörper, einem zellpenetrierenden Peptid, TAT, einem Liganden eines Rezeptors, Zytokinen, Hormonen, Wachstumsfaktoren, kleinen Molekülen, Kohlehydraten, Mannose, Galaktose, synthetischen Liganden, kleinen Molekül-Agonisten, Inhibitoren oder Antagonisten von Rezeptoren oder RGD-peptidomimetischen Analoga; und

n eine ganze Zahl ist, die aus einem Bereich von 1 bis 50, bevorzugt einem Bereich von 1, 2, 3, 4 oder 5 bis 10, noch bevorzugter einem Bereich von 1, 2, 3 oder 4 bis 9, ausgewählt ist,

wobei das Nanopartikel mit einem biologisch abbaubaren Polymer beschichtet ist, und wobei das biologisch abbaubare Polymer ein PLGA-Polymer ist.

2. Nanopartikel gemäß Anspruch 1, wobei das polymere Trägermolekül außerdem eine Aminosäurekomponente $(AA)_x$ enthält, wobei x eine ganze Zahl ist, die aus einem Bereich von 1 bis 100 ausgewählt ist.

3. Nanopartikel gemäß irgendeinem der Ansprüche 1 bis 2, wobei die -SH-Gruppe der Komponente(n) $P^2$ des polymeren Trägers durch ein Cystein bereitgestellt wird.

4. Nanopartikel gemäß irgendeinem der Ansprüche 1 bis 3, wobei die Komponente $P^2$ des polymeren Trägers ausgewählt ist aus einem Peptid, welches ein kationisches Peptid der Formel (IIb) umfasst:

$$Cys\{(Arg)_l;(Lys)_m;(His)_n;(Orn)_o;(Xaa)_x\}Cys \qquad (Formel\ IIb),$$

wobei

l + m + n + o + x = 8-15, und l, m, n oder o unabhängig voneinander irgendeine Zahl, ausgewählt aus 0, 1, 2, 3, 4, 5, 6, 7, 8, 9, 10, 11, 12, 13, 14 oder 15, sein können, mit der Maßgabe, dass der Gesamtgehalt an Arg, Lys, His und Orn mindestens 10% aller Aminosäuren des Oligopeptids darstellen; und Xaa eine beliebige Aminosäure, ausgewählt aus nativen oder nicht nativen Aminosäuren, ausgenommen Arg, Lys, His oder Orn, sein kann; und x irgendeine Zahl, ausgewählt aus 0, 1, 2, 3, 4, 5, 6, 7, 8, 9, 10, 11, 12, 13 oder 14, sein kann, mit der Maßgabe, dass der Gesamtgehalt an Xaa 90% aller Aminosäuren des Oligopeptids nicht übersteigt.

5. Nanopartikel gemäß irgendeinem der Ansprüche 1 bis 4, wobei die Nukleinsäure in einem Molverhältnis des polymeren Trägermoleküls : Nukleinsäure von 5 bis 10000 bereitgestellt wird.

6. Nanopartikel gemäß Anspruch 1 bis 5, wobei die DNA oder RNA eine kodierende DNA, eine kodierende RNA, eine kodierende mRNA, eine siRNA, eine immunstimulatorische Nukleinsäure, eine immunstimulatorische CpG-Nukleinsäure oder eine immunstimulatorische RNA (isRNA) ist.

7. Nanopartikel gemäß irgendeinem der Ansprüche 1 bis 6, wobei die Nukleinsäure ein therapeutisch aktives Protein oder Peptid, ein Antigen, einschließlich Tumorantigene, pathogene Antigene, tierische Antigene, virale Antigene, Protozoen-Antigene, bakterielle Antigene, Allergie-Antigene, Autoimmun-Antigene Allergene, Antikörper, immunstimulatorische Proteine oder Peptide, oder Antigen-spezifische T-Zell-Rezeptoren kodiert.

8. Nanopartikel gemäß irgendeinem der Ansprüche 1 bis 7, wobei das PLGA-Polymer durch ein mittleres Molekulargewicht in dem Bereich von 4 kDa bis 210 kDa, bevorzugter in dem Bereich von 10 kDa bis 110 kDa, noch bevorzugter in dem Bereich von 20 kDa bis 80 kDa, definiert ist.

9. Nanopartikel gemäß irgendeinem der Ansprüche 1 bis 8, wobei der Anteil an Milchsäure in dem PLGA-Polymer im Bereich von 25 bis 100% und bevorzugt im Bereich von 25 bis 85% liegt.

10. Zusammensetzung, umfassend ein Nanopartikel gemäß irgendeinem der Ansprüche 1 bis 9 und optional einen pharmazeutisch akzeptablen Trägerstoff und/oder ein pharmazeutisch akzeptables Vehikel.

11. Zusammensetzung gemäß Anspruch 10, wobei die Zusammensetzung eine pharmazeutische Zusammensetzung und/oder ein Vakzin ist.

12. Verfahren zur Herstellung eines beschichteten Nanopartikels, wie in irgendeinem der Ansprüche 1 bis 9 definiert, wobei das Verfahren die folgenden Schritte umfasst:

a) Bereitstellen eines Nanopartikels, welches einen Komplex aus einer Nukleinsäure, bei der es sich um eine DNA oder RNA handelt, und einem polymeren Trägermolekül gemäß der generischen Formel (I) umfasst oder aus diesem besteht:

$$L-P^1-S-[S-P^2-S]_n-S-P^3-L \qquad \text{(Formel I)},$$

wobei

$P^1$ und $P^3$ verschieden voneinander oder identisch sind und eine lineare oder verzweigte hydrophile Polymerkette darstellen, wobei $P^1$ und $P^3$ wenigstens eine -SH-Gruppe aufweisen, die eine Disulfidverknüpfung nach Kondensation mit Komponente $P^2$ ausbilden kann, wobei die lineare oder verzweigte hydrophile Polymerkette unabhängig voneinander aus Polyethylenglycol (PEG), Poly-*N*-(2-hydroxypropyl)methacrylamid, Poly-2-(methacryloyloxy)ethylphosphorylcholinen, Poly(hydroxyalkyl-L-asparagin), Poly(2-(methacryloyloxy)ethylphosphorylcholin), Hydroxyethylstärke oder Poly(hydroxyalkyl-L-glutamin) ausgewählt ist, wobei die hydrophile Polymerkette ein Molekulargewicht von etwa 1 kDa bis etwa 100 kDa aufweist,

$P^2$ ein kationisches oder polykationisches Peptid oder Protein mit einer Länge von 3 bis 100 Aminosäuren ist oder ein kationisches oder polykationisches Polymer mit einem Molekulargewicht von 0,5 kDa bis 30 kDa ist, wobei $P^2$ jeweils wenigstens zwei -SH-Gruppen aufweist, die eine Disulfidverknüpfung nach Kondensation mit weiteren Komponenten $P^2$ oder Komponente(n) $P^1$ und/oder $P^3$ ausbilden können, -S-S- eine (reversible) Disulfidbindung ist;

L ein optionaler Ligand ist, der vorliegen kann oder nicht, und unabhängig von den anderen ausgewählt sein kann aus RGD, Transferrin, Folat, einem Signalpeptid oder einer Signalsequenz, einem Lokalisationssignal oder einer Lokalisationssequenz, einem Kernlokalisationssignal oder einer Kernlokalisationssequenz (NLS), einem Antikörper, einem zellpenetrierenden Peptid, TAT, einem Liganden eines Rezeptors, Zytokinen, Hormonen, Wachstumsfaktoren, kleinen Molekülen, Kohlehydraten, Mannose, Galaktose, synthetischen Liganden, kleinen Molekül-Agonisten, Inhibitoren oder Antagonisten von Rezeptoren oder RGD-peptidomimetischen Analoga; und

n eine ganze Zahl ist, die aus einem Bereich von 1 bis 50, bevorzugt einem Bereich von 1, 2, 3, 4 oder 5 bis 10, noch bevorzugter einem Bereich von 1, 2, 3 oder 4 bis 9, ausgewählt ist, und

b) In-Kontakt-Bringen, vorzugsweise durch Mischen, des Nanopartikels von a) mit einem biologisch abbaubaren PLGA-Polymer in einer organisches Lösungsmittel enthaltenden Lösung und

c) optionales Entfernen, zum Beispiel durch Trocknen, des organischen Lösungsmittels und gegebenenfalls irgendeines anderen Lösungsmittels in der organisches Lösungsmittel enthaltenden Lösung.

**13.** Verfahren gemäß Anspruch 12, wobei das Verfahren die folgenden Schritte umfasst:

I. Herstellen des polymeren Trägers gemäß Formel (I) (oder irgendeiner ihrer Unterformeln) durch:

a) Bereitstellen wenigstens eines kationischen oder polykationischen Proteins oder Peptids als Komponente $P^2$ und/oder wenigstens eines kationischen oder polykationischen Polymers als Komponente $P^2$ und optional mindestens einer weiteren Komponente $(AA)_x$, Mischen dieser Komponenten, vorzugsweise in einem basischen Milieu, vorzugsweise in Gegenwart von Sauerstoff oder eines weiteren Starters, welcher zu milden Oxidationsbedingungen führt, dadurch Kondensieren und somit Polymerisieren dieser Komponenten miteinander über Disulfidbrücken in einer Polymerisationskondensation oder Polykondensation zum Erhalt einer repetitiven Komponente $H-[S-P^2-S]_n-H$ oder $H\{[S-P^2-S]_a[S-(AA)_x-S]_b\}H$; usw.;

b) Bereitstellen eines hydrophilen Polymers $P^1$ und/oder $P^3$, wie gemäß Anspruch 1 definiert, optional modifiziert mit einem Liganden L und/oder einer Aminosäurekomponente $(AA)_x$, wie gemäß Anspruch 1 bzw. Anspruch 2 definiert;

c) Mischen des hydrophilen Polymers $P^1$ und/oder $P^3$ gemäß Schritt b) mit der gemäß Schritt a) erhaltenen repetitiven Komponente $H-[S-P^2-S]_n-H$ oder $H\{[S-P^2-S]_a[S-(AA)_x-S]_b\}H$ in einem Verhältnis von 2 : 1, wodurch typischerweise die Polymerisationskondensation oder Polykondensationsreaktion beendet wird und das polymere Trägermolekül gemäß Formel (I) oder (Ia) erhalten wird;

d) optionales Reinigen des gemäß Schritt c) erhaltenen polymeren Trägermoleküls;

II. Komplexbildung des polymeren Trägers mit der Nukleinsäure-Fracht:
Zugabe einer Nukleinsäure, bei der es sich um eine DNA oder RNA handelt, zu dem gemäß Schritt I.c) oder I.d) erhaltenen polymeren Träger und Komplexieren der Nukleinsäure mit dem gemäß Schritt I.c) oder I.d) erhaltenen polymeren Träger zum Erhalt eines Komplexes aus polymerem Träger und Fracht;

III. Optionale Lyophilisierung des Komplexes aus polymerem Träger und Fracht:

a) optionales Lyophilisieren des aus Schritt II erhaltenen Komplexes aus polymerem Träger und Fracht;

b) vor der Beschichtung mit dem biologisch abbaubaren PLGA-Polymer Wiederherstellen des Komplexes aus polymerem Träger und Fracht in einem Lösungsmittel, vorzugsweise in einer organisches Lösungsmittel enthaltenden Lösung;

IV. Mischen der aus Schritt II. oder III. resultierenden Lösung mit dem biologisch abbaubaren PLGA-Polymer, welches in einer organisches Lösungsmittel enthaltenden Lösung gelöst ist; und

V. Optionales Entfernen, zum Beispiel durch Trocknen, des organischen Lösungsmittels und/oder gegebenenfalls irgendeines anderen Lösungsmittels in der aus Schritt IV. resultierenden Lösung.

14. Verfahren gemäß Anspruch 12 oder 13, wobei die organisches Lösungsmittel enthaltende Lösung Aceton, Ethanol und/oder THF umfasst oder daraus besteht.

15. Beschichtetes Nanopartikel gemäß irgendeinem der Ansprüche 1 bis 9 zur Verwendung als Medikament.

16. Beschichtetes Nanopartikel gemäß irgendeinem der Ansprüche 1 bis 9 oder pharmazeutische Zusammensetzung oder Vakzin gemäß Anspruch 10 oder Anspruch 11 zur Verwendung bei der Prophylaxe, Behandlung und/oder Linderung von Krankheiten, ausgewählt aus Krebs- oder Tumorerkrankungen; Infektionskrankheiten, vorzugsweise virale, bakterielle oder durch Protozoen hervorgerufene Infektionskrankheiten; Autoimmunerkrankungen; Allergien oder allergischen Erkrankungen; monogenetischen Krankheiten, d.h., vererbbaren Krankheiten, oder genetischen Krankheiten (Erbkrankheiten) allgemein; Krankheiten, welche einen genetisch vererbten Hintergrund haben und welche typischerweise durch einen definierten Gendefekt begründet sind und gemäß den Mendelschen Gesetzen vererbt werden; kardiovaskuläre Erkrankungen; neuronale Erkrankungen; Erkrankungen der Atemwege; Erkrankungen des Verdauungssystems; Hautkrankheiten; Muskel-Skelett-Erkrankungen; Erkrankungen des Bindegewebes; Neoplasmen; Immundefizienzen; endokrine, ernährungsbedingte und metabolische Krankheiten; Augenkrankheiten und Ohrenkrankheiten.

17. Beschichtetes Nanopartikel gemäß irgendeinem der Ansprüche 1 bis 9 oder Zusammensetzung gemäß Anspruch 10 oder Anspruch 11 zur Verwendung bei der Prophylaxe oder Behandlung von Restenose; Kalzifikation; Fremdkörperreaktion; und/oder Entzündung.

18. Medizinische oder diagnostische Vorrichtung, insbesondere Stent oder Implantat, welche ein beschichtetes Nanopartikel gemäß irgendeinem der Ansprüche 1 bis 9 umfasst.

19. Vorrichtung gemäß Anspruch 18, wobei das Nanopartikel in einer Beschichtung auf einer Oberfläche der Vorrichtung enthalten ist.

**Revendications**

1. Nanoparticule comprenant ou constituée par un complexe d'un acide nucléique, à savoir un ADN ou un ARN, et d'une molécule de support polymère répondant à la formule générique (I) :

$$L\text{-}P^1\text{-}S\text{-}[S\text{-}P^2\text{-}S]_n S\text{-}P^3\text{-}L \qquad \text{(formule I)}$$

dans laquelle

$P^1$ et $P^3$ sont identiques ou différents l'un de l'autre et représentent une chaîne polymère hydrophile linéaire ou ramifiée, chaque $P^1$ et $P^3$ présentant au moins une fraction -SH- capable de former une liaison disulfure après condensation avec le composant $P^2$, la chaîne polymère hydrophile linéaire ou ramifiée étant choisie, de manière respectivement indépendante, parmi le polyéthylène glycol (PEG), le poly-*N*-(2-hydroxypropyl)méthacrylamide, des poly-2-(méthacryloyloxy)éthyl phosphorylcholines, la poly(hydroxyalkyl L-asparagine), la poly(2-(méthacryloyloxy)éthyl phosphorylcholine), l'hydroxyéthylamidon ou la poly(hydroxyalkyl L-glutamine) ; dans laquelle la chaîne polymère hydrophile manifeste un poids moléculaire de 1 kDa à 100 kDa ;

$P^2$ représente un peptide ou une protéine cationique ou polycationique, dont la longueur s'élève de 3 à 100 acides aminés ; ou

représente un polymère cationique ou polycationique, dont le poids moléculaire s'élève de 0,5 kDa à 30 kDa ; chaque $P^2$ présentant au moins deux fractions -SH- capables de former une liaison disulfure après condensation avec d'autres composants $P^2$ ou bien un ou des composants $P^1$ et/ou $P^3$ ;

-S-S- représente une liaison disulfure (réversible) ;

L représente un ligand facultatif, qui peut être présent ou non, et qui peut être choisi de manière respectivement indépendante parmi le motif RGD, la transferrine, un folate, un peptide signal ou une séquence signal, un signal ou une séquence de localisation, un signal ou une séquence de localisation nucléaire (NLS), un anticorps, un peptide de pénétration cellulaire, le gène TAT, un ligand d'un récepteur, des cytokines, des hormones, des facteurs de croissance, des petites molécules, des hydrates de carbone, le mannose, le galactose, des ligands synthétiques, des agonistes à petites cellules, des inhibiteurs ou des antagonistes de récepteurs, ou des analogues peptidomimétiques de RGD ; et

n représente un entier choisi parmi une plage de 1 à 50, de préférence dans une plage de 1, 2, 3, 4 ou 5 à 10, de manière plus préférée dans une plage de 1, 2, 3 ou 4 à 9 ;

dans laquelle la nanoparticule est enrobée avec un polymère biodégradable et dans laquelle le polymère biodégradable est un polymère PLGA.

2. Nanoparticule selon la revendication 1, dans laquelle la molécule de support polymère contient en outre un composant d'acide aminé $(AA)_x$, où x représente un entier choisi parmi une plage de 1 à 100.

3. Nanoparticule selon l'une quelconque des revendications 1 à 2, dans laquelle la fraction -SH- du/des composant(s) $P^2$ du support polymère est fournie par une cystéine.

4. Nanoparticule selon l'une quelconque des revendications 1 à 3, dans laquelle le composant $P^2$ du support polymère est choisi parmi un peptide comprenant un peptide cationique répondant à la formule (IIb) :

$$Cys\{(Arg)_l;(Lys)_m;(His)_n;(Orn)_o;(Xaa)_x\}Cys, \qquad \text{(formule IIb)}$$

dans laquelle

$l + m + n + o + x = 8 - 15$, et l, m, n ou o peut représenter de manière respectivement indépendante n'importe quel nombre choisi parmi 0, 1, 2, 3, 4, 5, 6, 7, 8, 9, 10, 11, 12, 13, 14 ou 15, avec cette réserve que la teneur globale en Arg, Lys, His ou Orn représente au moins 10 % de la totalité des acides aminés de l'oligopeptide ; et Xaa peut représenter n'importe quel acide aminé choisi parmi des acides aminés d'origine naturelle ou non, à l'exception de Arg, Lys, His ou Orn ; et x peut représenter n'importe quel nombre choisi parmi 0, 1, 2, 3, 4, 5, 6, 7, 8, 9, 10, 11, 12, 13 ou 14, avec cette réserve que la teneur totale en Xaa n'excède pas 90 % de la totalité des acides aminés de l'oligopeptide.

5. Nanoparticule selon l'une quelconque des revendications 1 à 4, dans laquelle l'acide nucléique est fourni dans un rapport molaire molécule de support polymère : acide nucléique s'élevant de 5 à 10.000.

6. Nanoparticule selon l'une quelconque des revendications 1 à 5, dans laquelle l'ADN ou l'ARN représente un ADN codant, un ARN codant, un ARNm codant, un ARNsi, un acide nucléique immunostimulant, un acide nucléique CpG immunostimulant ou un ARN immunostimulant (ARNis).

7. Nanoparticule selon l'une quelconque des revendications 1 à 6, dans laquelle l'acide nucléique encode un peptide ou une protéine manifestant une activité thérapeutique, un antigène, y compris des antigènes tumoraux, des antigènes pathogènes, des antigènes d'origine animale, des antigènes viraux, des antigènes protozoaires, des antigènes bactériens, des antigènes allergiques, des antigènes auto-immuns, des allergènes, des anticorps, des peptides ou des protéines immunostimulants, ou des récepteurs des cellules T du type à spécificité antigénique.

8. Nanoparticule selon l'une quelconque des revendications 1 à 7, dans laquelle le polymère PLGA est défini par un poids moléculaire moyen dans la plage de 4 kDa à 210 kDa, de manière plus préférée dans la plage de 10 kDa à 110 kDa, de manière encore plus préférée dans la plage de 20 kDa à 80 kDa.

9. Nanoparticule selon l'une quelconque des revendications 1 à 8, dans laquelle la proportion de l'acide lactique dans le polymère PLGA se situe dans la plage de 25 à 100 % et de manière préférée dans la plage de 25 à 85 %.

10. Composition comprenant une nanoparticule selon l'une quelconque des revendications 1 à 9, et de manière facultative un support et/ou un véhicule pharmaceutiquement acceptable.

**11.** Composition selon la revendication 10, dans laquelle la composition est une composition pharmaceutique et/ou un vaccin.

**12.** Procédé pour la préparation d'une nanoparticule enrobée telle que définie dans l'une quelconque des revendications 1 à 9, le procédé comprenant les étapes suivantes dans lesquelles :

a) on procure une nanoparticule comprenant ou constituée par un complexe d'un acide nucléique, à savoir un ADN ou un ARN, et d'une molécule de support polymère répondant à la formule générique (I) :

$$L\text{-}P^1\text{-}S\text{-}[S\text{-}P^2\text{-}S]_n\text{-}S\text{-}P^3\text{-}L \qquad \text{(formule I)}$$

dans laquelle

$P^1$ et $P^3$ sont identiques ou différents l'un de l'autre et représentent une chaîne polymère hydrophile linéaire ou ramifiée, chaque $P^1$ et $P^3$ présentant au moins une fraction -SH- capable de former une liaison disulfure après condensation avec le composant $P^2$, la chaîne polymère hydrophile linéaire ou ramifiée étant choisie, de manière respectivement indépendante, parmi le polyéthylène glycol (PEG), le poly-*N*-(2-hydroxypropyl)méthacrylamide, des poly-2-(méthacryloyloxy)éthyl phosphorylcholines, la poly(hydroxyalkyl L-asparagine), la poly(2-(méthacryloyloxy)éthyl phosphorylcholine), l'hydroxyéthylamidon ou la poly(hydroxyalkyl L-glutamine) ; dans lequel la chaîne polymère hydrophile manifeste un poids moléculaire d'environ 1 kDa à environ 100 kDa ;

$P^2$ représente un peptide ou une protéine cationique ou polycationique, dont la longueur s'élève de 3 à 100 acides aminés ; ou

représente un polymère cationique ou polycationique, dont le poids moléculaire s'élève de 0,5 kDa à 30 kDa ; chaque $P^2$ présentant au moins deux fractions -SH- capables de former une liaison disulfure après condensation avec d'autres composants $P^2$ ou bien un ou des composants $P^1$ et/ou $P^3$ ;

-S-S- représente une liaison disulfure (réversible) ;

L représente un ligand facultatif, qui peut être présent ou non, et qui peut être choisi de manière respectivement indépendante parmi le motif RGD, la transferrine, un folate, un peptide signal ou une séquence signal, un signal ou une séquence de localisation, un signal ou une séquence de localisation nucléaire (NLS), un anticorps, un peptide de pénétration cellulaire, le gène TAT, un ligand d'un récepteur, des cytokines, des hormones, des facteurs de croissance, des petites molécules, des hydrates de carbone, le mannose, le galactose, des ligands synthétiques, des agonistes à petites cellules, des inhibiteurs ou des antagonistes de récepteurs, ou des analogues peptidomimétiques de RGD ; et

n représente un entier choisi parmi une plage de 1 à 50, de préférence dans une plage de 1, 2, 3, 4 ou 5 à 10, de manière plus préférée dans une plage de 1, 2, 3 ou 4 à 9 ; et

b) on met la nanoparticule de a) en contact, de préférence par mélange, avec un polymère PLGA biodégradable dans une solution contenant un solvant organique ; et

c) de manière facultative, on élimine, par exemple par séchage, le solvant organique, et le cas échéant n'importe quel autre solvant dans la solution contenant un solvant organique.

**13.** Procédé selon la revendication 12, le procédé comprenant les étapes suivantes dans lesquelles :

I. On prépare le support polymère répondant à la formule (I) (ou l'une quelconque de ses sous-formules) par le fait de :

a) procurer au moins un peptide ou une protéine cationique ou polycationique à titre de composant $P^2$ et/ou au moins un polymère cationique ou polycationique à titre de composant $P^2$, et de manière facultative au moins un composant supplémentaire $(AA)_x$ ; mélanger ces composants, de préférence dans un milieu basique, de préférence en présence d'oxygène ou d'un autre agent d'amorçage qui donne lieu à des conditions d'oxydation douce, pour ainsi obtenir une condensation et par conséquent une polymérisation de ces composants les uns avec les autres via des liaisons disulfure dans une condensation par polymérisation ou dans une polycondensation afin d'obtenir un composant répétitif

$$H\text{-}[S\text{-}P^2\text{-}S]_n\text{-}H \text{ ou } H\{[S\text{-}P^2\text{-}S]_a\text{-}[S\text{-}(AA)_x\text{-}S]_b\}H;$$

etc.;

b) procurer un polymère hydrophile P$^1$ et/ou P$^3$ tel que défini conformément à la revendication 1, modifié de manière facultative avec un ligand L et/ou un composant d'acide aminé (AA) tel que défini conformément à la revendication 1 ou 2, respectivement ;

c) mélanger le polymère hydrophile P$^1$ et/ou P$^3$ selon l'étape b) avec le composant répétitif H-[S-P$^2$-S]$_n$-H ou H{[S-P$^2$-S]$_a$-[S-(AA)$_x$-S]$_b$}H que l'on a obtenu conformément à l'étape a) dans un rapport de 2 : 1, pour ainsi terminer de manière spécifique la réaction de condensation par polymérisation ou de polycondensation et obtenir la molécule de support polymère répondant à la formule (I) ou (Ia) ;

d) de manière facultative, purifier la molécule de support polymère obtenu conformément à l'étape c) ;

II. On soumet le support polymère à une complexation avec l'acide nucléique cargo ;

en ajoutant un acide nucléique, à savoir un ADN ou un ARN, au support polymère que l'on a obtenu conformément à l'étape I.c) ou I.d), et en soumettant l'acide nucléique à une complexation avec le support polymère que l'on a obtenu conformément à l'étape I.c) ou I.d), afin d'obtenir un complexe de support polymère cargo ;

III. On soumet de manière facultative le complexe de support polymère cargo à une lyophilisation :

a) en lyophilisant de manière facultative le complexe de support polymère cargo tel qu'il a été obtenu à partir de l'étape II.;

b) avant l'enduction avec le polymère PLGA biodégradable, en reconstituant les complexes de support polymère cargo dans un solvant, de préférence dans une solution contenant un solvant organique ;

IV. On mélange la solution résultante de l'étape II. ou III. avec le polymère PLGA biodégradable dissous dans une solution contenant un solvant organique ; et

V. De manière facultative on élimine, par exemple par séchage, le solvant organique et/ou le cas échéant n'importe quel autre solvant dans la solution résultante de l'étape IV..

14. Procédé selon la revendication 12 ou 13, dans lequel la solution contenant un solvant organique comprend ou est constituée par de l'acétone, de l'éthanol et/ou du THF.

15. Nanoparticule enrobée selon l'une quelconque des revendications 1 à 9 pour son utilisation comme médicament.

16. Nanoparticule enrobée selon l'une quelconque des revendications 1 à 9 ou composition pharmaceutique ou vaccin selon la revendication 10 ou 11 pour son utilisation dans la prophylaxie, le traitement et/ou l'amélioration de maladies choisies parmi un cancer ou des maladies tumorales ; des maladies infectieuses, de préférence des maladies infectieuses (de type viral, bactérien ou protozoaire) ; des maladies auto-immunes ; des allergies ou des maladies allergiques ; des maladies monogéniques, c'est-à-dire des maladies (héréditaires) ou des maladies génétiques en général ; des maladies qui possèdent une base génétique héréditaire et qui sont spécifiquement dues à un gène défectueux défini et qui sont héritées conformément aux lois de Mendel ; des maladies cardio-vasculaires ; des maladies neuronales ; des maladies du système respiratoire ; des maladies du système digestif ; des maladies de la peau ; des troubles musculosquelettiques ; des troubles du tissu conjonctif ; des néoplasmes ; des déficiences du système immunitaire ; des maladies endocrines, nutritionnelles et métaboliques ; des maladies oculaires et des maladies auriculaires.

17. Nanoparticule enrobée selon l'une quelconque des revendications 1 à 9 ou composition pharmaceutique selon la revendication 10 ou 11 pour son utilisation dans la prévention ou le traitement d'une resténose ; d'une calcification ; d'une réaction à un corps étranger et/ou d'une inflammation.

18. Dispositif médical ou diagnostique, en particulier une endoprothèse ou un implant, comprenant une nanoparticule enrobée selon l'une quelconque des revendications 1 à 9.

19. Dispositif selon la revendication 18, dans lequel la nanoparticule est comprise dans un revêtement à la surface du dispositif.

Fig.1

Metal plate

PLGA immobilized nanoparticle coating

Full medium (RNAse containing)

Porcine vein graft

The coated metal plate is placed on top of the vein graft.

Fig.2

Fig.3

Fig.4

GGGAGAAAGCGAUCCAGCCACCAUGGGAGUCAAAGUUCUGUUUGCCCUGAUCUGCAUCGC
UGUGGCCGAGGCCAAGCCCACCGAGAACAACGAAGACUUCAACAUCGUGGCCGUGGCCAG
CAACUUCGCGACCACGGAUCUCGAUGCUGACCGCGGGAAGUUGCCCGGCAAGAAGCUGCC
GCUGGAGGUGCUCAAAGAGAUGGAAGCCAAUGCCCGGAAAGCUGGCUGCACCAGGGGCUG
UCUGAUCUGCCUGUCCCACAUCAAGUGCACGCCCAAGAUGAAGAAGUUCAUCCCAGGACG
CUGCCACACCUACGAAGGCGACAAAGAGUCCGCACAGGGCGGCAUAGGCGAGGCGAUCGU
CGACAUUCCUGAGAUUCCUGGGUUCAAGGACUUGGAGCCCAUGGAGCAGUUCAUCGCACA
GGUCGAUCUGUGUGUGGACUGCACAACUGGCUGCCUCAAAGGGCUUGCCAACGUGCAGUG
UUCUGACCUGCUCAAGAAGUGGCUGCCGCAACGCUGUGCGACCUUUGCCAGCAAGAUCCA
GGGCCAGGUGGACAAGAUCAAGGGGGCCGGUGGUGACUAAGCGGCCGCUCGAGCAUGCAU
CUAGUUAUAAGACUGACUAGCCCGAUGGGCCUCCCAACGGGCCCUCCUCCCCUCCUUGCA
CCGAGAUUAAUAAAAAAAAAAAAAAAAAAAAAAAAAAAAAAAAAAAAAAAAAAAAAAAAAA
AAAAAAAAAAAAAAAUAUUCCCCCCCCCCCCCCCCCCCCCCCCCCCCCCCUCUAG

## Fig.5

GGGAGAAAGCGATCCAGCCACCATGGGAGTCAAAGTTCTGTTTGCCCTGATCTGCATCGC

TGTGGCCGAGGCCAAGCCCACCGAGAACAACGAAGACTTCAACATCGTGGCCGTGGCCAG

CAACTTCGCGACCACGGATCTCGATGCTGACCGCGGGAAGTTGCCCGGCAAGAAGCTGCC

GCTGGAGGTGCTCAAAGAGATGGAAGCCAATGCCCGGAAAGCTGGCTGCACCAGGGGCTG

TCTGATCTGCCTGTCCCACATCAAGTGCACGCCCAAGATGAAGAAGTTCATCCCAGGACG

CTGCCACACCTACGAAGGCGACAAAGAGTCCGCACAGGGCGGCATAGGCGAGGCGATCGT

CGACATTCCTGAGATTCCTGGGTTCAAGGACTTGGAGCCCATGGAGCAGTTCATCGCACA

GGTCGATCTGTGTGTGGACTGCACAACTGGCTGCCTCAAAGGGCTTGCCAACGTGCAGTG

TTCTGACCTGCTCAAGAAGTGGCTGCCGCAACGCTGTGCGACCTTTGCCAGCAAGATCCA

GGGCCAGGTGGACAAGATCAAGGGGGCCGGTGGTGACTAAGCGGCCGCTCGAGCATGCAT

CTAGTTATAAGACTGACTAGCCCGATGGGCCTCCCAACGGGCCCTCCTCCCCTCCTTGCA

CCGAGATTAATAAAAAAAAAAAAAAAAAAAAAAAAAAAAAAAAAAAAAAAAAAAAAAAAAA

AAAAAAAAAAAAAAATATTCCCCCCCCCCCCCCCCCCCCCCCCCCCCCCTCTAG

Fig.6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011026641 A

- WO 03086280 A

**Non-patent literature cited in the description**

- **ONUKI, Y. ; U. BHARDWAJ et al.** A review of the biocompatibility of implantable devices: current challenges to overcome foreign body response. *J Diabetes Sci Technol,* 2008, vol. 2 (6), 1003-15
- **ONUMA, Y. ; J. ORMISTON et al.** Bioresorbable scaffold technologies. *Circ J,* vol. 75 (3), 509-20
- **ARMEANU, S. ; J. PELISEK et al.** Optimization of nonviral gene transfer of vascular smooth muscle cells in vitro and in vivo. *Mol Ther,* 2000, vol. 1 (4), 366-75
- **COHEN-SACKS, H. ; Y. NAJAJREH et al.** Novel PDGFbetaR antisense encapsulated in polymeric nanospheres for the treatment of restenosis. *Gene Ther,* 2002, vol. 9 (23), 1607-16
- **BRITO, L. ; M. AMIJI.** Nanoparticulate carriers for the treatment of coronary restenosis. *Int J Nanomedicine,* 2007, vol. 2 (2), 143-61
- **BRITO L. et al.** Non-viral eNos gene delivery and transfection with stents for the treatment of restenosis. *Biomedical Engineering Online,* 2010, vol. 9 (1

- **DANHIER et al.** PLGA-based nanoparticles: An overview of biomedical applications. *Journal of Controlled Release,* 2012, vol. 161 (2), 505-522
- **ZAMORE.** *Nat. Struct. Biol.,* 2001, vol. 9, 746-750
- **SHARP.** *Genes Dev.,* 2001, vol. 5, 485-490
- **HANNON.** *Nature,* 2002, vol. 41, 244-251
- **STARK et al.** *Annu. Rev. Biochem.,* 1998, vol. 67, 227-264
- **HE ; KATZE.** *Viral Immunol.,* 2002, vol. 15, 95-119
- **ELBASHIR et al.** *Nature,* 2001, vol. 411, 494-498
- **HEMMI H et al.** *Nature,* 2000, vol. 408, 740-5
- **BAUER S et al.** *Proc NatlAcadSci USA,* 2001, vol. 98, 9237-42
- **MEYLAN, E. ; TSCHOPP, J.** Toll-like receptors and RNA helicases: two parallel ways to trigger antiviral responses. *Mol. Cell,* 2006, vol. 22, 561-569
- **AKASHI.** *Curr. Opin. Genet. Dev.,* 2001, vol. 11 (6), 660-666
- **MEYLAN, E.** *Tschopp, J.,* 2006
- *Mol. Cell,* vol. 22, 561-569